# EUROPEAN PATENT APPLICATION

(11) **EP 4 534 533 A1**
(43) Date of publication of application: **09.04.2025**
(21) Application number: 23815246.6
(22) Date of filing: 31.05.2023
(51) Int. Cl.: C07D 401/14, C07D 403/14, C07D 471/04, A61K 31/498, A61K 41/00, A61K 31/336, A61P 35/00

(54) **AZAQUINOLINONE DERIVATIVE, PREPARATION METHOD THEREFOR AND USE THEREOF**

(30) Priority: 02.06.2022 CN 202210625197; 16.09.2022 CN 202211128451; 16.01.2023 CN 202310060982; 10.03.2023 CN 202310228231
(71) Applicant: Chengdu Easton Biopharmaceuticals Co., Ltd., Hi-tech District Chengdu, Sichuan 611731 (CN)
(72) Inventor: SONG, Liqiang, Chengdu, Sichuan 611731 (CN); WANG, Qiong, Chengdu, Sichuan 611731 (CN); MAN, Yi, Chengdu, Sichuan 611731 (CN); SUN, Ling, Chengdu, Sichuan 611731 (CN); ZHANG, Yi, Chengdu, Sichuan 611731 (CN); XIANG, Yongzhe, Chengdu, Sichuan 611731 (CN); LI, Li, Chengdu, Sichuan 611731 (CN); CHEN, Hong, Chengdu, Sichuan 611731 (CN); WANG, Ying, Chengdu, Sichuan 611731 (CN)
(74) Representative: Uexküll & Stolberg
(86) International application number: PCT/CN2023/097403
(87) International publication number: WO 2023/232069

(57) **Abstract**

An azacuinolinone compound having a structure of the following formula (I) as an enzyme inhibitor for the poly(ADP-ribose)polymerase (PARP) family, and a pharmaceutically acceptable salt, a stereoisomer, a tautomer or an N-oxide drug thereof, a preparation method therefor and the use thereof.

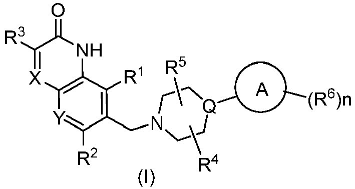

## Description

### TECHNICAL FIELD

The present application relates to an azaquinolinone compound as an enzyme inhibitor for the poly(ADP-ribose)polymerase (PARP) familiy, a pharmaceutically acceptable salt or a stereoisomer thereof, a pharmaceutical composition thereof, a preparation method therefor and use thereof.

### BACKGROUND

PARP1 and PARP2 (PARP1/2), by binding to DNA damage sites and catalyzing the synthesis of poly(ADP-ribose) chains on protein substrates, recruit other DNA repair proteins to the damage sites to repair DNA damage together, and meanwhile to trigger the release of PARP1 /2 from DNA. PARP inhibitors "trap" PARP1 /2 to DNA by binding to PARP1/2 catalytic sites, such that PARP1/2 cannot fall off from the DNA damage sites, thus inducing stagnation of DNA replication forks and hindering smooth implementation of DNA replication. At this time, cells usually fix this error by a homologous repeat repair (HRR) method. Proteins, such as BRCA1/2, play an important role in the HRR process, while in BRCA mutated cells, HRR is dysfunctional, and the presence of PARP inhibitors prevents the DNA damage-repairing action, thus leading to the death of cells.

Olaparib is the first PARP inhibitor developed by AstraZeneca and approved for marketing worldwide, which has been approved by the FDA for the treatment of a variety of cancers. Since then, rucaparib, niraparib and talazoparib etc. have also been approved for marketing. Meanwhile, there are a plurality of inhibitors in the clinical stage. Although PARP inhibitors have shown excellent clinical efficacy in patients with BRCA deficiencies, these compounds all have shown obvious hematotoxicity, including anemia, neutropenia, and thrombocytopenia, etc., whether used in monotherapy or combination therapy. Hematotoxicity limits the use of first-generation PARP inhibitors, and clinically requires dose reduction, suspension or discontinuation of administration.

Recent studies show that the inhibition of PARP2 is closely related to hematotoxicity. PARP2, rather than PARP1, is essential for the survival of mouse hematopoietic stem/progenitor cells (HSPCs) and is used for maintaining hematopoietic homeostasis. Moreover, the synthetic lethality of BRCA mutations is driven only by PARP1 without the need to "trap" PARP2 to DNA. Since PARP1 and PARP2 are highly homologous, most of the current PARP inhibitors lack selectivity for PARP1. Therefore, the development of PARP1 inhibitors with high selectivity is conducive to reducing hematotoxicity and improving therapeutic indexes.

Compared with other PARP1/2 inhibitors, PARP1 inhibitors with high selectivity are expected to improve efficacy and reduce toxicity. Therefore, there are unmet medical needs for effective and safe PARP inhibitors. At present, there are no PARP1 inhibitors with high selectivity on the market. AZD5305, a selective PARP1 inhibitor, has entered clinical phase I/II. AZD9574, a PARP1 inhibitor with higher selectivity than AZD5305, has also entered clinical phase I/II.

Compounds and experimental drugs disclosed in the prior art still have uncertainties in terms of effectiveness, safety and the like. Therefore, it is still necessary to screen out compounds that have excellent performance in terms of effectiveness, safety and selectivity and the like as PARP1 inhibitors.

### SUMMARY OF THE INVENTION

In order to solve the above problems of the prior art, a purpose of the present application is to provide an azaquinolinone compound and a pharmaceutically acceptable salt or stereoisomer thereof, to screen out a compound that has excellent performance in terms of effectiveness, safety and selectivity and the like as a PARP1 inhibitor.

In order to achieve this purpose of the present application, the following technical solutions are adopted in the present application:
In some embodiments, the present application provides a compound or a pharmaceutically acceptable salt, a stereoisomer, a tautomer or a N-oxide thereof, wherein the compound has a structure of Formula (I): 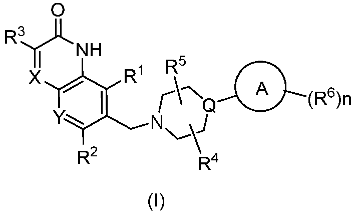 wherein:
X is selected from N, CH or CR^{a}, wherein R^{a} is selected from halogen, C₁₋₄ alkyl, C₃₋₆ cycloalkyl, -O-(C₁₋₄ alkyl) or halogenated C₁₋₄ alkyl;
Y is selected from N, CH or CR^{b}, wherein R^{b} is selected from halogen, cyano, C₁₋₄ alkyl, C₂₋₆ alkenyl, C₃₋₆ cycloalkyl, 4-6 membered heterocyclyl or -OR^{z}, wherein the C₁₋₄ alkyl, C₃₋₆ cycloalkyl or 4-6 membered heterocyclyl is unsubstituted or each independently substituted with one or more substituents selected from fluoro, cyano, hydroxy, C₁₋₄ alkyl or -O-(C₁₋₄ alkyl);
provided that: when X is selected from N or CH, Y is selected from CR^{b};
when X is selected from CR^{a}, Y is selected from N, CH or CR^{b};
R¹ is selected from hydrogen, halogen, cyano, C₁₋₄ alkyl, -O-(C₁₋₄ alkyl), -O-(halogenated C₁₋₄ alkyl) or halogenated C₁₋₄ alkyl;
R² is selected from hydrogen, F, Cl, Br, cyano, C₁₋₄ alkyl, C₃₋₆ cycloalkyl, -O-(C₁₋₄ alkyl) or halogenated C₁₋₄ alkyl;
R³ is selected from cyano, C₁₋₄ alkyl, C₃₋₆ cycloalkyl or 4-6 membered heterocyclyl, wherein the C₁₋₄ alkyl, C₃₋₆ cycloalkyl or 4-6 membered heterocyclyl is unsubstituted or each independently substituted with one or more substituents selected from F, cyano, C₁₋₄ alkyl, C₃₋₆ cycloalkyl, 4-6 membered heterocyclyl, -O-(C₁₋₄ alkyl) or halogenated C₁₋₄ alkyl;
Q is selected from N or CR^{c}, wherein R^{c} is selected from F, hydroxy, cyano, C₁₋₄ alkyl, -O-(C₁₋₄ alkyl) or halogenated C₁₋₄ alkyl;
R⁴ and R⁵ at each occurrence are each independently selected from hydrogen or C₁₋₄ alkyl, or R⁴ and R⁵ are connected to each other to form a ring; 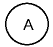
is selected from phenyl, or five-membered or six-membered heteroaryl containing 1 to 2 atoms selected from N, O or S;
R⁶ at each occurrence is each independently selected from hydrogen, halogen, cyano, -OR^{z}, -C(=O)-R^{z}, -C(=O)-NH-R^{z}, C₁₋₄ alkyl, C₃₋₆ cycloalkyl or 4-6 membered heterocyclyl, wherein the C₁₋₄ alkyl, C₃₋₆ cycloalkyl or 4-6 membered heterocyclyl is unsubstituted or each independently substituted with one or more substituents selected from F, cyano, hydroxy, C₁₋₄ alkyl, -O-(C₁₋₄ alkyl) or halogenated C₁₋₄ alkyl;
n is 1, 2 or 3.

R^{z} at each occurrence is each independently selected from hydrogen, C₁₋₄ alkyl, C₃₋₆ cycloalkyl or 4-6 membered heterocyclyl, wherein the C₁₋₄ alkyl, C₃₋₆ cycloalkyl or 4-6 membered heterocyclyl is unsubstituted or each independently substituted with one or more substituents selected from F, cyano, hydroxy, C₁₋₄ alkyl, -O-(C₁₋₄ alkyl) or fluorinated C₁₋₄ alkyl.

In some embodiments, X is selected from N, CH or CR^{a}, wherein R^{a} is selected from F, Cl, Br, C₁₋₄ alkyl, C₃₋₆ cycloalkyl, -OMe or fluorinated C₁₋₄ alkyl.

In some preferred embodiments, R^{a} is selected from F, Cl, Br, methyl, ethyl, isopropyl, cyclopropyl, -OMe, -CF₃, -CHF₂ or -CH₂F; more preferably, R^{a} is selected from F, Cl, Br, methyl, cyclopropyl, -OMe, -CHF₂ or -CH₂F; and
Y is selected from N, CH or CR^{b}, wherein R^{b} is selected from F, Cl, Br, cyano, methyl, ethyl, isopropyl, cyclopropyl, cyclobutyl, oxetanyl, oxacyclohexyl or -OR^{z}, wherein the methyl, ethyl, isopropyl, cyclopropyl, cyclobutyl, oxetanyl or oxacyclohexyl is unsubstituted or each independently substituted with one or more substituents selected from fluoro, cyano, hydroxy, methyl or -OMe.

In some preferred embodiments, R^{b} is selected from F, Cl, Br, cyano, methyl, ethyl, isopropyl, cyclopropyl, cyclobutyl, oxetanyl or -OR^{z}, wherein the methyl, ethyl, isopropyl, cyclopropyl or cyclobutyl is unsubstituted or each independently substituted with one or two substituents selected from fluoro, cyano, hydroxy or methyl;
provided that: when X is selected from N or CH, Y is selected from CR^{b};
when X is selected from CR^{a}, Y is selected from N, CH or CR^{b}.

In some embodiments, R¹ is selected from hydrogen, F, Cl, Br, cyano, methyl, ethyl, isopropyl, -OMe, -O-( fluorinated C₁₋₄ alkyl) or fluorinated C₁₋₄ alkyl.

In some preferred embodiments, R¹ is selected from hydrogen, F, Cl, methyl, -OMe, -CHF₂ or -CH₂F.

In some embodiments, R² is selected from hydrogen, F, Cl, Br, cyano, methyl, ethyl, cyclopropyl, -OMe or fluorinated C₁₋₄ alkyl.

In some preferred embodiments, R² is selected from hydrogen, F, Cl, Br, methyl, cyclopropyl, -OMe, -CHF₂ or -CH₂F.

In some embodiments, R³ is selected from cyano, methyl, ethyl, isopropyl, cyclopropyl, cyclobutyl or oxetanyl, wherein the methyl, ethyl, isopropyl, cyclopropyl, cyclobutyl or oxetanyl is unsubstituted or each independently substituted with one or more substituents selected from F, cyano, methyl, cyclopropyl, -OMe or fluorinated C₁₋₄ alkyl.

In some preferred embodiments, R³ is selected from cyano, methyl, ethyl, isopropyl, cyclopropyl, cyclobutyl or oxetanyl, wherein the methyl, ethyl, isopropyl, cyclopropyl, cyclobutyl or oxetanyl is unsubstituted or each independently substituted with one or two substituents selected from F, cyano, -OMe, -CHF₂ or -CH₂F.

In some embodiments, Q is selected from N or CR^{c}, wherein R^{c} is selected from F, hydroxy, cyano, methyl, ethyl, -OMe or fluorinated C₁₋₄ alkyl.

In some preferred embodiments, Q is selected from N or CR^{c}, wherein R^{c} is selected from F, hydroxy, cyano, methyl, -OMe, -CHF₂ or -CH₂F.

In some more preferred embodiments, Q is selected from N or CR^{c}, wherein R^{c} is selected from F, hydroxy, -OMe or methyl.

In some embodiments, R⁴ and R⁵ at each occurrence are each independently selected from hydrogen, methyl or ethyl, or R⁴ and R⁵ are connected to each other to form a ring.

In some preferred embodiments, R⁴ and R⁵ at each occurrence are each independently selected from hydrogen or methyl, or R⁴ and R⁵ are connected to each other to form a ring, forming

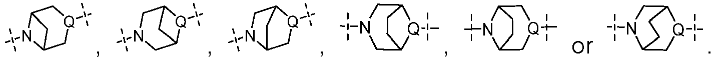

In some more preferred embodiments, R⁴ and R⁵ at each occurrence are each independently selected from hydrogen or methyl, or R⁴ and R⁵ are connected to each other to form a ring, forming

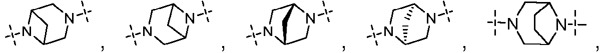

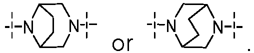

In some embodiments, 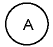 is selected from phenyl, pyridyl, pyrimidyl, pyrazinyl, pyridazinyl, pyrazolyl, oxazolyl, isooxazolyl, thienyl or thiazolyl.

In some preferred embodiments, 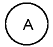 is selected from phenyl, pyridyl, pyrazolyl, oxazolyl, thienyl or thiazolyl.

In some more preferred embodiments, 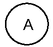 is selected from

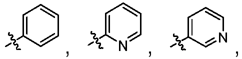

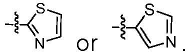

In some embodiments, R⁶ at each occurrence is each independently selected from hydrogen, F, Cl, Br, cyano, -OR^{z}, -C(=O)-R^{z}, -C(=O)-NH-R^{z}, methyl, ethyl, isopropyl, cyclopropyl, cyclobutyl or oxetanyl, wherein the methyl, ethyl, isopropyl, cyclopropyl, cyclobutyl or oxetanyl is unsubstituted or each independently substituted with one or more substituents selected from F, cyano, hydroxy, methyl, -OMe, -CF₃ or -CHF₂.

In some preferred embodiments, R⁶ at each occurrence is each independently selected from hydrogen, F, Cl, Br, cyano, -OR^{z}, -C(=O)-R^{z}, -C(=O)-NH-R^{z}, methyl, ethyl, isopropyl, cyclopropyl, cyclobutyl or oxetanyl, wherein the methyl, ethyl, isopropyl, cyclopropyl, cyclobutyl or oxetanyl is unsubstituted or each independently substituted with one or two substituents selected from F, hydroxy or -OMe.

In some embodiments, R^{z} at each occurrence is each independently selected from hydrogen, methyl, ethyl, isopropyl, cyclopropyl, cyclobutyl, oxetanyl or oxacyclopentyl, wherein the methyl, ethyl, cyclopropyl, cyclobutyl or oxacyclopentyl is unsubstituted or each independently substituted with one or more substituents selected from fluoro, cyano, hydroxy, -OMe, oxetanyl or methyl.

In some preferred embodiments, R^{z} at each occurrence is each independently selected from hydrogen, methyl, ethyl, cyclopropyl, oxetanyl, oxacyclopentyl, methylene-oxetanyl, -CF₃, -CF₂H or -CH₂F.

In some embodiments, n is 1 or 2.

In some embodiments, the present application provides a compound or a pharmaceutically acceptable salt, a stereoisomer, a tautomer or a N-oxide thereof, wherein the compound has a structure of Formula (II): 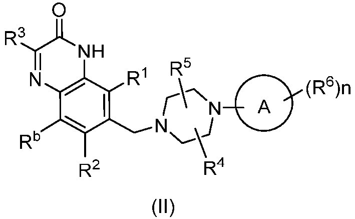 wherein:
R¹ is selected from hydrogen, F, Cl, Br, cyano, methyl, ethyl, isopropyl or -OMe;
R² is selected from hydrogen, halogen, cyano, C₁₋₄ alkyl, C₃₋₆ cycloalkyl, -O-(C₁₋₄ alkyl) or fluorinated C₁₋₄ alkyl;
R³ is selected from cyano, methyl, ethyl, isopropyl, cyclopropyl, cyclobutyl or oxetanyl, wherein the methyl, ethyl, isopropyl, cyclopropyl, cyclobutyl or oxetanyl is unsubstituted or each independently substituted with one or more substituents selected from F, cyano, methyl, cyclopropyl or -OMe;
R^{b} is selected from F, Cl, Br, cyano, C₁₋₄ alkyl, C₃₋₆ cycloalkyl, 4-6 membered heterocyclyl or -OR^{z}, wherein the C₁₋₄ alkyl, C₃₋₆ cycloalkyl or 4-6 membered heterocyclyl is unsubstituted or each independently substituted with one or more substituents selected from fluoro, cyano, hydroxy, C₁₋₄ alkyl or -O-(C₁₋₄ alkyl);
R⁴ and R⁵ at each occurrence are each independently selected from hydrogen or C₁₋₄ alkyl, or R⁴ and R⁵ are connected to each other to form a ring, forming 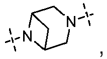 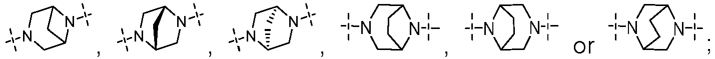 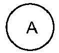
is selected from phenyl, pyridyl, pyrimidyl, pyrazinyl, pyridazinyl, pyrazolyl, oxazolyl, isooxazolyl, thienyl or thiazolyl;
R⁶ at each occurrence is each independently selected from hydrogen, halogen, cyano, -OR^{z}, -C(=O)-R^{z}, -C(=O)-NH-R^{z}, C₁₋₄ alkyl, C₃₋₆ cycloalkyl or 4-6 membered heterocyclyl, wherein the C₁₋₄ alkyl, C₃₋₆ cycloalkyl or 4-6 membered heterocyclyl is unsubstituted or each independently substituted with one or more substituents selected from F, cyano, hydroxy, C₁₋₄ alkyl, -O-(C₁₋₄ alkyl) or halogenated C₁₋₄ alkyl;
n is 1, 2 or 3; and
R^{z} at each occurrence is each independently selected from hydrogen, C₁₋₄ alkyl, C₃₋₆ cycloalkyl or 4-6 membered heterocyclyl, wherein the C₁₋₄ alkyl, C₃₋₆ cycloalkyl or 4-6 membered heterocyclyl is unsubstituted or each independently substituted with one or more substituents selected from F, cyano, hydroxy, C₁₋₄ alkyl, -O-(C₁₋₄ alkyl), 4-6 membered heterocyclyl or fluorinated C₁₋₄ alkyl.

In some embodiments, the present application provides a compound or a pharmaceutically acceptable salt, a stereoisomer, a tautomer or a N-oxide thereof, wherein the compound has a structure of Formula (III): 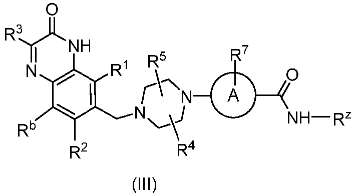 wherein:
R¹ is selected from hydrogen, F, Cl, methyl, -OMe, -CHF₂ or -CH₂F;
R² is selected from hydrogen, halogen, cyano, C₁₋₄ alkyl, C₃₋₆ cycloalkyl, -O-(C₁₋₄ alkyl) or halogenated C₁₋₄ alkyl;
R³ is selected from cyano, methyl, ethyl, isopropyl, cyclopropyl, cyclobutyl or oxetanyl, wherein the methyl, ethyl, isopropyl, cyclopropyl, cyclobutyl or oxetanyl is unsubstituted or each independently substituted with one or two substituents selected from F, cyano, methyl, cyclopropyl or -OMe;
R^{b} is selected from F, Cl, Br, cyano, methyl, ethyl, isopropyl, cyclopropyl, cyclobutyl, ooxetanyl or -OR^{z}, wherein the methyl, ethyl, isopropyl, cyclopropyl or cyclobutyl is unsubstituted or each independently substituted with one or two substituents selected from fluoro, cyano, hydroxy or methyl;
R⁴ and R⁵ at each occurrence are each independently selected from hydrogen or C₁₋₄ alkyl, or R⁴ and R⁵ are connected to each other to form a ring, forming 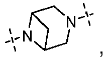 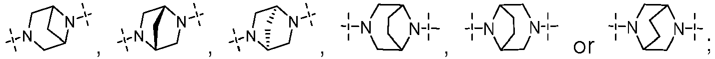 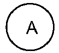
is selected from 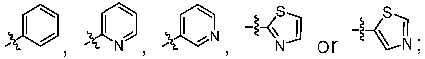
R⁷ is selected from hydrogen, F, Cl, Br, cyano, C₁₋₄ alkyl, C₃₋₆ cycloalkyl, 4-6 membered heterocyclyl or -OR^{z}, wherein the C₁₋₄ alkyl, C₃₋₆ cycloalkyl or 4-6 membered heterocyclyl is unsubstituted or each independently substituted with one or more substituents selected from F, cyano, hydroxy, C₁₋₄ alkyl, -O-(C₁₋₄ alkyl) or halogenated C₁₋₄ alkyl;
n is 1 or 2; and
R^{z} at each occurrence is each independently selected from hydrogen, C₁₋₄ alkyl, C₃₋₆ cycloalkyl or 4-6 membered heterocyclyl, wherein the C₁₋₄ alkyl, C₃₋₆ cycloalkyl or 4-6 membered heterocyclyl is unsubstituted or each independently substituted with one or more substituents selected from F, cyano, hydroxy, C₁₋₄ alkyl, -O-(C₁₋₄ alkyl), 4-6 membered heterocyclyl or fluorinated C₁₋₄ alkyl.

In some embodiments, the present application provides a compound or a pharmaceutically acceptable salt, a stereoisomer, a tautomer or a N-oxide thereof, wherein the compound has a structure of Formula (IV): 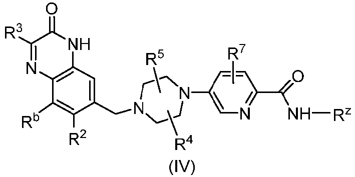 wherein,
R² is selected from hydrogen, F, Cl, Br, cyano, C₁₋₄ alkyl, C₃₋₆ cycloalkyl, -O-(C₁₋₄ alkyl) or fluorinated C₁₋₄ alkyl;
R³ is selected from cyano, methyl, ethyl, n-propyl, isopropyl, cyclopropyl, cyclobutyl or 4-6 membered heterocyclyl, wherein the methyl, ethyl, n-propyl, isopropyl, cyclopropyl, cyclobutyl or 4-6 membered heterocyclyl is unsubstituted or each independently substituted with one or more substituents selected from F, cyano, methyl or -OMe;
R^{b} is selected from F, Cl, Br, cyano, methyl, ethyl, n-propyl, isopropyl, cyclopropyl, cyclobutyl, oxetanyl or -OR^{z}, wherein the methyl, ethyl, isopropyl, cyclopropyl or cyclobutyl is unsubstituted or each independently substituted with one or two substituents selected from fluoro, cyano, hydroxy or methyl;
R⁴ and R⁵ at each occurrence are each independently selected from hydrogen, methyl or ethyl, or R⁴ and R⁵ are connected to each other to form a ring, forming 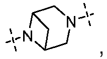 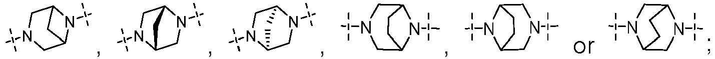
R⁷ is selected from hydrogen, halogen, cyano, -OR^{z}, C₁₋₄ alkyl, C₃₋₆ cycloalkyl or 4-6 membered heterocyclyl, wherein the C₁₋₄ alkyl, C₃₋₆ cycloalkyl or 4-6 membered heterocyclyl is unsubstituted or each independently substituted with one or more substituents selected from F, hydroxy, methyl, ethyl, methoxy or ethoxy;
n is 1 or 2; and
R^{z} at each occurrence is each independently selected from hydrogen, methyl, ethyl, n-propyl, isopropyl, cyclopropyl, cyclobutyl, oxetanyl or oxacyclopentyl, wherein the methyl, ethyl, n-propyl, isopropyl, cyclopropyl or cyclobutyl is unsubstituted or each independently substituted with one or more substituents selected from F, cyano, hydroxy, methyl, ethyl, methoxy, ethoxy, oxetanyl or fluoromethyl.

In some specific embodiments, the present application provides a compound or a pharmaceutically acceptable salt, a stereoisomer, a tautomer or a N-oxide thereof, wherein the compound is selected from:

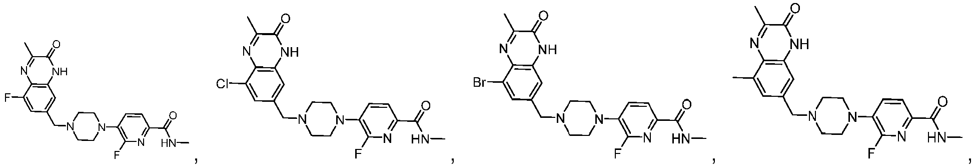

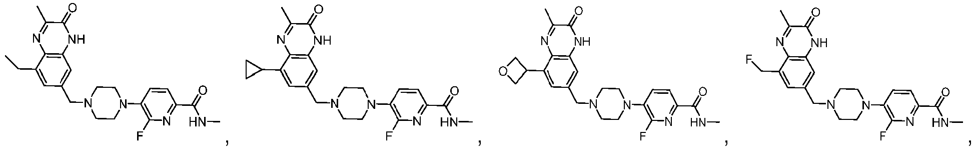

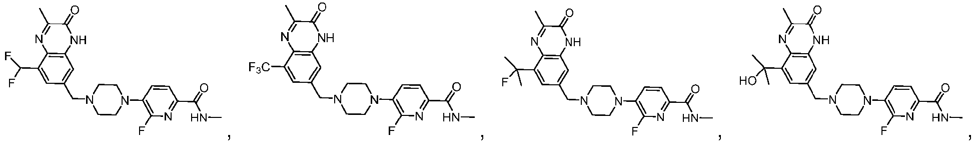

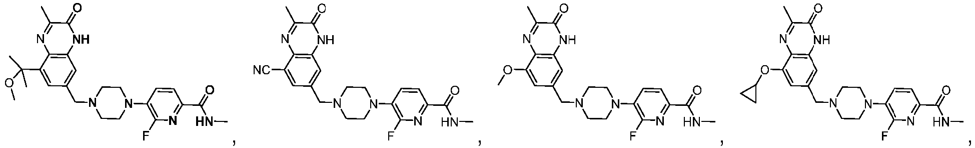

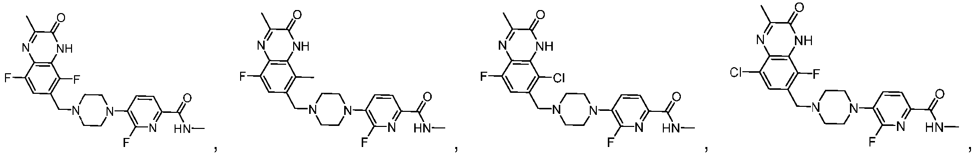

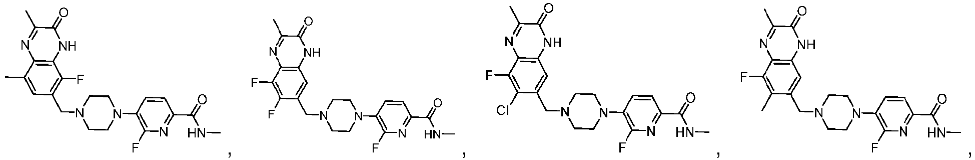

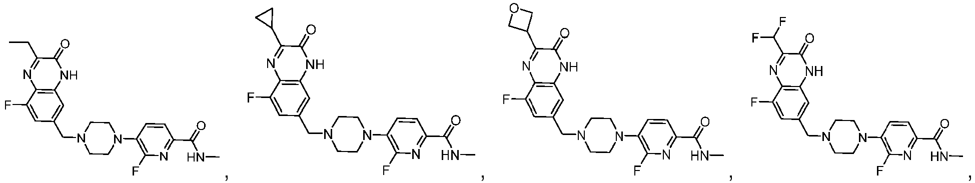

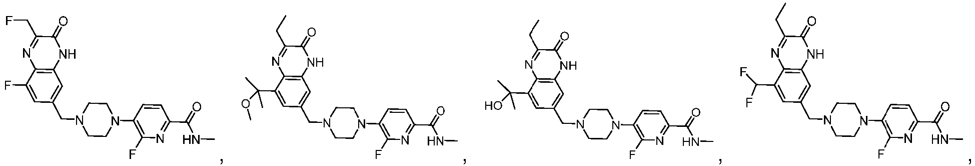

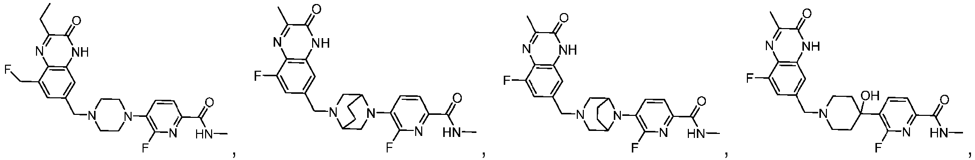

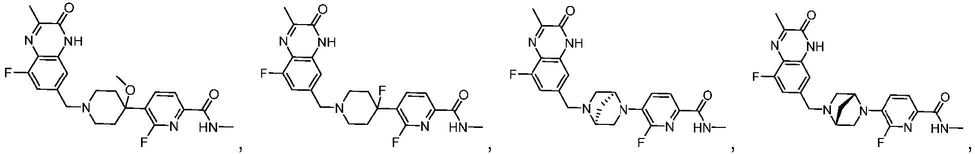

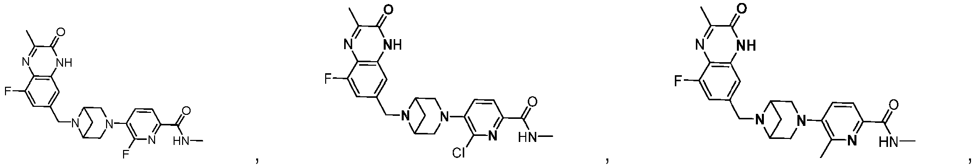

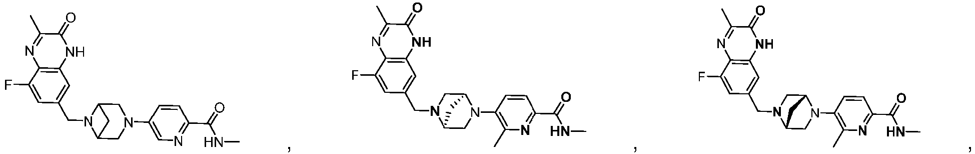

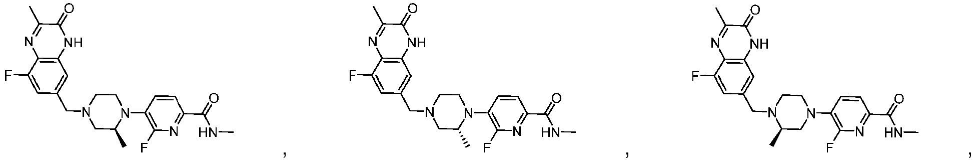

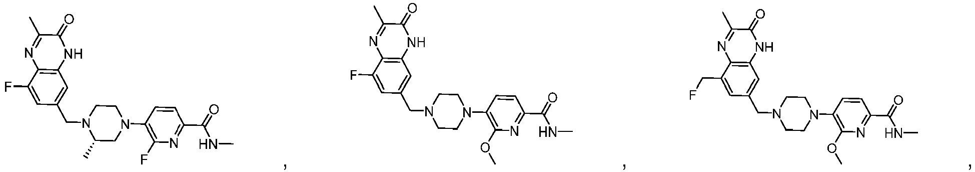

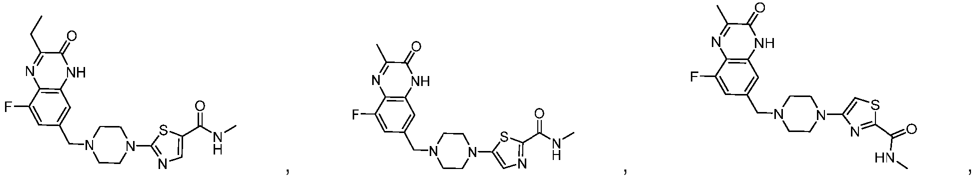

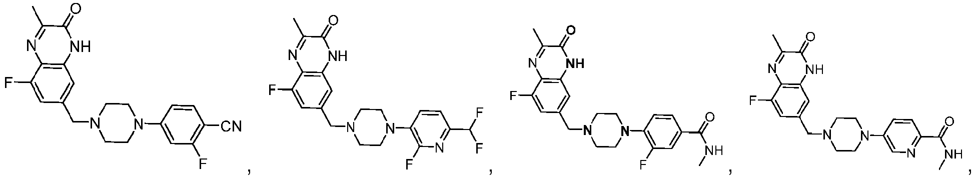

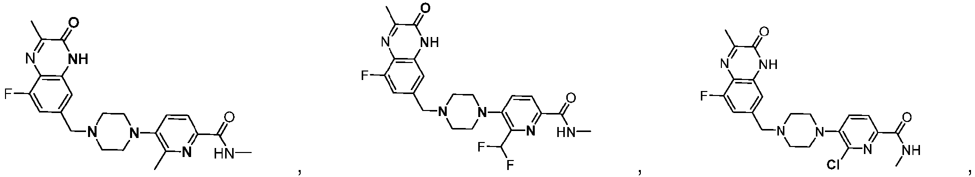

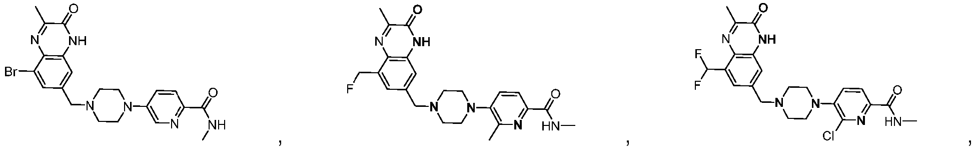

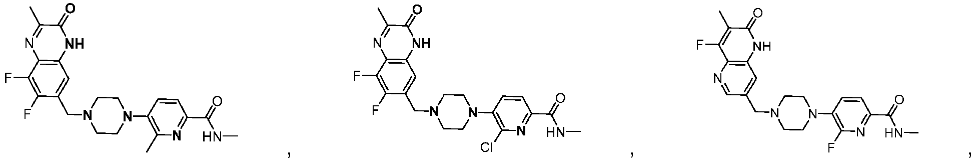

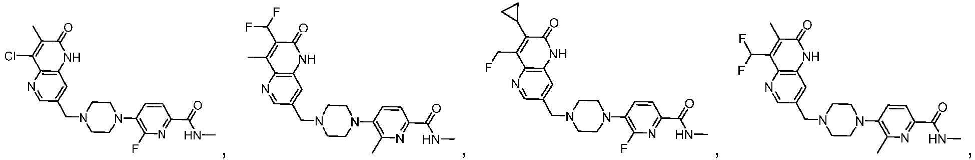

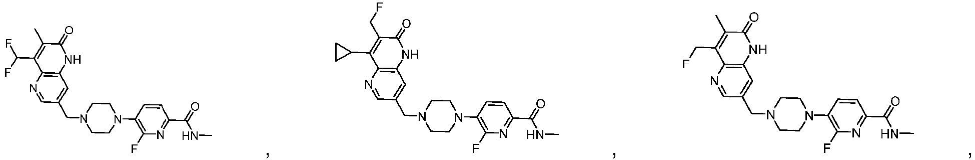

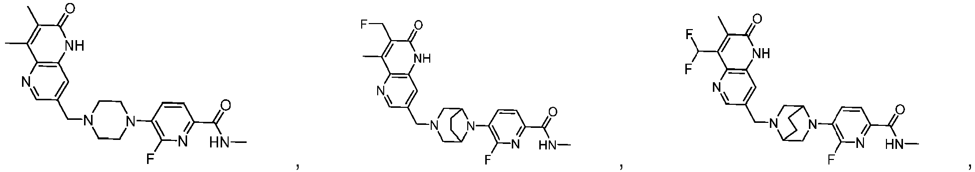

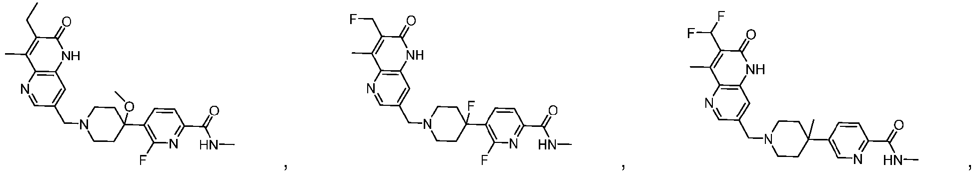

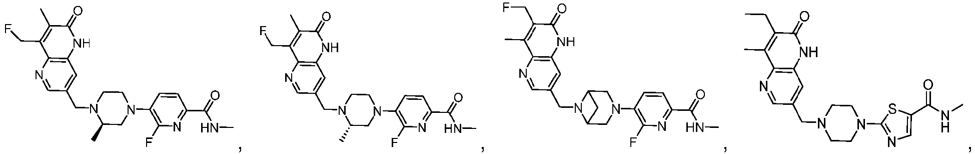

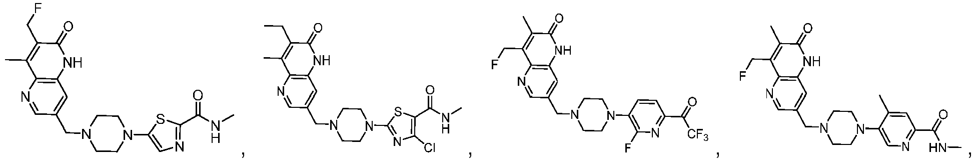

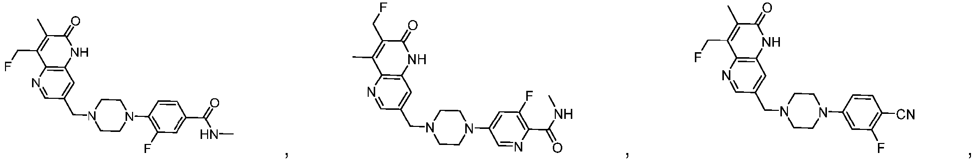

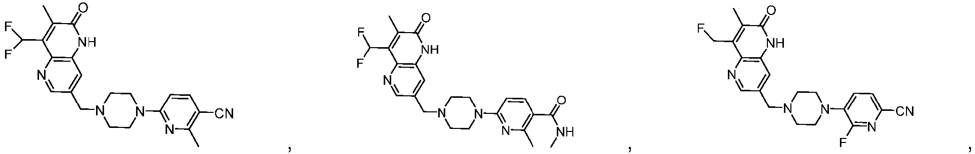

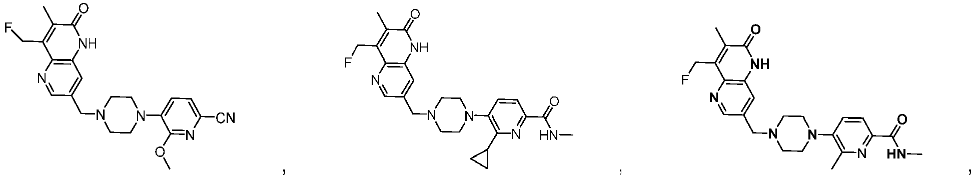

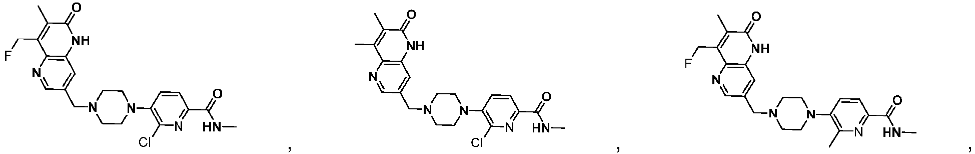

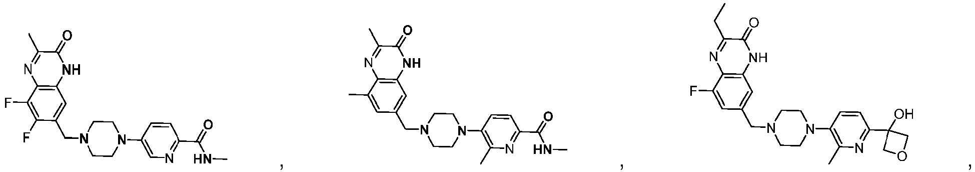

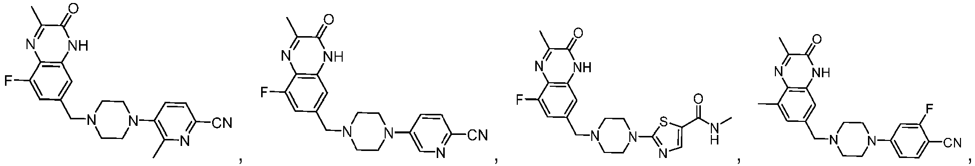

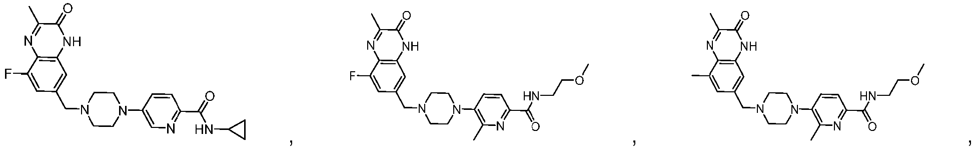

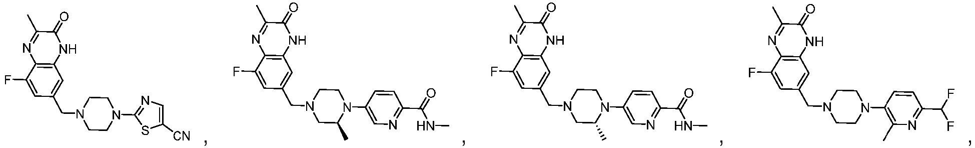

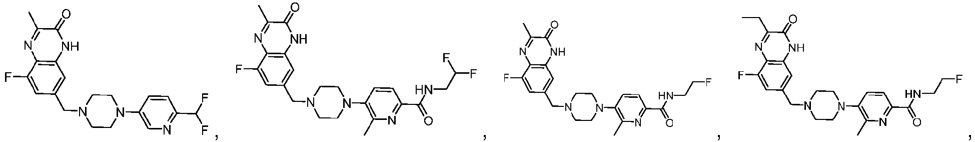

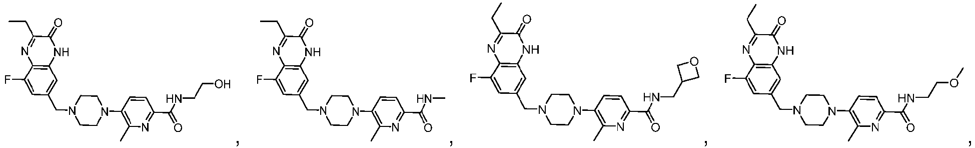

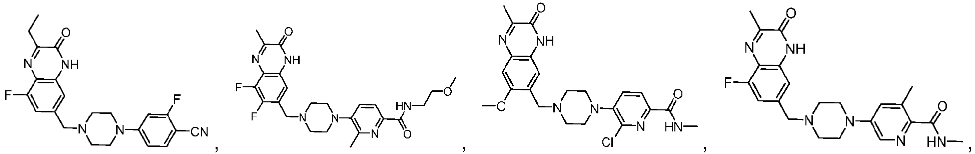

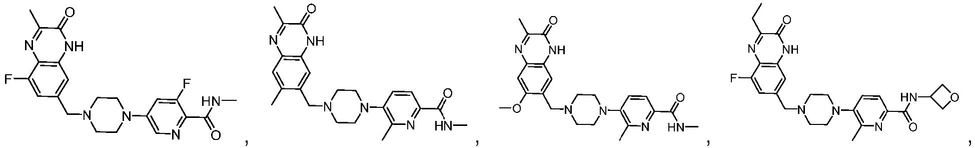

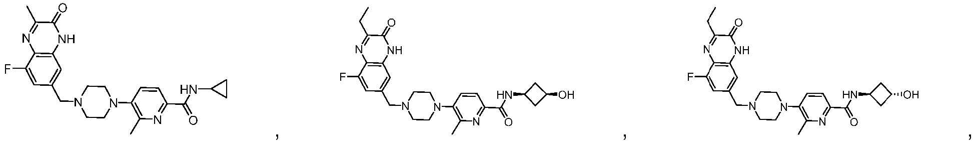

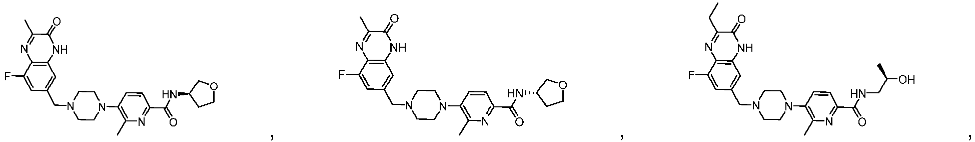

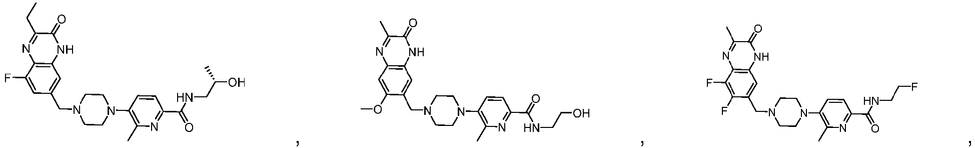

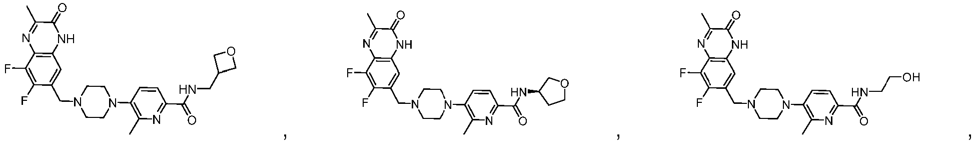

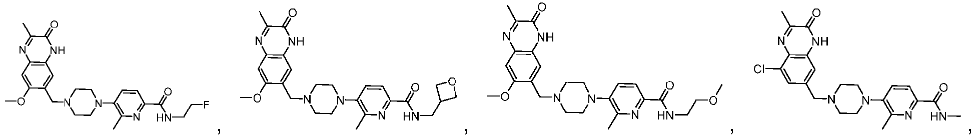

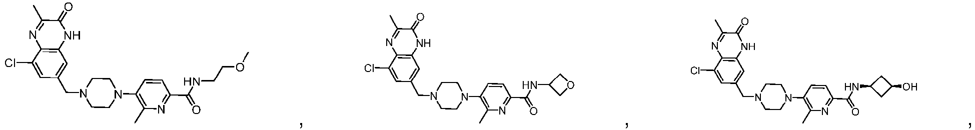

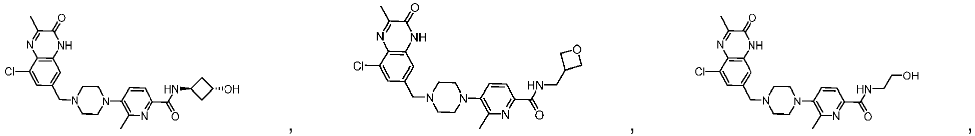

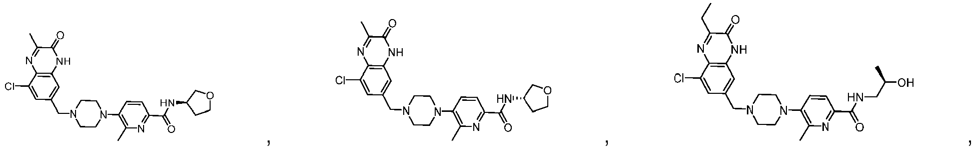

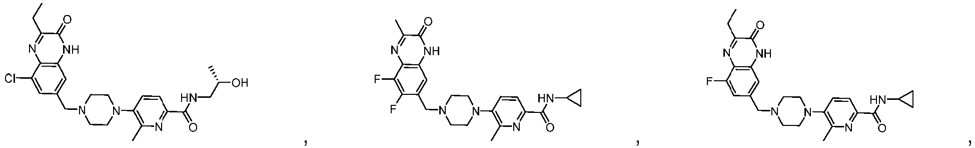

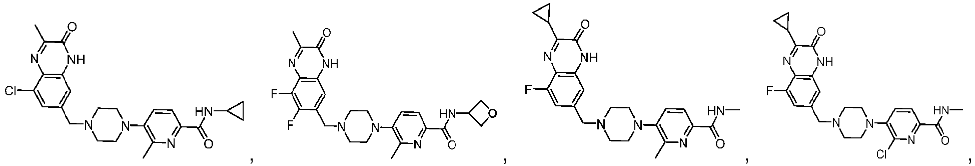

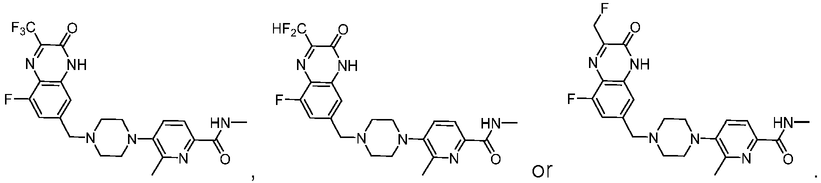

In another aspect, the present application further relates to a pharmaceutical composition, comprising: an effective dose of any one of the compounds of the present application or the pharmaceutically acceptable salt, the stereoisomer, the tautomer or the N-oxide thereof, and a pharmaceutically acceptable carrier and/or excipient; or further comprising one or more other therapeutic agents.

In another aspect, the present application provides use of the compound of Formula (I) or the pharmaceutically acceptable salt, the stereoisomer, the tautomer or the N-oxide thereof, or the pharmaceutical composition of the compound of the present application in the preparation of a PARP1 inhibitor.

In another aspect, the present application provides use of the compound of Formula (I) or the pharmaceutically acceptable salt, the stereoisomer, the tautomer or the N-oxide thereof in the preparation of a drug for treating cancer, wherein the cancer is a PARP1-mediated BRCA gene defective tumor.

In more detail, the present application provides use of the compound of Formula (I) or the pharmaceutically acceptable salt, the stereoisomer, the tautomer or the N-oxide thereof in the preparation of a drug for treating cancer or other diseases associated with PARP1, wherein the cancer is selected from breast cancer, ovarian cancer, pancreatic cancer, prostate cancer, colorectal cancer, bladder cancer, gastrointestinal cancer, lung cancer or blood cancer.

### Definition

Unless otherwise defined hereinafter, all technical terms and scientific terms used herein are intended to have the same meanings as commonly understood by the skilled in the art. References to technologies used herein are intended to refer to technologies commonly understood in the art, including variations of technologies or substitutions of equivalent technologies that are obvious to the skilled in the art. Although it is believed that the following terms are understood by the skilled in the art, the following definitions are still elaborated to better explain the present application.

Compounds described in the present application are named according to chemical structural formulas. If the naming of a compound does not match the chemical formula when representing the same compound, the chemical formula shall prevail.

The terms "comprise", "include", "have", "contain" or "relate to" and other variant forms thereof herein are inclusive or open-ended without excluding other elements or method steps that are not listed.

As used herein, the term "alkylene" represents a saturated bivalent hydrocarbon group, preferably represents a saturated bivalent hydrocarbon group having 1, 2, 3, 4, 5 or 6 carbon atoms, such as methylene, ethylidene, propylidene or butylidene.

As used herein, the term "alkyl" is defined as a linear or branched saturated aliphatic hydrocarbon. In some embodiments, the alkyl has 1 to 12, for example, 1 to 6, carbon atoms. For example, as used herein, the term "C₁₋₆ alkyl" refers to a linear or branched group having 1 to 6 carbon atoms (e.g., methyl, ethyl, n-propyl, isopropyl, n-butyl, isobutyl, sec-butyl, tert-butyl, n-pentyl, isopentyl, neopentyl or n-hexyl), which is optionally substituted with 1 or more (such as 1 to 3) suitable substituents such as halogen (the group in this case is referred to as "haloalkyl (halogenated alkyl)") (e.g., CH₂F, CHF₂, CF₃, CCl₃, CH₂CF₃, CH₂Cl or -CH₂CH₂CF₃, etc.). The term "C₁₋₄ alkyl" refers to a linear or branched aliphatic hydrocarbon chain having 1 to 4 carbon atoms (i.e., methyl, ethyl, n-propyl, isopropyl, n-butyl, isobutyl, sec-butyl or tert-butyl).

As used herein, the term "alkenyl" means a linear or branched monovalent hydrocarbon group, which comprises one double bond and has 2-5 carbon atoms (C₂₋₅ alkenyl). The alkenyl includes, for example, vinyl, 1-propenyl, 2-propenyl, 2-butenyl, 3-butenyl, 2-alkenyl, 3-pentenyl, 4-pentenyl, 2-hexenyl, 3-hexenyl, 4-hexenyl, 5-hexenyl, 2-methyl-2-propenyl, and 4-methyl-3-pentenyl. When the compound of the present application comprises alkenylene, the compound may exist in a pure E (entgegen) form, a pure Z (zusammen) form or any mixture form thereof.

As used herein, the term "alkynyl" means a linear or branched monovalent hydrocarbon group, which comprises one triple bond and has 2-5 carbon atoms (C₂₋₅ alkynyl). The alkynyl includes, for example, acetylenyl, 1-propynyl, 2-propynyl, 2-butynyl, 3-butynyl, 2-pentynyl, 3-pentynyl, 4-pentynyl, 2-hexynyl, 3-hexynyl, 4-hexynyl, 5-hexynyl, 2-methyl-2-propynyl, and 4-methyl-3-pentynyl.

As used herein, the term "cycloalkyl" refers to a saturated monocyclic or polycyclic (such as bicyclic) hydrocarbon ring (e.g., a monocyclic ring, such as cyclopropyl, cyclobutyl, cyclopentyl, cyclohexyl, cycloheptyl, cyclooctyl, cyclononyl, or a bicyclic ring, including a spirocyclic, fused or bridged system (such as bicyclo[1.1.1]pentyl, bicyclo[2.2.1]heptyl, bicyclo[3.2.1]octyl or bicyclo[5.2.0]nonyl, etc.)), which is optionally substituted with one or more (such as 1 to 3) suitable substituents. The cycloalkyl has 3 to 15 carbon atoms. For example, the term "C₃₋₆ cycloalkyl" refers to a saturated monocyclic or polycyclic (such as bicyclic) hydrocarbon ring having 3 to 6 ring forming carbon atoms (e.g., cyclopropyl, cyclobutyl, cyclopentyl or cyclohexyl), which is optionally substituted with 1 or more (such as to 3) suitable substituents, for example, cyclopropyl substituted with methyl.

As used herein, the terms "cyclohydrocarbylene" and "cyclohydrocarbyl" refer to a saturated (that is, "cycloalkylene" and "cycloalkyl") or unsaturated (that is, having one or more double bonds and/or triple bonds within a ring) monocyclic or polycyclic hydrocarbon ring having, for example, 3-10 (suitably having 3-8, more suitably having 3-6) cyclic carbon atoms, which includes but is not limited to cyclopropyl(ene), cyclobutyl(ene), cyclopentyl(ene), cyclohexyl(ene), cycloheptyl(ene), cyclooctyl(ene), cyclononyl(ene), cyclohexenyl(ene), etc.

As used herein, the terms "heterocyclyl" and "heterocyclylene" refer to a saturated (that is, heterocycloalkyl) or partially unsaturated (that is, having one or more double bonds and/or triple bonds within a ring) cyclic group having, for example, 3-10 (suitably having 3-8, more suitably having 3-6) cyclic atoms, in which at least one cyclic atom is a heteroatom selected from N, O, and S, and the remaining cyclic atoms are C. For example, "3-10 membered heterocyclyl(ene)" refers to a saturated or partially unsaturated heterocyclyl(ene) having 2-9 (e.g., 2, 3, 4, 5, 6, 7, 8 or 9) cyclic carbon atoms and one or more (e.g., 1, 2, 3 or 4) heteroatoms independently selected from N, O, and S. Examples of the heterocyclylene and the heterocyclyl include, but are not limited to: oxiranyl(ene), aziridinyl(ene), azetidinyl(ene), oxetanyl(ene), tetrahydrofuryl(ene), dioxolinyl(ene), pyrrolidinyl(ene), pyrrolidonyl(ene), imidazolidinyl(ene), pyrazolidinyl(ene), pyrrolinyl(ene), tetrahydropyranyl(ene), piperidyl(ene), morpholinyl(ene), dithianyl(ene), thiomorpholinyl(ene), piperazinyl(ene) or trithianyl(ene). The heterocyclylene and the heterocyclyl may be optionally substituted with one or more (e.g., 1, 2, 3 or 4) suitable substituents.

As used herein, the term "halo (halogenated)" or "halogen" group is defined as including F, Cl, Br or I.

As used herein, the term "nitrogen-containing heterocyclic ring" refers to a saturated or unsaturated monocyclic or bicyclic group, which has 2, 3, 4, 5, 6, 7, 8, 9, 10, 11, 12 or 13 carbon atoms and at least one nitrogen atom in the ring, may also optionally comprise one or more (e.g., one, two, three or four) ring members selected from N, O, C=O, S, S=O, and S(=O)₂, and the remaining portion of the molecule is connected through the nitrogen atom in the nitrogen-containing heterocyclic ring and any one of other cyclic atoms. The nitrogen-containing heterocyclic ring is optionally benzo-fused, and the remaining portion of the molecule is connected through the nitrogen atom in the nitrogen-containing heterocyclic ring and any one of carbon atoms in the fused benzene ring.

The term "substitution" means that one or more (e.g., one, two, three or four) hydrogens on a specified atom are replaced by a selection from a specified group, under the provision that the normal valence of the specified atom is not exceeded in the current instance and that the substitution forms a stable compound. Combinations of substituents and/or variables are permitted only when such combinations form stable compounds.

If substituents are described as "each independently selected from" one group, each substituent is selected independently of the other. Thus, each substituent may be the same as or different from another (other) substituent.

As used herein, the term "one or more" means 1 or more than 1, for example, 2, 3, 4, 5 or 10, under reasonable conditions.

Unless indicated, as used herein, connecting points of substituents may derive from any suitable position of the substituents.

When a bond of a substituent is shown as passing through a bond connecting two atoms in a ring, such substituent may be bonded to any one of ring forming atoms of the substitutable ring.

The present application further includes all pharmaceutically acceptable isotope-labeled compounds that are identical to the compounds of the present application, except that one or more atoms are replaced by atoms having same atomic numbers but different atomic masses or mass numbers from atoms having prevailing atomic masses or mass numbers in nature. Examples of isotopes suitable for inclusion in the compounds of the present application include (but are not limited to) isotopes of hydrogen (e.g., deuterium (²H) and tritium (³H)); isotopes of carbon (e.g., ¹¹C, ¹³C, and ¹⁴C); isotopes of chlorine (e.g., ³⁶Cl); isotopes of fluorine (e.g., ¹⁸F); isotopes of iodine (e.g., ¹²³I and ¹²⁵I); isotopes of nitrogen (e.g., ¹³N and ¹⁵N); isotopes of oxygen (e.g., ¹⁵O, ¹⁷O, and ¹⁸O); isotopes of phosphorus (e.g., ³²P); and isotopes of sulfur (e.g., ³⁵S).

The term "stereoisomer" represents an isomer formed due to at least one asymmetric center. In compounds having one or more (e.g., one, two, three or four) asymmetric centers, racemic mixtures, single enantiomers, diastereoisomer mixtures, and single diastereoisomer may be produced. Specific individual molecules may also exist in geometric isomers (cis/trans). Similarly, the compounds of the present application may exist in mixtures of two or more different structural forms in rapid equilibrium (commonly referred to as tautomers). Representative examples of tautomers include keto-enol tautomers, phenol-ketone tautomers, nitroso-oxime tautomers, imine-enamine tautomers, etc. It is understood that the scope of the present application encompasses all of such isomers or mixtures thereof in any proportion (e.g., 60%, 65%, 70%, 75%, 80%, 85%, 90%, 95%, 96%, 97%, 98% or 99%).

A solid line ( 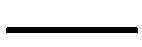 ), a solid wedge ( 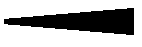 ) or a dashed wedge ( 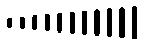 ) may be used herein to describe carbon-carbon bonds of the compounds of the present application. The solid line is used to describe a bond bonded to an asymmetric carbon atom, indicating that all possible stereoisomers at that carbon atom are included (e.g., specific enantiomers, racemic mixtures, etc.). The solid or dashed wedge is used to describe the presence of a stereoisomer shown as being bonded to an asymmetric carbon. When present in a racemic mixture, the solid and dashed wedges are used to define relative stereochemistry, rather than absolute stereochemistry. Unless otherwise indicated, the compounds of the present application are intended to exist in the forms of stereoisomers (including cis-isomers and trans-isomers, optical isomers (e.g., R and S enantiomers), diastereoisomers, geometric isomers, rotational isomers, atropisomers, and mixtures thereof). The compounds of the present application may exhibit more than one type of isomerism and consist of mixtures thereof (e.g., racemic mixtures and diastereoisomer pairs).

The present application encompasses all possible crystalline forms or polycrystalline forms of the compounds of the present application, which may be a single polycrystalline form or a mixture of more than one polycrystalline form in any proportion.

It should also be understood that some of the compounds of the present application may exist in a free form for treatment, or exist in the form of pharmaceutically acceptable derivatives thereof, as appropriate. In the present application, the pharmaceutically acceptable derivatives include, but are not limited to, pharmaceutically acceptable salts, esters, solvates, N-oxides, metabolites or prodrugs that can directly or indirectly provide the compounds of the present application or their metabolites or residues after being administered to patients in need thereof. Therefore, when being referred to herein, the "compounds of the present application" are also intended to encompass various derivative forms of the compounds described above.

The pharmaceutically acceptable salts of the compounds of the present application include acid addition salts and alkali addition salts thereof.

Suitable acid addition salts are formed from acids that form pharmaceutically acceptable salts. Examples include hydrochlorides, acetates, aspartates, benzoates, bicarbonates/carbonates, glucoheptonates, gluconates, nitrates, orotates, palmitates, and other similar salts.

Suitable alkali addition salts are formed from alkalies that form pharmaceutically acceptable salts. Examples include aluminum salts, arginine salts, choline salts, magnesium salts, and other similar salts.

For an overview of suitable salts, see Stahl and Wermuth, "Handbook of Pharmaceutical Salts: Properties, Selection and Use" (Wiley-VCH, 2002). Pharmaceutically acceptable salts used for preparing the compounds of the present application are known to the skilled in the art.

As used herein, the term "ester" means esters derived from each of the general formula compounds in the present application, including physiologically hydrolyzable esters (capable of being hydrolyzed under physiological conditions to release the compounds of the present application in the form of free acid or alcohol). The compounds of the present application themselves may also be esters.

The compounds of the present application may exist in the form of solvates (preferably hydrates), wherein the compounds of the present application include polar solvents, especially, for example, water, methanol or ethanol, as structural elements of the crystal lattices of the compounds. The amount of polar solvents, especially water, may be present in a stoichiometric ratio or a non-stoichiometric ratio.

The skilled in the art will understand that since available lone pair electrons are required for oxidizing nitrogen into oxides, not all of nitrogen-containing heterocyclic rings are capable of forming N-oxides; the skilled in the art will recognize nitrogen-containing heterocyclic rings capable of forming N-oxides. The skilled in the art will also recognize that tertiary amines are capable of forming N-oxides. Synthesis methods for preparing N-oxides of heterocyclic rings and tertiary amines are well known to the skilled in the art. These include oxidation of heterocyclic rings and tertiary amines with peroxyacids such as peroxyacetic acid and m-chloroperoxybenzoic acid (MCPBA), hydrogen peroxide, alkyl hydroperoxides such as tert-butyl hydroperoxide, sodium perborate, and dioxirane such as dimethyl dioxirane. These methods for preparing N-oxides have been widely described and reviewed in literature, see, for example: T. L. Gilehrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392. A. R. Katritzky and A. J. Boulton, Eds., Academic Press.

Metabolites of the compounds of the present application, that is, substances formed in the body when the compounds of the present application are administered, are also included within the scope of the present application. Such products may be produced by, for example, oxidation, reduction, hydrolysis, amidation, deamidation, esterification, enzymolysis and the like of the compounds administered. Therefore, the present application includes the metabolites of the compounds of the present application, including compounds prepared by a method of contacting the compounds of the present application with mammals for a time sufficient to produce their metabolites.

The present application further includes prodrugs of the compounds of the present application within its scope, which are certain derivatives of the compounds of the present application that may themselves have minor pharmacological activity or no pharmacological activity and may be converted into the compounds of the present application with desired activity by, for example, hydrolytic cleavage, when being administered into or onto the body. Usually, such prodrugs are functional group derivatives of the compounds, which are easily converted in vivo into compounds with desired therapeutic activity. Other information about the use of prodrugs may refer to "Pro-drugs as Novel Delivery Systems", Vol. 14, ACS Symposium Series (T. Higuchi and V. Stella). The prodrugs of the present application may be prepared by, for example, replacing appropriate functional groups present in the compounds of the present application with certain moieties as "pro-moieties" known to the skilled in the art (e.g., as described in "Design of Prorugs", H. Bundgaard (Elsevier, 1985)).

The present application also encompasses the compounds of the present application containing protective groups. In any process of preparing the compounds of the present application, it may be necessary and/or desirable to protect sensitive groups or reactive groups on any related molecules, thereby forming chemical protection forms of the compounds of the present application. This may be achieved by conventional protective groups, for example, those protective groups described in T. W. Greene & P. G. M. Wuts, Protective Groups in Organic Synthesis, John Wiley & Sons, 1991. These reference documents are incorporated herein by reference. The protective groups may be removed at appropriate subsequent stages by methods known in the art.

The term "about" refers to within the range of ±10% of the stated numerical value, preferably within the range of ±5%, more preferably within the range of ±2%.

### DETAILED DESCRIPTION OF THE INVENTION

### EXAMPLES

The present application is further described in detail below in conjunction with the examples, but the present application is not limited thereto. Any equivalent substitutions in the art that are made in accordance with the disclosed contents of the present application fall within the protection scope of the present application.

The structure of the compound is determined by mass spectrometry (MS) or nuclear magnetic resonance (¹HNMR).

Nuclear magnetic resonance (¹HNMR) is measured with a BrukerAVANCE 400 nuclear magnetometer, the measuring solvent is deuterated chloroform (CDCl₃), the internal standard is tetramethylsilane (TMS), and the chemical shift is given in units of 10⁻⁶ (ppm).

Mass spectrometry (MS) is measured with a FINNIGAN LCQAd (ESI) mass spectrometer (manufacturer: Therm, model: Finnigan LCQ advantage MAX).

Yantai Huanghai HSGF254 or Qingdao GF254 silica gel plate is used as a thin layer silica gel.

Yantai Huanghai silica gel, 200-300 mesh silica gel, is generally used as a carrier in column chromatography.

Unless otherwise specified in the present application, reactions referred to in the present application are all carried out under N₂ protection or under nitrogen atmosphere.

The term "N₂ protection" or "nitrogen atmosphere" in the present application refers to, for example, connecting a reaction flask to a nitrogen balloon with a volume of 1 L.

Unless otherwise specified in the present application, solutions referred to in reactions of the present application are aqueous solutions.

The term "room temperature" in the present application refers to a temperature between 10°C and 25°C.

Abbreviations as used herein have the following meanings:

| Abbrevi ation | Meaning | Abbrevi ation | Meaning |
|---|---|---|---|
| EtOAc | Ethyl acetate | DCM | Dichloromethane |
| Ruphos Pd G3 | (2-dicyclohexylphosphino-2',6'-diisopropoxy-1,1'-biphenyl)(2-amino-1,1' -biphenyl-2-yl)palladium (II) methanesulfonate | Pd(dppf )Cl₂ | [1,1'-bis(diphenylphosphino)ferrocene]di chloropalladium(II) |
| Cs₂CO₃ | Cesium carbonate | NaIO₄ | Sodium periodate |
| XPhosP dG₂ | Chloro(2-dicyclohexylphosphino-2',4',6'-triisopropyl-1,1'-biphenyl)[2-(2'-a mino-1,1'-biphenyl)]palladium(II) | DDO | 2,3-dichloro-5,6-dicyanobenzoquinone |
| NaBH₄ | Sodium borohydride | ACN | Acetonitrile |
| DIPEA | Diisopropylethylamine | HATU | (7-azabenzotriazol)-N,N,N',N'-tetrameth yluronium hexafluorophosphate |
| BAST | Bis(2-methoxyethyl)aminosulfur trifluoride | DMF | N,N-dimethylformamide |
| DMSO | Dimethyl sulfoxide | DIBAL-H | Diisobutylaluminum hydride |

### Example A1: Preparation of dihydrochloride of intermediate N-methyl-5-(piperazin-1-yl)picolinamide (Int-1)

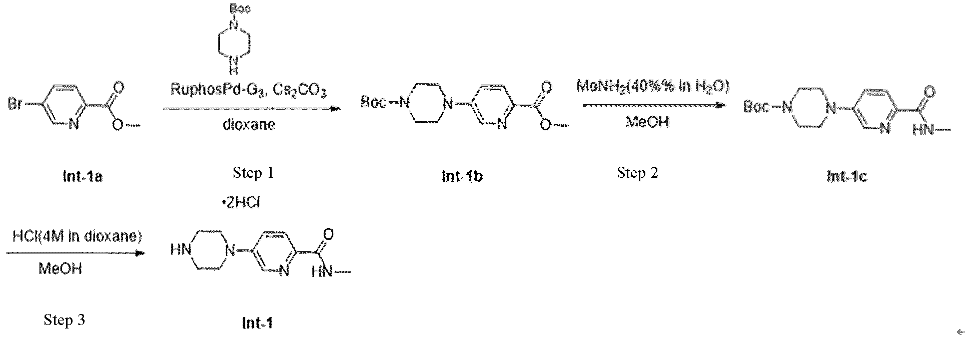

### Step 1: Preparation of compound tert-butyl 4-(6-(methoxycarbonyl)pyridin-3-yl)piperazin-1-carboxylate (Int-1b)

Compound **Int-1a** (10.0 g, 46.3 mmol), tert-butyl piperazin-1-carboxylate (9.07 g, 48.6 mmol) and Cs₂CO₃ (30.0 g, 92.6 mmol) were weighed into a round-bottomed flask, 1,4-dioxane (180 mL) was added, Ruphos Pd G3 (1.36 g, 1.62 mmol) was added under N₂ protection, and after the addition was completed, the temperature was raised to 80°C for reaction. After the reaction was completed, water and ethyl acetate were added to the reaction system for dilution and liquid separation. Aqueous phase was extracted with ethyl acetate for three times. Organic phases were combined, dried with anhydrous sodium sulfate and subjected to suction filtration. Filtrate was concentrated to obtain a crude product of compound. The obtained crude product of compound was purified by column chromatography to obtain the title compound **Int-1b** (8.18 g, yield: 55%).

MS m/z (ES): 322.2 [M+H]⁺.

### Step 2: Preparation of compound tert-butyl 4-(6-(methyl carbamoyl)pyridin-3-yl)piperazin-1-carboxylate (Int-1c)

Compound **Int-1b** (12.1 g, 37.3 mmol) was dissolved in methanol (50 mL), then added to a 40% methylamine aqueous solution (33 mL, 0.38 mol) and stirred at room temperature. After the reaction was completed, the reaction system was concentrated, and residue was diluted with a saturated ammonium chloride solution and DCM for liquid separation. Aqueous phase was extracted with DCM for three times. Organic phases were combined, dried with anhydrous sodium sulfate and subjected to suction filtration. Filtrate was concentrated to obtain a crude product of compound. The obtained crude product was purified by column chromatography to obtain the title compound **Int-1c** (12.0 g, yield: 100%).

MS m/z (ES): 321.2 [M+H]⁺.

### Step 3: Preparation of compound N-methyl-5-(piperazin-1-yl)picolinamide (Int-1)

Compound **Int-1c** (12.0 g, 37.3 mmol) was dissolved in MeOH (100 mL), and a 1,4-dioxane solution (4 M, 50 mL, 200 mmol) of HCl was added at 0°C, then slowly warmed up to room temperature and stirred. After the reaction was completed, ethyl ether was added to the reaction system for dilution. A solid was precipitated out of the system, filtered and washed with ethyl ether. The solid was collected and subjected to vacuum drying to obtain dihydrochloride of the title compound Int-1 (11.1 g, yield: 99%).

MS m/z (ES): 221.1 [M+H]⁺.

### Example A2: Preparation of hydrochloride of intermediate 5-(3,6-diazabicyclo[3.1.1]heptan-3-yl)-N,6-dimethylpicolinamide (Int-A2)

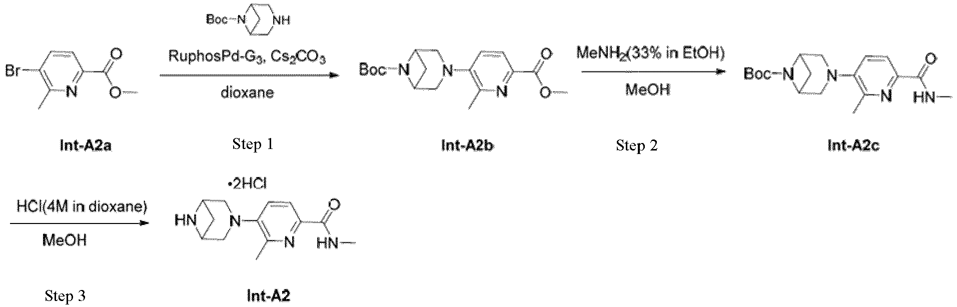

### Step 1: Preparation of compound tert-butyl 3-(6-(methoxycarbonyl)-2-methylpyridin-3-yl)-3,6-diazabicyclo[3.1.1]heptan-6-car boxylate (Int-A2b)

Except that compound **Int-A2a** was used to replace compound **Int-1a** in Step 1 of Example A1, a crude product of compound **Int-A2b** was synthesized by a method similar to that described in Step 1 of Example A1. The obtained crude product was purified by column chromatography to obtain the title compound **Int-A2b** (1.90 g, yield: 79%).

MS m/z (ES): 348.2 [M+H]⁺.

### Step 2: Preparation of compound tert-butyl 3-(2-methyl-6-(methylcarbamoyl)pyridin-3-yl)-3,6-diazabicyclo[3.1.1]heptan-6-car boxylate (Int-A2c)

Compound **Int-A2b** (1.90 g, 5.48 mmol) was dissolved in methanol (10 mL), then a 33% methylamine ethanol solution (5 mL, 54.8 mol) was added and stirred at room temperature. After the reaction was completed, the reaction system was concentrated to obtain a crude product of the title compound **Int-A2c** (1.83 g), and the obtained crude product was directly used in the next step without further purification.

MS m/z (ES): 347.1 [M+H]⁺.

### Step 3: Preparation of compound 5-(3,6-diazabicyclo[3.1.1]heptan-3-yl)-N,6-dimethylpicolinamide (Int-A2)

Except that compound **Int-A2c** was used to replace compound **Int-1c** in Step 3 of Example A1, a crude product of compound **Int-A2** was synthesized by a method similar to that described in Example A1. Ethyl ether was added to the crude product for dilution. A solid was precipitated out of the system, filtered and washed with ethyl ether. The solid was collected and subjected to vacuum drying to obtain hydrochloride of the title compound **Int-A2** (1.24 g, two-step yield: 92%).

MS m/z (ES): 247.2 [M+H]⁺.

### Example A3: Preparation of intermediate 5-((1R,4R)-2,5-diazabicyclo[2.2.1]heptan-2-yl)-N,6-dimethylpicolinamide (Int-A3)

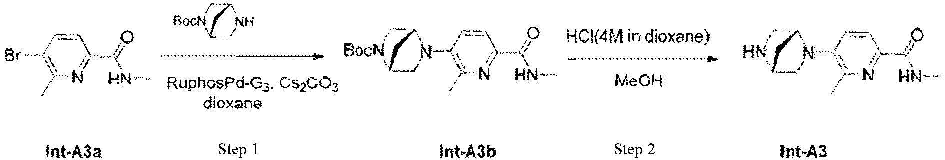

### Step 1: Preparation of compound tert-butyl (1R,4R)-5-(2-methyl-6-(methyl carbamoyl)pyridin-3-yl)-2,5-diazabicyclo[2.2.1]heptan-2-carboxylate (Int-A3b)

Compound **Int-A3a** (3.0 g, 13.1 mmol), tert-butyl (1R,4R)-2,5-diazabicyclo[2.2.1]heptan-2-carboxylate (2.8 g, 14.4 mmol) and Cs₂CO₃ (12.1 g, 39.3 mmol) were weighed into a round-bottomed flask, 1,4-dioxane (20 mL) was added, Ruphos Pd G3 (0.5 g, 0.65 mmol) was added under N₂ protection, and after the addition was completed, the temperature was raised to 90°C for reaction. After the reaction was completed, water and ethyl acetate were added to the reaction system for dilution and liquid separation. Aqueous phase was extracted with ethyl acetate for three times. Organic phases were combined, dried with anhydrous sodium sulfate and subjected to suction filtration. Filtrate was concentrated to obtain a crude product of compound. The obtained crude product of compound was purified by column chromatography to obtain the title compound **Int-A3b** (3.8 g, yield: 84%).

MS m/z (ES): 347.1 [M+H]⁺.

### Step 2: Preparation of compound 5-((1R,4R)-2,5-diazabicyclo[2.2.1]heptan-2-yl)-N,6-dimethylpicolinamide (Int-A3)

Compound **Int-A3b** (200 mg, 0.57 mmol) was dissolved in a 1,4-dioxane solution (2 mL), and a 1,4-dioxane solution (4 M, 1.5 mL, 5.7 mmol) of HCl was added thereto at 0°C, then slowly warmed up to room temperature and stirred. After the reaction was completed, petroleum ether was added to the reaction system for dilution. A solid was precipitated out of the system, filtered and washed with petroleum ether. The solid was collected and subjected to vacuum drying to obtain hydrochloride of the title compound **Int-A3** (123 mg, yield: 86%).

MS m/z (ES): 247.1 [M+H]⁺.

¹H NMR (400 MHz, DMSO-*d*₆) δ 9.82 (s, 1H), 9.26 (s, 1H), 8.66 (s, 1H), 7.87 (d, *J* = 8.4 Hz, 1H), 7.44 (d, *J* = 8.4 Hz, 1H), 4.56 (s, 1H), 4.39 (s, 1H), 3.75 (d, *J* = 10.0 Hz, 1H), 3.60 (d, *J =* 10.4 Hz, 1H), 3.40 - 3.30 (m, 1H), 3.30 - 3.18 (m, 1H), 2.81 (d, *J* = 4.4 Hz, 3H), 2.55 (s, 3H), 2.11 (d, *J* = 10.8 Hz, 1H), 1.98 (d, *J* = 10.8 Hz, 1H).

Referring to the synthesis methods in Example A1, A2 or A3, hydrochlorides of the following intermediate compounds were synthesized by using appropriate aryl halides and amino compounds to replace the raw materials and amino compounds in the reactions of Step 1 under similar reaction conditions.

| Intermediate number | Hydrochloride of intermediate | Reference example | LCMS |
|---|---|---|---|
| Int-A4 | | Example A3 | m/z (ES) :247.1[M+H]+ |
| Int-A5 | | Example A2 | m/z (ES) :235.1[M+H]⁺ |
| Int-A6 | | Example A2 | m/z (ES) :233.1[M+H]⁺ |
| Int-A7 | | Example A2 | m/z (ES) :235.1[M+H]⁺ |
| Int-A10 | | Example A2 | m/z (ES) :251.1[M+H]⁺ |
| Int-A11 | | Example A2 | m/z (ES) :235.1[M+H]⁺ |
| Int-A12 | | Example A2 | m/z (ES) :227.0[M+H]⁺ |
| Int-A16 | | Example A3 | m/z (ES) :214.1[M+H]⁺ |
| Int-A18 | | Example A3 | m/z (ES) :203.1[M+H]⁺ |
| Int-A19 | | Example A3 | m/z (ES) :189.1[M+H]⁺ |
| Int-A37 | | Example A2 | m/z (ES) :239.1[M+H]⁺ |

### Example A4: Preparation of intermediate 6-chloro-N-methyl-5-(piperazin-1-yl)picolinamide (Int-A8)

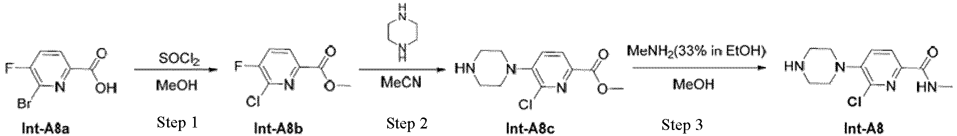

### Step 1: Preparation of compound methyl 6-chloro-5-fluoropicolinate (Int-A8b)

Compound **Int-A8a** (5.0 g, 22.7 mmol) was weighed into a round-bottomed flask, methanol (25 mL) was added for dissolution, SOCl₂ (8 ml, 113.6 mmol) was added under an ice bath, and after the addition was completed, the reaction was carried out at room temperature. After the reaction was completed, the reaction solution was concentrated to dryness, and ice water was slowly added for quenching the reaction. Aqueous phase was extracted with dichloromethane for three times. Organic phases were combined, dried with anhydrous sodium sulfate and subjected to suction filtration. Filtrate was concentrated to obtain a crude product of compound **Int-A8b** (4.2 g, yield: 97%).

MS m/z (ES): 190.0 [M+H]⁺.

### Step 2: Preparation of compound methyl 6-chloro-5-(piperazin-1-yl)picolinate (Int-A8c)

Compound **Int-A8b** (1.45 g, 7.57 mmol) was dissolved in acetonitrile (50 mL), then anhydrous piperazine (1.8 g, 21.0 mmol) was added, and after the additionwas completed, the reaction was carried out at 80°C. After the reaction was completed, the reaction system was concentrated to obtain a crude product of compound **Int-A8c,** and the obtained crude product was directly used in the next step without further purification.

MS m/z (ES): 256.1 [M+H]⁺.

### Step 3: Preparation of compound 6-chloro-N-methyl-5-(piperazin-1-yl)picolinamide (Int-A8)

The crude product of compound **Int-A8c** in the above step was dissolved in EtOH (10 mL), then a 33% methylamine ethanol solution (9.8 mL, 78 mmol) was added and stirred at room temperature for reaction. After the reaction was completed, concentration was performed to obtain the title compound **Int-A8** (1.9 g, two-step yield: 98%).

MS m/z (ES): 255.1 [M+H]⁺.

### Example A5: Preparation of intermediate 3-(6-methyl-5-(piperazin-1-yl)pyridin-2-yl)oxetan-3-ol (Int-A9)

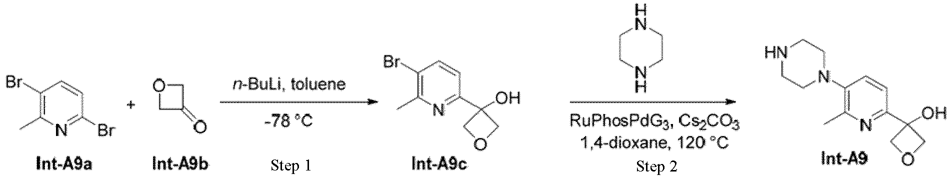

### Step 1: Preparation of compound 3-(5-bromo-6-methylpyridin-2-yl)oxetan-3-ol (Int-A9c)

Compound **Int-A9a** (2.0 g, 8.0 mmol) was dissolved in toluene (25 mL) and cooled to -78°C, n-butyllithium (1.6 M in hexane, 5 mL, 8.0 mmol) was slowly added dropwise under nitrogen atmosphere, continuous stirring was performed at -78°C for 20 min after the addition was completed, and a toluene (3 mL) solution of **Int-A9b** (577 mg, 8.0 mmol) was added. The reaction system continued to react at this temperature for 1 h. After the reaction was completed as monitored by LCMS, a saturated NH₄Cl solution (15 mL) was added to the reaction system for quenching the reaction, and then extraction was performed with ethyl acetate for three times. Organic phases were combined, dried with anhydrous sodium sulfate and filtered. Filtrate was concentrated to obtain a crude product of compound. The obtained crude product was purified by column chromatography to obtain the title compound **(Int-A9c)** (1.76 g, yield: 90%).

MS m/z (ES): 244.0 [M+H]⁺.

### Step 2: Preparation of compound 3-(6-methyl-5-(piperazin-1-yl)pyridin-2-yl)oxetan-3-ol (Int-A9)

Compound **Int-A9c** (500 mg, 2.1 mmol), piperazine (361 mg, 4.2 mmol), RuPhosPdG₃ (176 mg, 0.21 mmol) and Cs₂CO₃ (2.05 g, 6.3 mmol) were dissolved in 1 ,4-dioxane (20 mL), and the reaction system was placed at 120°C to react for 4 h under nitrogen atmosphere. When the reaction was completed as monitored by LCMS, water was added for quenching the reaction, and then extraction was performed with ethyl acetate for three times. Organic phases were combined, dried with anhydrous sodium sulfate and filtered. Filtrate was concentrated to obtain a crude product of compound. The obtained crude product was purified by column chromatography to obtain the title compound **(Int-A9)** (60 mg, yield: 11%).

MS m/z (ES): 250.1 [M+H]⁺.

### Example A6: Preparation of hydrochloride of intermediate N-methyl-5-(piperazin-1-yl)thiazol-2-amide hydrochloride (Int-A13)

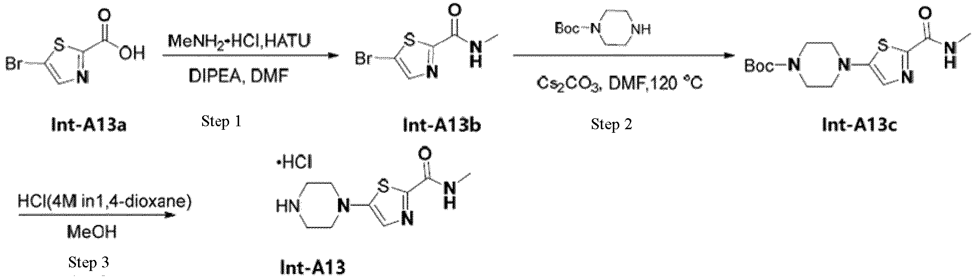

### Step 1: Preparation of compound 5-bromo-N-methylthiazol-2-formamide (Int-A13b)

Compound **Int-A13a** (3.0 g, 14.4 mmol) and methylamine hydrochloride (1.46 g, 21.6 mmol) were dissolved in N,N-dimethylformamide (30 mL), DIPEA (4.64 g, 36.0 mmol) and HATU (13.69 g, 36.0 mmol) were added at 0°C, and the reaction system was warmed up to room temperature for reaction. When the reaction was completed as monitored by LCMS, water (50 mL) was added for quenching the reaction, and then extraction was performed with ethyl acetate for three times. Organic phases were combined, dried with anhydrous sodium sulfate and filtered. Filtrate was concentrated to obtain a crude product of compound. The obtained crude product was purified by column chromatography to obtain the title compound **(Int-A13b)** (2.5 g, yield: 79%).

MS m/z (ES): 220.9 [M+H]⁺.

### Step 2: Preparation of compound tert-butyl 4-(2-(methyl carbamoyl)thiazol-5-yl)piperazin-1-carboxylate (Int-A13c)

Compound **Int-A13b** (300 mg, 1.36 mmol), tert-butyl piperazin-1-carboxylate (454 mg, 2.04 mmol) and cesium carbonate (1.33 g, 4.08 mmol) were dissolved in N,N-dimethylformamide (5 mL), and the reaction system was heated to 120°C and stirred. When the reaction was completed as monitored by LCMS, water (10 mL) was added thereto for quenching the reaction, and then extraction was performed with ethyl acetate for three times. Organic phases were combined, dried with anhydrous sodium sulfate and filtered. Filtrate was concentrated to obtain a crude product of compound. The obtained crude product was purified by column chromatography to obtain the title compound **Int-A13c** (70 mg, yield: 16%).

MS m/z (ES): 327.0 [M+H]⁺.

### Step 3: Preparation of hydrochloride of compound N-methyl-5-(piperazin-1-yl)thiazol-2-amide (Int-A13)

Compound **Int-A13c** (70 mg, 0.21 mmol) was dissolved in MeOH (5 mL), and a 1,4-dioxane solution (4 M, 0.53 mL, 2.1 mmol) of HCl was added and stirred under room temperature conditions. After the reaction was completed as monitored by LCMS, the reaction solution was concentrated to obtain a crude product of the title compound **Int-A13** (60 mg), and the obtained crude product was directly used in the next reaction without further purification.

MS m/z (ES): 227.0 [M+H]⁺.

### Example A7: Preparation of hydrochloride of intermediate N-methyl-2-(piperazin-1-yl)thiazol-5-amide hydrochloride (Int-A14)

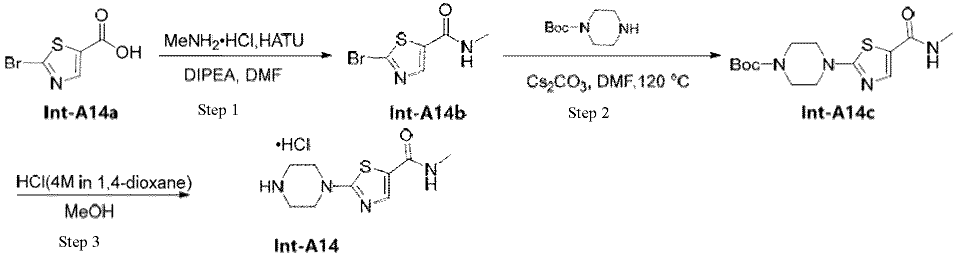

### Step 1 to Step 3: Preparation of hydrochloride of intermediate N-methyl-2-(piperazin-1-yl)thiazol-5-amide (Int-A14)

Except that compound **Int-A14a** was used to replace compound **Int-A13a** in Step 1 of Example A6, a crude product of compound **Int-A14** was synthesized by a method similar to that described in Step 1 to Step 3 of Example A6. The obtained crude product was directly used in the next reaction without further purification.

MS m/z (ES): 227.0 [M+H]⁺.

### Example A8: Preparation of hydrochloride of intermediate N-cyclopropyl-5-(piperazin-1-yl)picolinamide (Int-A15)

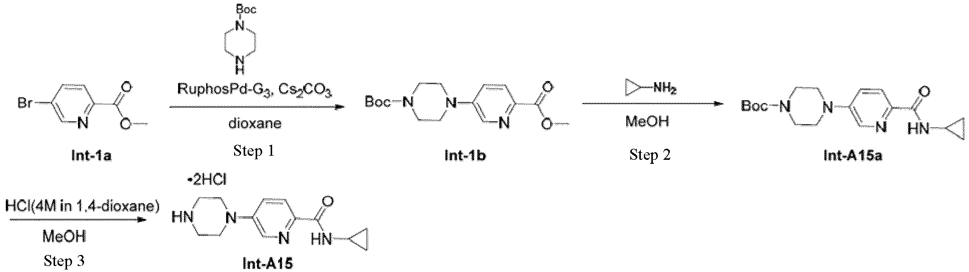

### Step 1 to Step 3: Preparation of hydrochloride of intermediate N-cyclopropyl-5-(piperazin-1-yl)picolinamide (Int-A15)

Except that compound cyclopropylamine was used to replace the 40% methylamine aqueous solution in Step 2 of Example A1, a crude product of compound **Int-A15** was synthesized by a method similar to that described in Step 1 to Step 3 of Example A1. The obtained crude product was directly used in the next reaction without further purification.

MS m/z (ES): 247.1 [M+H]⁺.

### Example A9: Preparation of hydrochloride of intermediate 1-(6-(difluoromethyl)-2-methylpyridin-3-yl)piperazine (Int-A17)

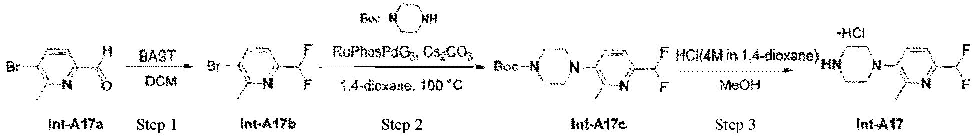

### Step 1: Preparation of compound 3-bromo-6-(difluoromethyl)-2-methylpyridine (Int-A17b)

Compound **Int-A17a** (300 mg, 1.50 mmol) was dissolved in DCM (20 mL), and then BAST (0.69 mL, 3.75 mmol) was added dropwise. Then, the reaction system was heated to 40°C and stirred. When the reaction was completed as monitored by LCMS, a saturated sodium bicarbonate aqueous solution (50 mL) was added thereto for quenching the reaction and for liquid separation. Aqueous phase was extracted with dichloromethane for three times. Organic phases were combined, dried with anhydrous sodium sulfate and filtered. Filtrate was concentrated to obtain a crude product of the title compound **Int-A17b.** The obtained crude product was directly used in the next reaction without further purification.

MS m/z (ES): 221.9 [M+H]⁺.

### Step 2 to Step 3: Preparation of hydrochloride of intermediate 1-(6-(difluoromethyl)-2-methylpyridin-3-yl)piperazine (Int-A17)

Except that compound **Int-A17b** was used to replace compound **Int-A3a** in Step 1 of Example A3 and tert-butyl piperazin-1-carboxylate was used to replace compound tert-butyl (1R,4R)-2,5-diazabicyclo[2.2.1]heptan-2-carboxylate in Step 1 of Example A3, a crude product of hydrochloride of the title compound **Int-A17** was synthesized by a method similar to that described in Step 1 to Step 2 of Example A3. The obtained crude product was directly used in the next reaction without further purification.

MS m/z (ES): 228.1 [M+H]⁺.

### Example A10: Preparation of hydrochloride of intermediate 2-fluoro-4-(piperazin-1-yl)benzonitrile (Int-A20)

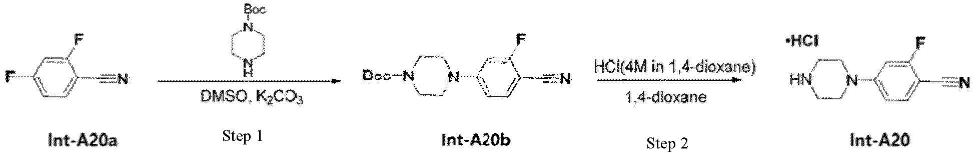

### Step 1: Preparation of compound tert-butyl 4-(4-cyano-3-fluorophenyl)piperazin-1-carboxylate

Compound **Int-A20a** (1.1 g, 7.9 mmol), tert-butyl piperazin-1-carboxylate (1.0 g, 5.3 mmol) and K₂CO₃ (1.1 g, 7.9 mmol) were weighed into a round-bottomed flask, DMSO (20 mL) was added, and after the addition was completed, the temperature was raised to 100°C to react overnight. After the reaction was completed, water and ethyl acetate were added to the reaction system for dilution and liquid separation. Aqueous phase was extracted with ethyl acetate for three times. Organic phases were combined, dried with anhydrous sodium sulfate and subjected to suction filtration. Filtrate was concentrated to obtain a crude product of compound **Int-A20b** (2.2 g, yield: 50%). The obtained crude product was directly used in the next step without further purification.

MS m/z (ES): 306.1 [M+H]⁺.

### Step 2: Preparation of compound 2-fluoro-4-(piperazin-1-yl)benzonitrile (Int-A20)

Compound **Int-A20b** (2.2 g, 7.2 mmol) was dissolved in a 1,4-dioxane solution (10 mL), and a 1,4-dioxane solution (4 M, 15 mL, 60 mmol) of HCl was added thereto at 0°C, then slowly warmed up to room temperature and stirred. After the reaction was completed, petroleum ether was added to the reaction system for dilution. A solid was precipitated out of the system, filtered and washed with petroleum ether. The solid was collected and subjected to vacuum drying to obtain hydrochloride of the title compound **Int-A20** (1.5 g, yield: 86%).

MS m/z (ES): 206.1 [M+H]⁺.

### Example A11: Preparation of hydrochloride of intermediate 2-(piperazin-1-yl)thiazol-5-nitrile (Int-A21)

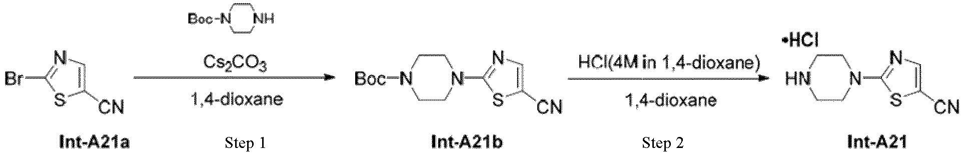

### Step 1: Preparation of compound tert-butyl 4-(5-cyanothiazol-2-yl)piperazin-1-carboxylate (Int-A21b)

Compound **Int-A21a** (2.1 g, 11.0 mmol), tert-butyl piperazin-1-carboxylate (2.5 g, 13.1 mmol) and cesium carbonate (10.7 g, 32.9 mmol) were weighed into a round-bottomed flask, 1 ,4-dioxane (30 mL) was added, and the reaction system was heated to 110°C to react overnight. After the reaction was completed, water and ethyl acetate were added to the reaction system for dilution and liquid separation. Aqueous phase was extracted with ethyl acetate for three times. Organic phases were combined, dried with anhydrous sodium sulfate and subjected to suction filtration. Filtrate was concentrated to obtain a crude product of compound. The obtained crude product was purified by silica gel column to obtain the title compound **Int-A21b** (2.6 g, yield: 80%).

MS m/z (ES): 295.1 [M+H]⁺.

### Step 2: Preparation of intermediate 2-(piperazin-1-yl)thiazol-5-nitrile (Int-A21)

Except that compound **Int-A21b** was used to replace **Int-A20b** in Step 2 of Example A10, a crude product of hydrochloride of the title compound **Int-A21** was synthesized by a method similar to that described in Step 2 of Example A10. The obtained crude product was directly used in the next reaction without further purification.

MS m/z (ES): 195.1 [M+H]⁺.

### Example A12: Preparation of hydrochloride of intermediate N-(2-methoxyethyl)-6-methyl-5-(piperazin-1-yl)picolinamide (Int-A22)

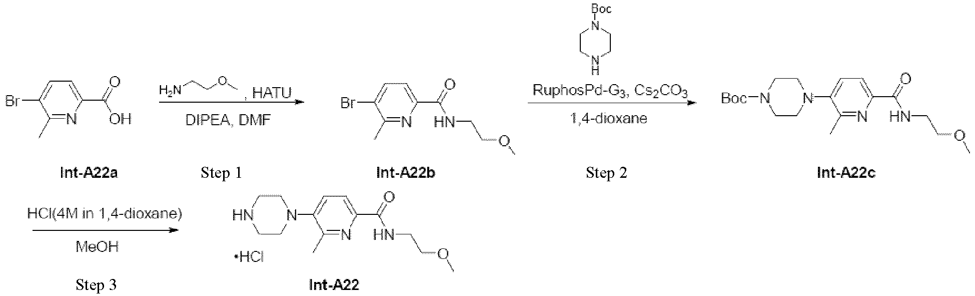

### Step 1: Preparation of compound 5-bromo-N-(2-methoxyethyl)-6-methylpicolinamide (Int-A22b)

Compound **Int-A22a** (5.0 g, 23.1 mmol), 2-methoxyethylamine (3.48 g, 46.3 mmol), HATU (17.6 g, 46.3 mmol) and DIPEA (5.97 g, 46.3 mmol) were dissolved in DMF (40 mL), and the reaction system was stirred at room temperature. When the reaction was completed as monitored by LCMS, water (50 mL) was added thereto for quenching the reaction, and then extraction was performed with ethyl acetate for three times. Organic phases were combined, dried with anhydrous sodium sulfate and filtered. Filtrate was concentrated to obtain a crude product of compound. The obtained crude product was purified by column chromatography to obtain the title compound **Int-A22b** (5.3 g, yield: 73%).

MS m/z (ES): 273.0 [M+H]⁺.

### Step 2 to Step 3: Preparation of intermediate N-(2-methoxyethyl)-6-methyl-5-(piperazin-1-yl)picolinamide (Int-A22)

Except that compound **Int-A22b** was used to replace **Int-A3a** in Step 1 of Example A3, a crude product of hydrochloride of the title compound **Int-A22** was synthesized by a method similar to that described in Step 1 to Step 2 of Example A3. The obtained crude product was directly used in the next reaction without further purification.

MS m/z (ES): 279.1 [M+H]⁺.

Referring to the synthesis method in Example A12, the following intermediate compounds were synthesized under similar reaction conditions.

| Intermediate number | Hydrochloride of intermediate | LCMS |
|---|---|---|
| Int-A23 | 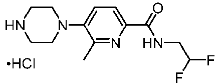 | m/z (ES) : 285.1[M+H]+ |
| Int-A24 | 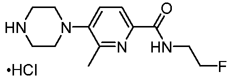 | m/z (ES) : 267.1[M+H]⁺ |
| Int-A25 | 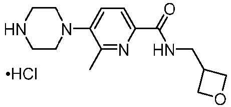 | m/z (ES) : 291.1[M+H]⁺ |
| Int-A26 | 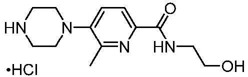 | m/z (ES) : 265.1[M+H]⁺ |
| Int-A27 | 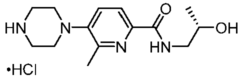 | m/z (ES) : 279.1 [M+H]⁺ |
| Int-A28 | 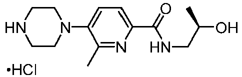 | m/z (ES) : 279.1[M+H]⁺ |
| Int-A29 | 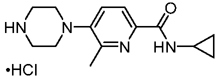 | m/z (ES) : 261.1[M+H]⁺ |
| Int-A30 | 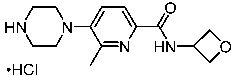 | m/z (ES) : 277.1[M+H]⁺ |
| Int-A31 | 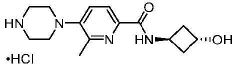 | m/z (ES) : 291.1[M+H]⁺ |
| Int-A32 | 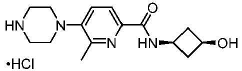 | m/z (ES) : 291.1[M+H]⁺ |
| Int-A33 | 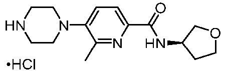 | m/z (ES) : 291.1[M+H]⁺ |
| Int-A34 | 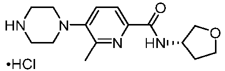 | m/z (ES) : 291.1[M+H]⁺ |
| Int-A35 | 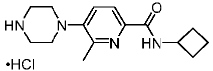 | m/z (ES) : 275.1[M+H]⁺ |
| Int-A36 | 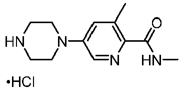 | m/z (ES) : 235.1[M+H]⁺ |

### Example B: Preparation of 7-(bromomethyl)-5-fluoro-3-methylquinoxalin-2(1H)-one (Int-2)

### Route 1:

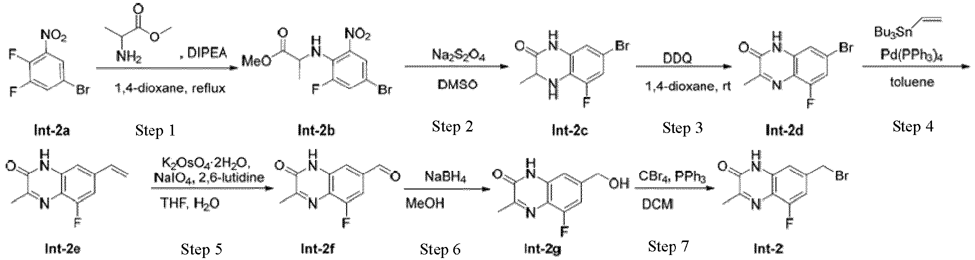

### Step 1: Preparation of compound methyl (4-bromo-2-fluoro-6-nitrophenyl)alaninate (Int-2b)

Compound **Int-2a** (5.0 g, 21.0 mmol), methyl alaninate hydrochloride (4.39 g, 31.5 mmol) and DIPEA (14.6 mL, 84.0 mmol) were dissolved in 1,4-dioxane (70 mL), and the reaction system was heated to 110°C to react for 24 h. After the reaction was completed, the reaction solution was concentrated, and the obtained crude product was purified by silica gel column to obtain the title compound **(Int-2b)** (3.97 g, yield: 73%).

MS m/z (ES): 321.0 [M+H]⁺.

### Step 2: Preparation of compound 7-bromo-5-fluoro-3-methyl-3,4-dihydroquinoxalin-2(1H)-one (Int-2c)

Compound **Int-2b** (3.50 g, 10.9 mmol) and sodium dithionite (5.70 g, 32.7 mmol) were dissolved in DMSO (80 mL), and the reaction system was heated to 120°C to react for 4 h. When the reaction was completed, the reaction solution was cooled to 0°C, water (200 mL) was slowly added thereto for quenching the reaction, and then extraction was performed with ethyl acetate for three times. Organic phases were combined, dried with anhydrous sodium sulfate and filtered. Filtrate was concentrated to obtain a crude product of compound. The obtained crude product was purified by column chromatography to obtain the title compound **Int-2c** (734 mg, yield: 26%).

MS m/z (ES): 259.0 [M+H]⁺.

### Step 3: Preparation of compound 7-bromo-5-fluoro-3-methylquinolin-2(1H)-one (Int-2d)

Compound **Int-2c** (700 mg, 2.70 mmol) and DDQ (683 mg, 3.01 mmol) were dissolved in 1,4-dioxane (20 mL) to react under room temperature conditions for 4 h. When the reaction was completed, the reaction solution was concentrated, and a saturated sodium bicarbonate aqueous solution (20 mL) was added to a crude product of the obtained solid. After being stirred for 2 h, it was filted, and washed with water and a saturated sodium bicarbonate solution to obtain the title compound **Int-2d** (624 mg, yield: 90%).

MS m/z (ES): 257.0 [M+H]⁺.

### Step 4: Preparation of compound 5-fluoro-3-methyl-7-vinylquinolin-2(1H)-one (Int-2e)

Compound **Int-2d** (300 g, 1.17 mmol) and tributylvinyltin (740 mg, 2.33 mmol) were dissolved in toluene (10 mL), and Pd(PPh₃)₄ (136 mg, 0.12 mmol) was added under N₂ protection and heated to 100°C to react overnight. The reaction solution was concentrated to obtain a crude product of compound. The obtained crude product was purified by column chromatography to obtain the title compound **Int-2e** (228 mg, yield: 95%).

MS m/z (ES): 205.1 [M+H]⁺.

### Step 5: Preparation of compound 8-fluoro-2-methyl-3-oxo-3,4-dihydroquinoxalin-6-formaldehyde (Int-2f)

Compound **Int-2e** (800 mg, 3.86 mmol), potassium osmate dihydrate (60 mg, 0.193 mmol), 2,6-dimethylpyridine (0.9 mL, 7.72 mmol) and NaIO₄ (4.13 g, 19.3 mmol) were dissolved in THF (30 mL) and water (20 mL) to react overnight at room temperature. When the reaction was completed, a saturated ammonium chloride aqueous solution (50 mL) was added for quenching the reaction, and then extraction was performed with dichloromethane for three times. Organic phases were combined, dried with anhydrous sodium sulfate and filtered. Filtrate was concentrated to obtain a crude product of compound. The obtained crude product was purified by column chromatography to obtain the title compound **Int-2f** (711 mg, yield: 89%).

MS m/z (ES): 207.1 [M+H]⁺.

### Step 6: Preparation of compound 5-fluoro-7-hydroxymethyl-3-methylquinoxalin-2(1H)-one (Int-2g)

At 0°C, compound **Int-2f** (700 mg, 3.35 mmol) was dissolved in methanol (20 mL), and NaBH₄ (253 mg, 6.70 mmol) was added. After the addition was completed, the reaction was carried out at 0°C for 1 h. When the reaction was completed, water (2 mL) was slowly added thereto for quenching the reaction at 0°C, and the reaction solution was concentrated to obtain a crude product of compound. The obtained crude product was purified by column chromatography to obtain the title compound **Int-2g** (504 mg, yield: 72%).

MS m/z (ES): 209.1 [M+H]⁺.

### Step 7: Preparation of compound 7-(bromomethyl)-5-fluoro-3-methylquinoxalin-2(1H)-one (Int-2)

At 0°C, compound **Int-2** (600 mg, 2.87 mmol), carbon tetrabromide (1.90 g, 5.74 mmol) and PPh₃ (1.51 g, 5.74 mmol) were dissolved in DCM (30 mL) and stirred at 0°C. When the reaction was completed, the reaction solution was concentrated to obtain a crude product of compound. The obtained crude product was purified by column chromatography to obtain the title compound **Int-2** (163 mg, yield: 21%).

### MS m/z (ES): 271.0 [M+H]⁺.

### Route 2:

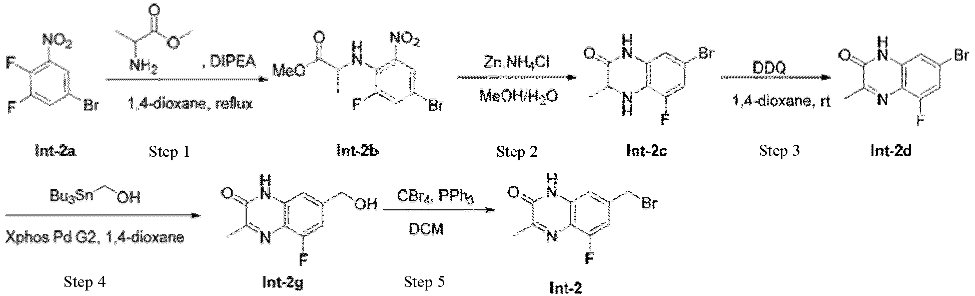

### Step 1: Preparation of compound methyl (4-bromo-2-fluoro-6-nitrophenyl)alaninate (Int-2b)

The title compound **(Int-2b)** (6.20 g, yield: 90%) was obtained by the procedure similar to that in Step 1 of Route 1.

MS m/z (ES): 321.0 [M+H]⁺.

### Step 2: Preparation of compound 7-bromo-5-fluoro-3-methyl-3,4-dihydroquinoxalin-2(1H)-one (Int-2c)

Compound **Int-2b** (6.20 g, 19.4 mmol) and NH₄Cl (20.7 g, 388 mmol) were dissolved in MeOH/H₂O (70 mL/7 mL), the reaction system was cooled to 0°C, zinc powder was added, and after the addition was completed, the reaction system was warmed up to 25°C to react for 6 h. When the reaction was completed, the reaction system was filtered, filter cake was washed with methanol, and filtrate was concentrated to obtain a crude product of compound. The obtained crude product of compound **Int-2-3** (4.7 g) was directly used in the next reaction.

MS m/z (ES): 259.0 [M+H]⁺.

### Step 3: Preparation of compound 7-bromo-5-fluoro-3-methylquinolin-2(1H)-one (Int-2d)

A crude product of compound was obtained by the procedure similar to that in Step 3 of Route 1. The obtained crude product of compound was purified by column chromatography to obtain **Int-2d** (2.5 g, two-step yield: 50%).

MS m/z (ES): 257.0 [M+H]⁺.

### Step 4: Preparation of compound 5-fluoro-7-hydroxymethyl-3-methylquinoxalin-2(1H)-one (Int-2g)

Compound **Int-2d** (300 mg, 1.17 mmol) and (tributyltin)methanol (450 mg, 1.41 mmol) were dissolved in dioxane (10 mL), and Xphos Pd G2 (92 mg, 0.12 mmol) was added under N₂ protection, heated to 80°C and stirred to react overnight. The reaction solution was concentrated to obtain a crude product of compound. The obtained crude product was purified by column chromatography to obtain the title compound **Int-2g** (190 mg, yield: 78%).

MS m/z (ES): 209.1 [M+H]⁺.

### Step 5: Preparation of compound 7-(bromomethyl)-5-fluoro-3-methylquinoxalin-2(1H)-one (Int-2)

A crude product of compound was obtained by the procedure similar to that in Step 7 of Route 1. The obtained crude product of compound was purified by column chromatography to obtain **Int-2** (148 mg, yield: 60%).

MS m/z (ES): 271.0 [M+H]⁺.

### Example C: Preparation of 5-bromo-7-(bromomethyl)-3-3-methylquinoxalin-2(1H)-one (Int-3)

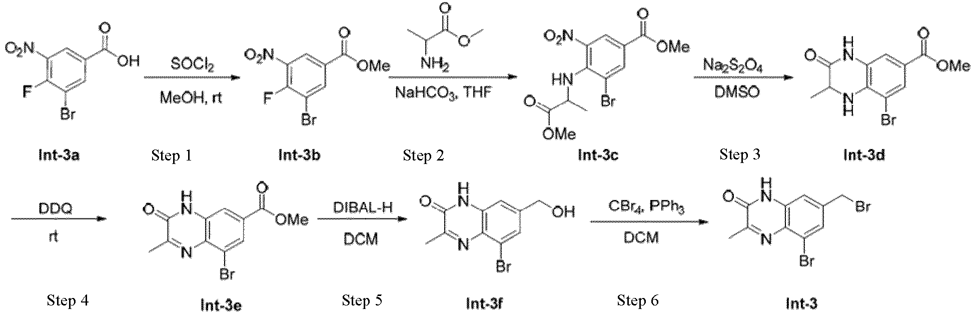

### Step 1: Preparation of compound methyl 3-bromo-4-fluoro-5-nitrobenzoate (Int-3b)

At 0°C, compound **Int-3a** (5.0 g, 18.9 mmol) was dissolved in MeOH (100 mL), and then SOCl₂ (4.1 mL, 56.7 mmol) was added thereto dropwise. The reaction system was warmed up to room temperature to react overnight. When the reaction was completed, saturated sodium bicarbonate (200 mL) was added for dilution at 0°C, extraction was performed with ethyl acetate for three times, drying was performed with anhydrous sodium sulfate, filtration was performed, and filtrate was concentrated to obtain a crude product of compound. The obtained crude product was purified by column chromatography to obtain the title compound **Int-3b** (4.47 g, yield: 86%).

MS m/z (ES): 278.0 [M+H]⁺.

### Step 2: Preparation of compound methyl 3-bromo-4-(1-methoxy-1-oxopropan-2-yl)amino-5-nitrobenzoate (Int-3c)

Compound **Int-3b** (4.4 g, 15.8 mmol), methyl alaninate hydrochloride (4.41 g, 31.6 mmol) and NaHCO₃(4.0 g, 47.4 mmol) were dissolved in THF (70 mL). The reaction system was left to react overnight under room temperature conditions. When the reaction was completed, water (150 mL) was added for dilution, and extraction was performed with ethyl acetate for three times. Organic phases were washed with saturated saline solution, dried with anhydrous sodium sulfate and filtered. Filtrate was concentrated to obtain a crude product of compound. The obtained crude product was purified by column chromatography to obtain the title compound **Int-3c** (5.1 g, yield: 89%).

MS m/z (ES): 361.0 [M+H]⁺.

### Step 3 to Step 4: Preparation of compound methyl 8-bromo-2-methyl-3-oxo-3,4-dihydroquinoxalin-6-carboxylate (Int-3e)

Except that compound **Int-3c** was used to replace compound **Int-2b** in Step 2 of Example B, a crude product of compound **Int-3e** was synthesized by a method similar to that described in Step 2 to Step 3 of Example B. The obtained crude product was purified by column chromatography to obtain the title compound **Int-3e** (1.4 g, yield: 25%).

MS m/z (ES): 297.0 [M+H]⁺.

### Step 5: Preparation of compound 5-bromo-7-hydroxymethyl-3-methylquinoxalin-2(1H)-one (Int-3f)

Compound **(Int-3e)** (400 mg, 1.34 mmol) was dissolved in DCM (10 mL), and after the reaction solution was cooled to 0°C, DIBAL-H (1 M in hexane, 4.04 mL, 4.04 mmol) was added to the reaction system. After the addition was completed, the reaction was carried out overnight at room temperature. After the reaction was completed, a saturated potassium sodium tartrate aqueous solution (40 mL) was added to the reaction system at 0°C for quenching the reaction, and dichloromethane was added for liquid separation. Aqueous phase was extracted with dichloromethane for three times. Organic phases were combined, dried with anhydrous sodium sulfate and subjected to suction filtration. Filtrate was concentrated to obtain a crude product of compound. The obtained crude product was purified by silica gel column to obtain the title compound **(Int-3f)** (220 mg, yield: 61%).

MS m/z (ES): 269.0 [M+H]⁺.

### Step 6: Preparation of compound 5-bromo-7-(hydroxymethyl)-3-methylquinoxalin-2(1H)-one (Int-3)

Except that compound **Int-3f** was used to replace compound **Int-2g** in Step 7 of Example B, a crude product of compound **Int-3** was synthesized by a method similar to that described in Step 7 of Example B. The obtained crude product was purified by a preparative silica gel plate to obtain the title compound **Int-3** (120 mg, yield: 31 %).

MS m/z (ES): 471.1 [M+H]⁺.

### Example D: Preparation of compound 7-(bromomethyl)-5,6-difluoro-3-methylquinoxalin-2(1H)-one (Int-4)

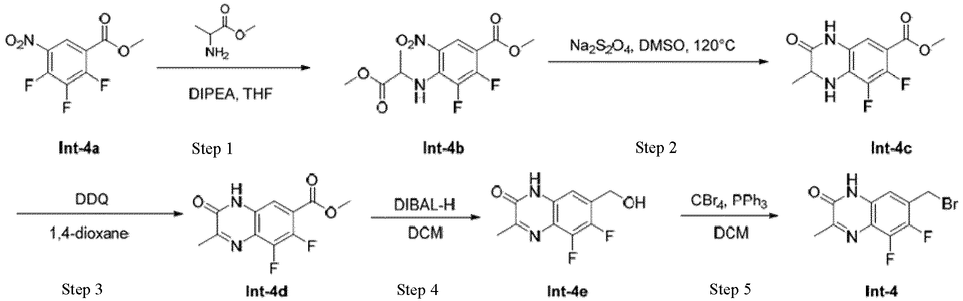

### Step 1: Preparation of compound methyl 2,3-difluoro-4-((1-methoxy-1-oxopropan-2-yl)amino)-5-nitrobenzoate (Int-4b)

Compound **Int-4a** (4.0 g, 17.0 mmol), methyl alaninate hydrochloride (3.31 g, 23.8 mmol) and DIPEA (4.39 g, 34.0 mmol) were dissolved in THF (70 mL), and the reaction system was lefted to react at room temperature. When the reaction was completed, water (200 mL) was slowly added for quenching the reaction, and then extraction was performed with ethyl acetate for three times. Organic phases were combined, dried with anhydrous sodium sulfate and filtered. Filtrate was concentrated to obtain a crude product of the title compound **Int-4b** (5.41 g). The obtained crude product was directly used in the next reaction without purification.

MS m/z (ES): 319.0 [M+H]⁺.

### Step 2: Preparation of compound methyl 7,8-difluoro-2-methyl-3-oxo-1,2,3,4-tetrahydroquinoxalin-6-carboxylate (Int-4c)

The crude product of compound **Int-4b** (5.41 g, 17.0 mmol) and sodium dithionite (8.87 g, 51.0 mmol) were dissolved in DMSO (80 mL), and the reaction system was heated to 120°C and stirred. When the reaction was completed, the reaction solution was cooled to 0°C, water (200 mL) was slowly added thereto for quenching the reaction, and then extraction was performed with ethyl acetate for three times. Organic phases were combined, dried with anhydrous sodium sulfate and filtered. Filtrate was concentrated to obtain a crude product of compound. The obtained crude product was purified by column chromatography to obtain the title compound **Int-4c** (1.63 g, two-step yield: 37%).

MS m/z (ES): 257.0 [M+H]⁺.

### Step 3: Preparation of compound methyl 7,8-difluoro-2-methyl-3-oxo-3,4-dihydroquinoxalin-6-carboxylate (Int-4d)

Compound **(Int-4c)** (1.63 g, 6.36 mmol) and DDQ (1.6 g, 7.00 mmol) were dissolved in 1,4-dioxane (40 mL) and stirred under room temperature conditions. When the reaction was completed, the reaction solution was concentrated, and a saturated sodium bicarbonate aqueous solution (20 mL) was added to the crude product of the obtained solid. After being stirred for 2 h, it was filted. Filter cake was washed with water and a saturated sodium bicarbonate solution. The solid was collected and dried to obtain the title compound **Int-4d** (1.17 g, yield: 72%).

MS m/z (ES): 255.0 [M+H]⁺.

### Step 4: Preparation of compound 5,6-difluoro-7-(hydroxymethyl)-3-methylquinoxalin-2(1H)-one (Int-4e)

Compound **(Int-4d)** (1.17 g, 4.60 mmol) was dissolved in DCM (30 mL), cooled to 0°C in an ice water bath, and then DIBAL-H (1 M in toluene, 13.8 mL, 13.8 mmol) was added to the reaction system. After the addition was completed, stirring was performed at 0°C. After the reaction was completed, a saturated potassium sodium tartrate aqueous solution (40 mL) was added to the reaction system at 0°C for quenching the reaction, dichloromethane was added for dilution, and stirring was performed for 30 min for liquid separation. Aqueous phase was extracted with dichloromethane for three times. Organic phases were combined, dried with anhydrous sodium sulfate and subjected to suction filtration. Filtrate was concentrated to obtain a crude product of compound. The obtained crude product was purified by silica gel column to obtain the title compound **(Int-4e)** (800 mg, yield: 77%).

MS m/z (ES): 227.0 [M+H]⁺.

### Step 5: Preparation of compound 7-(bromomethyl)-5,6-difluoro-3-methylquinoxalin-2(1H)-one (Int-4)

At 0°C, PPh₃ (401 mg, 1.53 mmol) was added to a DCM (10 mL) solution of compound **Int-4e** (115 mg, 0.51 mmol) and carbon tetrabromide (505 mg, 1.53 mmol), and the reaction system was stirred at 0°C. When the reaction was completed, the reaction solution was concentrated to obtain a crude product of compound. The obtained crude product was purified by column chromatography to obtain the title compound **Int-4** (110 mg, yield: 75%).

MS m/z (ES): 289.0 [M+H]⁺.

### Example E: Preparation 7-(bromomethyl)-3,5-dimethylquinoxalin-2(1H)-one (Int-5)

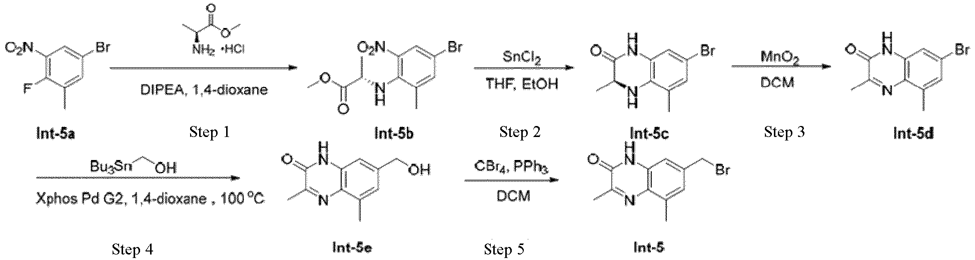

### Step 1: Preparation of compound methyl (4-bromo-2-methyl-6-nitrophenyl)-L-alaninate (Int-5b)

Compound **Int-5a** (5.0 g, 21.4 mmol), methyl L-alaninate hydrochloride (4.47 g, 32.1 mmol) and DIPEA (14.6 mL, 84.0 mmol) were dissolved in 1,4-dioxane (70 mL), and the reaction system was heated to 110°C to react, and stirred. After the reaction was completed, the reaction system was cooled to room temperature, and water and ethyl acetate were added to the system for dilution and liquid separation. Aqueous phase was extracted with ethyl acetate for three times. Organic phases were combined, dried with anhydrous sodium sulfate and subjected to suction filtration. Filtrate was concentrated to obtain a crude product of the title compound **(Int-5b)** (6.6 g). The obtained crude product was directly used in the next reaction without further purification.

MS m/z (ES): 317.0 [M+H]⁺.

### Step 2: Preparation of compound (S)-7-bromo-3,5-dimethyl-3,4-dihydroquinoxalin-2(1H)-one (Int-5c)

The crude product of compound **Int-5b** (6.6 g, 20.8 mmol) was dissolved in THF/EtOH (100 mL/100 mL) and cooled to 0°C, and tin dichloride (11.8 g, 62.2 mmol) was added under nitrogen atmosphere, then warmed up to room temperature and stirred. After the reaction was completed, the reaction solution was concentrated, and residue was diluted with water and dichloromethane. Then, the pH of aqueous phase was adjusted to 9-10 with a saturated sodium bicarbonate solution. Filtration was performed with diatomite to filter out insoluble substances. Filter cake was washed with dichloromethane. Filtrate was subjected to liquid separation. The aqueous phase was extracted with dichloromethane for three times. Organic phases were combined, dried with anhydrous sodium sulfate and filtered. The filtrate was concentrated to obtain a crude product of the title compound **Int-5c** (4.2 g). The obtained crude product was directly used in the next reaction without further purification.

MS m/z (ES): 255.0 [M+H]⁺.

### Step 3: Preparation of compound 7-bromo-3,5-dimethylquinoxalin-2(1H)-one (Int-5d)

The crude product of compound **Int-5c** (4.2 g, 16.5 mmol) was dissolved in dichloromethane (100 mL), and manganese dioxide (7.2 g, 82.8 mmol) was added to react overnight at room temperature. After the reaction was completed, filtration was performed with diatomite. Filter cake was washed with methanol for three times. Filtrate was concentrated to dryness to obtain a crude product of the title compound **Int-5d** (4.0 g). The obtained crude product was directly used in the next reaction without further purification.

MS m/z (ES): 253.0 [M+H]⁺.

### Step 4 to Step 5: Preparation of compound 7-(bromomethyl)-3,5-dimethylquinoxalin-2(1H)-one (Int-5)

Except that compound **Int-5d** was used to replace compound **Int-2d** in Step 4 of Route 2 of Example B, a crude product of compound **Int-5** was synthesized by a method similar to that described in Step 4 to Step 5 of Route 2 of Example B. The obtained crude product was purified by column chromatography to obtain the title compound **Int-5** (760 mg, five-step yield: 13%).

MS m/z (ES): 267.0 [M+H]⁺.

### Example F: Preparation of compound 7-(bromomethyl)-3,6-dimethylquinoxalin-2(1H)-one (Int-6)

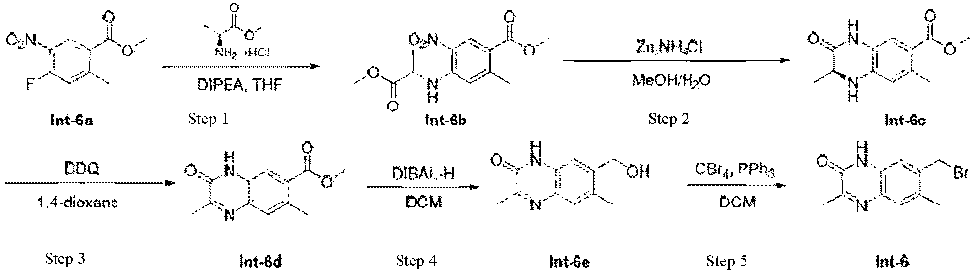

### Step 1 to Step 3: Preparation of compound methyl 2,7-dimethyl-3-oxo-3,4-dihydroquinoxalin-6-carboxylate (Int-6d)

Except that compound **Int-6a** was used to replace compound **Int-2a** in Step 1 of Route 2 of Example B, a crude product of compound **Int-6d** was synthesized by a method similar to that described in Step 1 to Step 3 of Route 2 of Example B. The obtained crude product was purified by column chromatography to obtain the title compound **Int-6d** (7.1 g, three-step yield: 75%).

MS m/z (ES): 233.0 [M+H]⁺.

### Step 4 to Step 5: Preparation of compound 7-(bromomethyl)-3,6-dimethylquinoxalin-2(1H)-one (Int-6)

Except that compound **Int-6d** was used to replace compound **Int-4d** in Step 4 of Example D, a crude product of compound **Int-6** was synthesized by a method similar to that described in Step 4 to Step 5 of Example D. The obtained crude product was purified by column chromatography to obtain the title compound **Int-6** (101 mg, two-step yield: 41 %).

MS m/z (ES): 267.0 [M+H]⁺.

### Example G: Preparation of compound 7-(bromomethyl)-5-chloro-3-methylquinoxalin-2(1H)-one (Int-9)

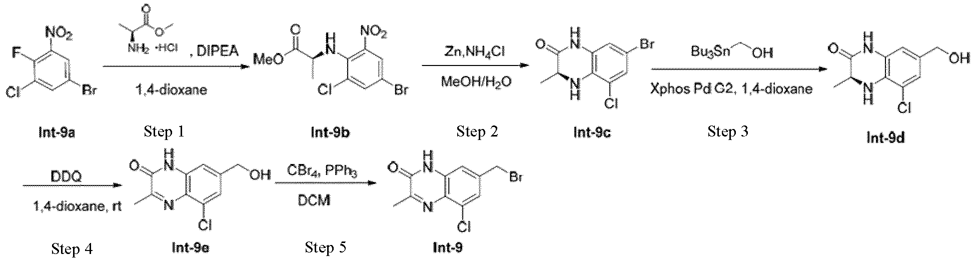

### Step 1 to Step 2: Preparation of compound (S)-7-bromo-5-chloro-3-methyl-3,4-dihydroquinoxalin-2(1H)-one (Int-9c)

Except that compound **Int-9a** was used to replace compound **Int-2a** in Step 1 of Route 2 of Example B, a crude product (2.41 g) of compound **Int-9c** was synthesized by a method similar to that described in Step 1 to Step 2 of Route 2 of Example B. The obtained crude product was directly used in the next step without further purification.

MS m/z (ES): 274.9 [M+H]⁺.

### Step 3: Preparation of compound (S)-5-chloro-7-hydroxymethyl-3-methyl-3,4-dihydroquinoxalin-2(1H)-one (Int-9d)

Except that compound **Int-9c** was used to replace compound **Int-2d** in Step 4 of Route 2 of Example B, a crude product of compound **Int-9d** was synthesized by a method similar to that described in Step 4 of Route 2 of Example B. The obtained crude product was purified by column chromatography to obtain the title compound **Int-9d** (157 mg, three-step yield: 35%).

MS m/z (ES): 227.0 [M+H]⁺.

### Step 4: Preparation of compound 5-chloro-7-hydroxymethyl-3-methylquinoxalin-2(1H)-one (Int-9e)

Except that compound **Int-9d** was used to replace compound **Int-2c** in Step 3 of Route 2 of Example B, a crude product of compound **Int-9e** was synthesized by a method similar to that described in Step 3 of Route 2 of Example B. The obtained crude product was purified by column chromatography to obtain the title compound **Int-9e** (53 mg, yield: 34%).

MS m/z (ES): 225.0 [M+H]⁺.

### Step 5: Preparation of compound 7-(bromomethyl)-5-chloro-3-methylquinoxalin-2(1H)-one (Int-9)

Except that compound **Int-9e** was used to replace compound **Int-2g** in Step 5 of Route 2 of Example B, a crude product of compound **Int-9** was synthesized by a method similar to that described in Step 5 of Route 2 of Example B. The obtained crude product was purified by column chromatography to obtain the title compound **Int-9** (64 mg, yield: 93%).

MS m/z (ES): 286.9 [M+H]⁺.

Referring to the synthesis methods in Example B to Example G, the following intermediate compounds were synthesized under similar reaction conditions.

| Intermediate number | Intermediate structure | Reference method and Example | LCMS |
|---|---|---|---|
| Int-7 | | Example B Route 2 | m/z (ES) : 285.0 [M+H]+ |
| Int-8 | | Example B Route 2 | m/z (ES) : 283.0 [M+H]⁺ |
| Int-10 | | Example B Route 2 | m/z (ES) : 297.0[M+H]⁺ |

### Example 1: Preparation of 5-(4-(8-fluoro-2-methyl-3-oxo-3,4-dihydroquinoxalin-6-yl)methyl)piperazin-1-yl)-N -methylpicolinamide (1)

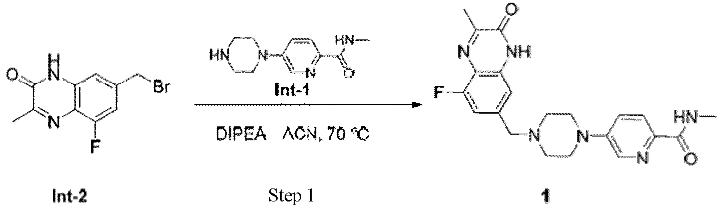

### Step 1: Preparation of 5-(4-(8-fluoro-2-methyl-3-oxo-3,4-dihydroquinoxalin-6-yl)methyl)piperazin-1-yl)-N -methylpicolinamide (1)

Compound **Int-2** (50 mg, 0.185 mmol), compound Int-1 (41 mg, 0.185 mmol) and DIPEA (0.10 mL, 0.57 mmol) were dissolved in acetonitrile (4 mL). The reaction system was heated to 70°C to react for 2 h. When the reaction was completed, the reaction solution was concentrated to obtain a crude product of compound. The obtained crude product was purified by a preparative silica gel plate to obtain the title compound 1 (21.04 mg, yield: 28%).

MS m/z (ES): 411.2 [M+H]⁺.

¹H NMR (400 MHz, DMSO-*d*₆) δ 12.43 (s, 1H), 8.40 (q, *J* = 4.4 Hz, 1H), 8.27 (d, *J= 2.8* Hz, 1H), 7.84 (d, *J=* 8.4 Hz, 1H), 7.39 (dd, *J* = 8.8, 2.8 Hz, 1H), 7.13 - 7.06 (m, 2H), 3.60 (s, 2H), 3.39 - 3.31 (m, 4H), 2.78 (d, *J=* 4.8 Hz, 3H), 2.59 - 2.53 (m, 4H), 2.41 (s, 3H).

### Example 2: Preparation of 5-(4-((8-bromo-2-methyl-3-oxo-3,4-dihydroquinoxalin-6-yl)methyl) piperazin-1-yl)-N-methylpicolinamide (2)

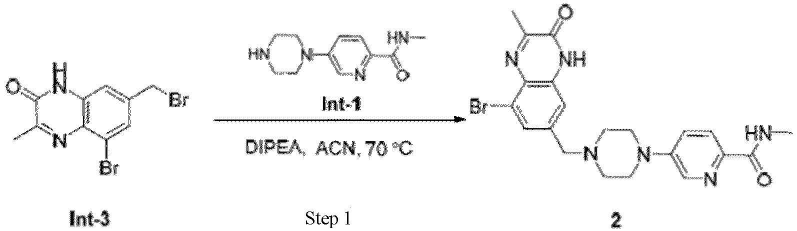

Except that compound **Int-3** was used to replace compound **Int-2** in Example 1, a crude product of compound 2 was synthesized by a method similar to that described in Example 1. The obtained crude product was purified by a preparative silica gel plate to obtain the title compound 2 (9.33 mg).

MS m/z (ES): 471.1 [M+H]⁺.

### Example 3: Preparation of compound 5-(6-(8-fluoro-2-methyl-3-oxo-3,4-dihydroquinoxalin-6-yl)methyl)-3,6-diazabicyclo [3.1.1]heptan-3-yl)-N,6-dimethylpicolinamide (3)

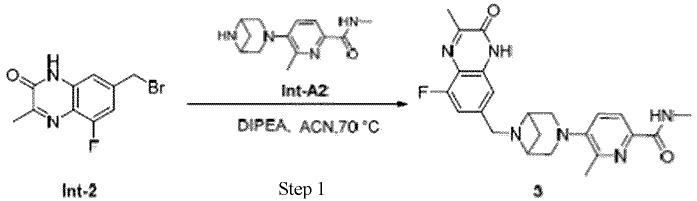

Except that compound **Int-A2** was used to replace compound Int-1 in Example 1, a crude product of compound 3 was synthesized by a method similar to that described in Example 1. The obtained crude product was purified by a preparative silica gel plate to obtain the title compound 3 (8.04 mg).

MS m/z (ES): 437.2 [M+H]⁺.

¹H NMR (400 MHz, DMSO-*d*₆) δ 8.38 (q, *J* = 5.2 Hz, 1H), 7.78 (d, *J=* 8.4 Hz, 1H), 7.61 (d, *J =* 8.4 Hz, 1H), 7.08 (s, 1H), 7.04 (d, *J* = 12.4 Hz, 1H), 3.72 - 3.61 (m, 6H), 3.39 (d, *J =* 10.8 Hz, 2H), 2.81 (d, *J* = 4.8 Hz, 3H), 2.63 (s, 3H), 2.53 - 2.51 (m, 1H), 2.37 (s, 3H), 1.86 (d, *J= 8.0* Hz, 1H).

### Example 4: Preparation of compound 5-((1S,4S)-5-((8-fluoro-2-methyl-3-oxo-3,4-dihydroquinoxalin-6-yl)methyl)-2,5-diazabicyclo[2.2.1]heptan-2-yl)-N,6-dimethylpicolinamide (4)

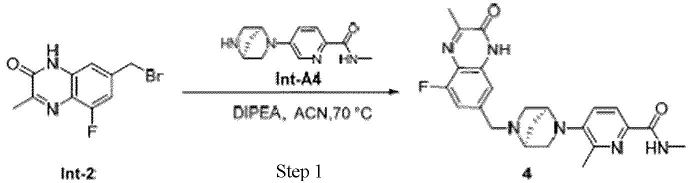

Except that compound **Int-A4** was used to replace compound **Int-1** in Example 1, a crude product of compound 4 was synthesized by a method similar to that described in Example 1. The obtained crude product was purified by a preparative silica gel plate to obtain the title compound 4 (11.5 mg).

MS m/z (ES): 437.2 [M+H]⁺.

¹H NMR (400 MHz, DMSO-*d*₆) δ 12.37 (s, 1H), 8.29 (q, *J= 4.8* Hz, 1H), 7.65 (d, *J* = 8.0 Hz, 1H), 7.10 (d, *J= 8.8* Hz, 1H), 7.06 (s, 1H), 7.05 (d, *J* = 12.8 Hz, 1H), 4.24 (s, 1H), 3.74 (s, 2H), 3.65 - 3.57 (m, 1H), 3.53 (s, 1H), 3.36 - 3.30 (m, 1H), 2.87 - 2.81 (m, 1H), 2.79 (d, *J= 4.8* Hz, 3H), 2.77 - 2.71 (m, 1H), 2.51 (s, 3H), 2.39 (s, 3H), 1.97 - 1.90 (m, 1H), 1.85 - 1.78 (m, 1H).

### Example 5: Preparation of compound 5-(6-(8-fluoro-2-methyl-3-oxo-3,4-dihydroquinoxalin-6-yl)methyl)-3,6-diazabicyclo [3.1.1]heptan-3-yl)-N-methylpicolinamide (5)

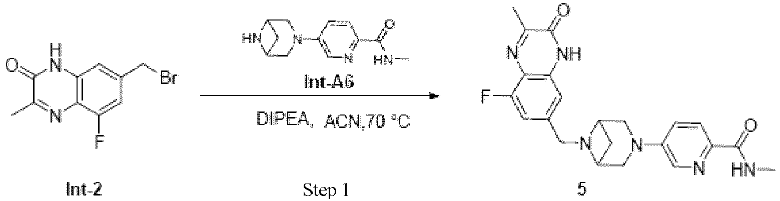

Except that compound **Int-A6** was used to replace compound **Int-1** in Example 1, a crude product of compound 5 was synthesized by a method similar to that described in Example 1. The obtained crude product was purified by a preparative silica gel plate to obtain the title compound 5 (3.21 mg).

MS m/z (ES): 423.2 [M+H]⁺.

¹H NMR (400 MHz, DMSO-*d*₆) δ 12.36 (s, 1H), 8.37 (q, *J* = 4.8 Hz, 1H), 8.11 (d, *J*= 2.8 Hz, 1H), 7.89 (d, *J=* 8.8 Hz, 1H), 7.20 (dd, *J* = 8.8, 2.8 Hz, 1H), 7.11 (s, 1H), 7.10 (d, *J =* 9.6 Hz, 1H), 3.74 (d, *J =* 6.0 Hz, 2H), 3.61 (s, 2H), 3.59 (d, *J* = 11.2 Hz, 2H), 3.43 (d, *J* = 11.6 Hz, 2H), 2.80 (d, *J* = 5.2 Hz, 3H), 2.63 - 2.55 (m, 1H), 2.40 (s, 3H), 1.63 (d, *J* = 8.4 Hz, 1H).

### Example 6: Preparation of compound 5-(4-((8-fluoro-2-methyl-3-oxo-3,4-dihydroquinoxalin-6-yl)methyl)piperazin-1-yl)-N,6-dimethylpicolinamide (6)

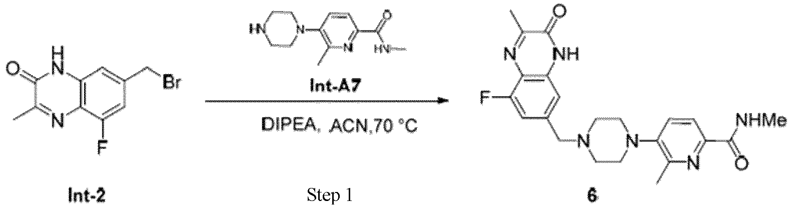

Except that compound Int-A7 was used to replace compound Int-1 in Example 1, a crude product of compound 6 was synthesized by a method similar to that described in Example 1. The obtained crude product was purified by a preparative silica gel plate to obtain the title compound 6 (6.48 mg).

MS m/z (ES): 425.2 [M+H]⁺.

¹H NMR (400 MHz, DMSO-*d*₆) δ 8.41 (q, *J* = 4.8 Hz, 1H), 7.79 (d, *J* = 8.4 Hz, 1H), 7.48 (d, *J* = 8.4 Hz, 1H), 7.07 (s, 1H), 7.05 (d, *J= 10.8* Hz, 1H), 3.61 (s, 2H), 3.00 - 2.91 (m, 4H), 2.80 (d, *J= 4.8* Hz, 3H), 2.62 - 2.54 (m, 4H), 2.49 (s, 3H), 2.40 (s, 3H).

### Example 7: Preparation of compound 6-chloro-5-(4-((8-fluoro-2-methyl-3-oxo-3,4-dihydroquinoxalin-6-yl)methyl) piperazin-1-yl)-N-methylpicolinamide (7)

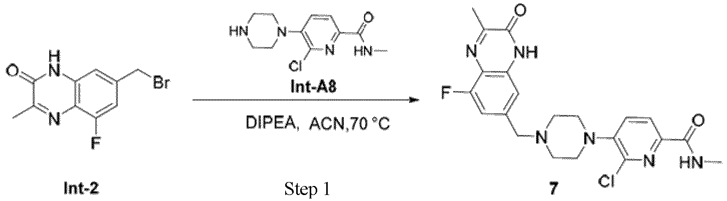

Except that compound **Int-A8** was used to replace compound **Int-1** in Example 1, a crude product of compound 7 was synthesized by a method similar to that described in Example 1. The obtained crude product was purified by a preparative silica gel plate to obtain the title compound 7 (2.05 mg).

MS m/z (ES): 445.1 [M+H]⁺.

¹H NMR (400 MHz, DMSO-*d*₆) δ 12.45 (s, 1H), 8.43 (q, *J* = 4.8 Hz, 1H), 7.94 (d, *J* = 8.0 Hz, 1H), 7.67 (d, *J* = 8.0 Hz, 1H), 7.11 (d, *J* = 11.2 Hz, 1H), 7.09 (s, 1H), 3.63 (s, 2H), 3.18 - 3.09 (m, 4H), 2.79 (d, *J=* 4.8 Hz, 3H), 2.66 - 2.56 (m, 4H), 2.41 (s, 3H).

### Example 8: Preparation of compound 5-(4-((7,8-difluoro-2-methyl-3-oxo-3,4-dihydroquinoxalin-6-yl)methyl)piperazin-1-yl)-N-methylpicolinamide (8)

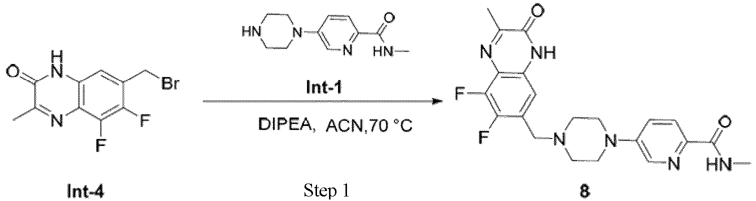

Except that compound **Int-4** was used to replace compound **Int-2** in Example 1, a crude product of compound **8** was synthesized by a method similar to that described in Example 1. The obtained crude product was purified by a preparative silica gel plate to obtain the title compound **8** (8.11 mg).

MS m/z (ES): 429.1 [M+H]⁺.

¹H NMR (400 MHz, DMSO-*d*₆) δ 8.40 (q, *J* = 4.8 Hz, 1H), 8.26 (d, *J =* 2.8 Hz, 1H), 7.82 (d, *J= 8.8* Hz, 1H), 7.39 (dd, *J= 8.8,* 2.8 Hz, 1H), 7.08 - 7.04 (m, 1H), 3.68 (s, 2H), 3.39 - 3.31 (m, 4H), 2.78 (d, *J=* 4.8 Hz, 3H), 2.61 - 2.56 (m, 4H), 2.37 (s, 3H).

### Example 9: Preparation of compound 5-(4-((7,8-difluoro-2-methyl-3-oxo-3,4-dihydroquinoxalin-6-yl)methyl)piperazin-1-yl)-N,6-dimethylpicolinamide (9)

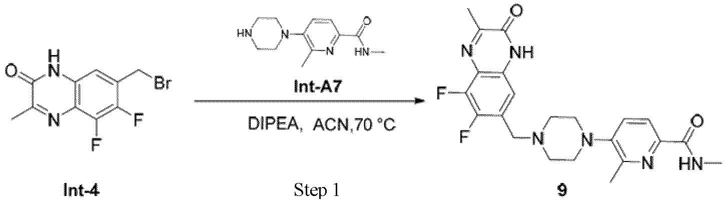

Except that compound **Int-4** was used to replace compound **Int-2** in Example 1 and compound **Int-A7** was used to replace compound **Int-1** in Example 1, a crude product of compound **9** was synthesized by a method similar to that described in Example 1. The obtained crude product was purified by a preparative silica gel plate to obtain the title compound **9** (26.62 mg).

MS m/z (ES): 443.1 [M+H]⁺.

¹H NMR (400 MHz, DMSO-*d*₆) δ 8.42 (q, *J* = 4.4 Hz, 1H), 7.79 (d, *J* = 8.4 Hz, 1H), 7.47 (d, *J=* 8.0 Hz, 1H), 7.12 - 7.04 (m, 1H), 3.70 (s, 2H), 2.99 - 2.92 (m, 4H), 2.80 (d, *J* = 4.8 Hz, 3H), 2.65 - 2.58 (m, 4H), 2.48 (s, 3H), 2.38 (s, 3H).

### Example 10: Preparation of compound 5-(4-((2,8-dimethyl-3-oxo-3,4-dihydroquinoxalin-6-yl)methyl)piperazin-1-yl)-N,6-dimethylpicolinamide (10)

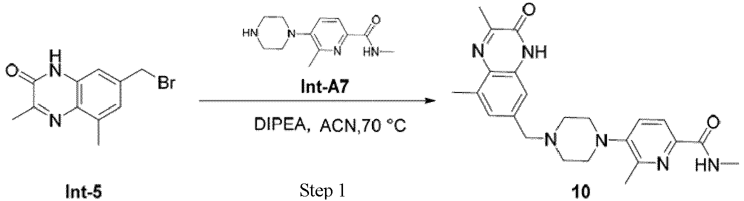

Except that compound **Int-5** was used to replace compound **Int-2** in Example 1 and compound **Int-A7** was used to replace compound **Int-1** in Example 1, a crude product of compound **10** was synthesized by a method similar to that described in Example 1. The obtained crude product was purified by a preparative silica gel plate to obtain the title compound **10** (9.66 mg).

MS m/z (ES): 421.2 [M+H]⁺.

¹H NMR (400 MHz, DMSO-*d*₆)) δ 12.23 (s, 1H), 8.43 (q, *J= 4.8* Hz, 1H), 7.79 (d, *J* = 8.0 Hz, 1H), 7.47 (d, *J* = 8.4 Hz, 1H), 7.15-7.04 (m, 2H), 3.57 (s, 2H), 2.99 - 2.91 (m, 4H), 2.79 (d, *J= 4.8* Hz, 3H), 2.63 - 2.54 (m, 4H), 2.54 (s, 3H), 2.48 (s, 3H), 2.40 (s, 3H).

### Example 11: Preparation of compound 3-ethyl-5-fluoro-7-((4-(6-(3-hydroxyoxetan-3-yl)-2-methylpyridin-3-yl)piperazin-1-yl)methyl)quinoxalin-2(1H)-one (11)

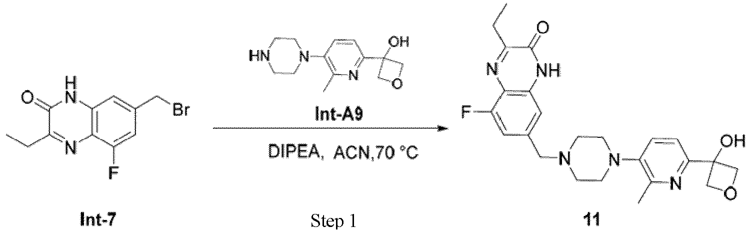

Except that compound **Int-A9** was used to replace compound **Int-1** in Example 1 and compound **Int-7** was used to replace compound **Int-2** in Example 1, a crude product of compound 11 was synthesized by a method similar to that described in Example 1. The obtained crude product was purified by a preparative silica gel plate to obtain the title compound 11 (6.30 mg).

MS m/z (ES): 454.2 [M+H]⁺.

¹H NMR (400 MHz, DMSO-*d*₆) δ 12.40 (s, 1H), 7.43 (d, *J= 8.4* Hz, 1H), 7.37 (d, *J= 8.4* Hz, 1H), 7.09 - 7.00 (m, 2H), 6.33 (s, 1H), 4.88 (d, *J* = 6.0 Hz, 2H), 4.60 (d, *J* = 6.0 Hz, 2H), 3.61 (s, 2H), 2.95 - 2.84 (m, 4H), 2.79 (q, *J=* 7.6 Hz, 2H), 2.63 - 2.53 (m, 4H), 2.47 (s, 3H), 1.21 (t, *J* = 7.6 Hz, 3H).

### Example 12: Preparation of compound 5-(4-((8-fluoro-2-methyl-3-oxo-3,4-dihydroquinoxalin-6-yl)methyl)piperazin-1-yl)-6-methylpyridinitrile (12)

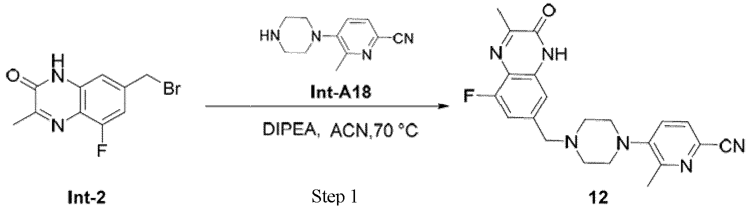

Except that compound **Int-A18** was used to replace compound **Int-1** in Example 1, a crude product of compound **12** was synthesized by a method similar to that described in Example 1. The obtained crude product was purified by a preparative silica gel plate to obtain the title compound **12** (45.17 mg).

MS m/z (ES): 393.1 [M+H]⁺.

¹H NMR (400 MHz, DMSO-*d*₆) δ 12.42 (s, 1H), 7.80 (d, *J= 8.4* Hz, 1H), 7.48 (d, *J=* 8.4 Hz, 1H), 7.12 - 7.07 (m, 2H), 3.62 (s, 2H), 3.05 - 2.97 (m, 4H), 2.63 - 2.54 (m, 4H), 2.46 (s, 3H), 2.41 (s, 3H).

### Example 13: Preparation of compound 5-(4-((8-fluoro-2-methyl-3-oxo-3,4-dihydroquinoxalin-6-yl)methyl)piperazin-1-yl) pyridinitrile (13)

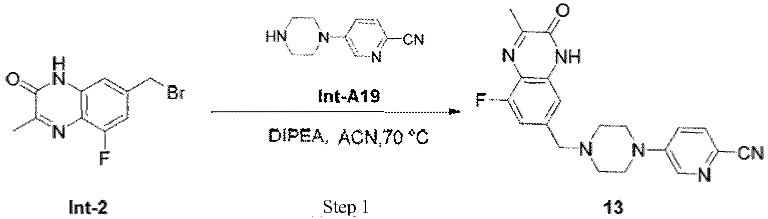

Except that compound **Int-A19** was used to replace compound Int-1 in Example 1, a crude product of compound 13 was synthesized by a method similar to that described in Example 1. The obtained crude product was purified by a preparative silica gel plate to obtain the title compound 13 (5.46 mg).

MS m/z (ES): 379.1 [M+H]⁺.

¹H NMR (400 MHz, DMSO-*d*₆) δ 12.43 (s, 1H), 8.42 (d, *J* = 3.2 Hz, 1H), 7.75 (d, *J=* 8.4 Hz, 1H), 7.36 (dd, *J* = 8.8, 2.8 Hz, 1H), 7.12 - 7.06 (m, 2H), 3.60 (s, 2H), 3.47 - 3.40 (m, 4H), 2.56 - 2.50 (m, 4H), 2.41 (s, 3H).

### Example 14: Preparation of compound 5-(4-((8-fluoro-2-methyl-3-oxo-3,4-dihydroquinoxalin-6-yl)methyl)piperazin-1-yl)-6-methoxy-N-methylpicolinamide (14)

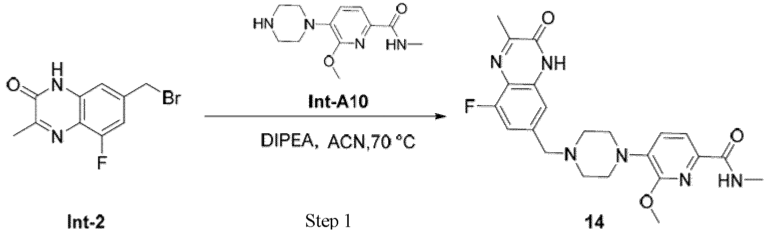

Except that compound **Int-A10** was used to replace compound **Int-1** in Example 1, a crude product of compound **14** was synthesized by a method similar to that described in Example 1. The obtained crude product was purified by a preparative silica gel plate to obtain the title compound **14** (66.65 mg).

MS m/z (ES): 441.2 [M+H]⁺.

¹H NMR (400 MHz, DMSO-*d*₆)) δ 8.30 (q, *J=* 4.8 Hz, 1H), 7.54 (d, *J* = 8.0 Hz, 1H), 7.25 (d, *J= 8.0* Hz, 1H), 7.04 (s, 1H), 7.00 (d, *J* = 11.2 Hz, 1H), 3.99 (s, 3H), 3.58 (s, 2H), 3.16 - 3.05 (m, 4H), 2.81 (d, *J= 4.8* Hz, 3H), 2.59 - 2.52 (m, 4H), 2.38 (s, 3H).

### Example 15: Preparation of compound 4-(4-((8-fluoro-2-methyl-3-oxo-3,4-dihydroquinoxalin-6-yl)methyl)piperazin-1-yl)-N-methylthiazol-2-formamide (15)

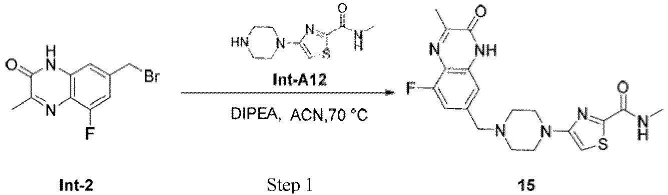

Except that compound **Int-A12** was used to replace compound **Int-1** in Example 1, a crude product of compound **15** was synthesized by a method similar to that described in Example 1. The obtained crude product was purified by a preparative silica gel plate to obtain the title compound **15** (12.8 mg).

MS m/z (ES): 417.1 [M+H]⁺.

¹H NMR (400 MHz, DMSO-*d*₆) δ 12.42 (s, 1H), 8.54 (q, *J* = 4.4 Hz, 1H), 7.13-7.07 (m, 2H), 6.57 (s, 1H), 3.60 (s, 2H), 3.32 - 3.26 (m, 4H), 2.77 (d, *J=* 4.8 Hz, 3H), 2.56 - 2.52 (m, 4H), 2.41 (s, 3H).

### Example 16: Preparation of compound 5-(4-((8-fluoro-2-methyl-3-oxo-3,4-dihydroquinoxalin-6-yl)methyl)piperazin-1-yl)-N-methylthiazol-2-formamide (16)

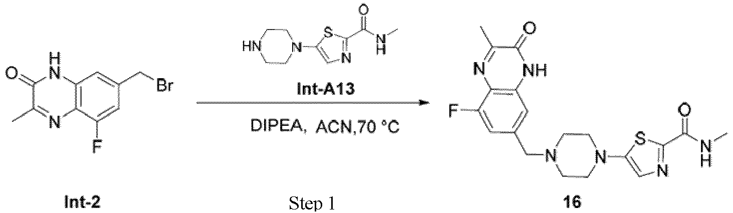

Except that compound **Int-A13** was used to replace compound **Int-1** in Example 1, a crude product of compound **16** was synthesized by a method similar to that described in Example 1. The obtained crude product was purified by a preparative silica gel plate to obtain the title compound **16** (15.78 mg).

MS m/z (ES): 417.1 [M+H]⁺.

¹H NMR (400 MHz, DMSO-*d*₆) δ 12.38 (s, 1H), 8.38 (q, *J=* 4.4 Hz, 1H), 7.13 (s, 1H), 7.11 - 7.05 (m, 2H), 3.60 (s, 2H), 3.27 - 3.18 (m, 4H), 2.74 (d, *J* = 4.8 Hz, 3H), 2.59 - 2.53 (m, 4H), 2.41 (s, 3H).

### Example 17: Preparation of compound 2-(4-((8-fluoro-2-methyl-3-oxo-3,4-dihydroquinoxalin-6-yl)methyl)piperazin-1-yl)-N-methylthiazol-5-formamide (17)

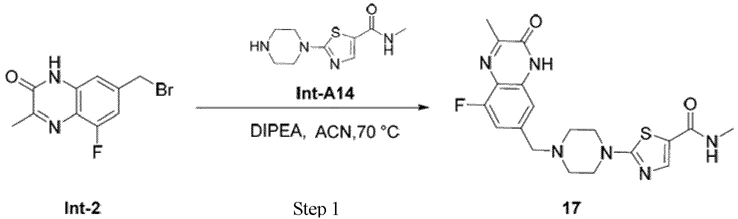

Except that compound **Int-A14** was used to replace compound **Int-1** in Example 1, a crude product of compound 17 was synthesized by a method similar to that described in Example 1. The obtained crude product was purified by a preparative silica gel plate to obtain the title compound 17 (15.78 mg).

MS m/z (ES): 417.1 [M+H]⁺.

### Example 18: Preparation of compound 2-fluoro-4-(4-((8-fluoro-2-methyl-3-oxo-3,4-dihydroquinoxalin-6-yl)methyl) piperazin-1-yl)benzonitrile (18)

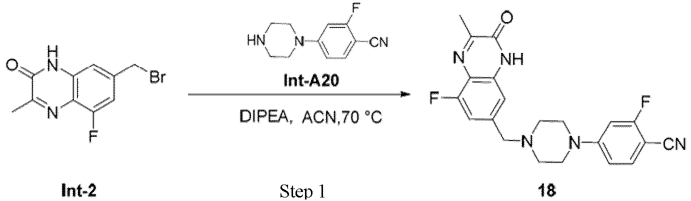

Except that compound **Int-A20** was used to replace compound Int-1 in Example 1, a crude product of compound **18** was synthesized by a method similar to that described in Example 1. The obtained crude product was purified by a preparative silica gel plate to obtain the title compound 18 (5.92 mg).

MS m/z (ES): 396.1 [M+H]⁺.

¹H NMR (400 MHz, DMSO-*d*₆) δ 12.42 (s, 1H), 7.59 (t, *J= 8.4* Hz, 1H), 7.12 - 7.05 (m, 2H), 6.95 (dd, *J* = 14.0, 2.4 Hz, 1H), 6.85 (dd, *J* = 9.2, 2.4 Hz, 1H), 3.59 (s, 2H), 3.44 - 3.36 (m, 4H), 2.51 - 2.47 (m, 4H), 2.41 (s, 3H).

### Example 19: Preparation of compound 4-(4-((2,8-dimethyl-3-oxo-3,4-dihydroquinoxalin-6-yl)methyl)piperazin-1-yl) -2-fluorobenzonitrile (19)

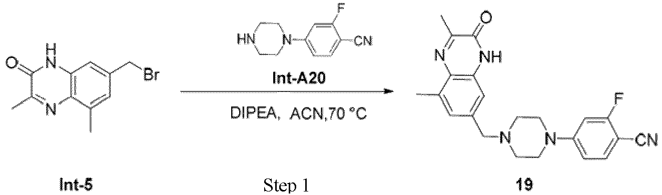

Except that compound **Int-5** was used to replace compound **Int-2** in Example 18, a crude product of compound **19** was synthesized by a method similar to that described in Example 18. The obtained crude product was purified by a preparative silica gel plate to obtain the title compound 19 (4.63 mg).

MS m/z (ES): 392.1 [M+H]⁺.

¹H NMR (400 MHz, DMSO-*d*₆) δ 12.19 (s, 1H), 7.62-7.56 (m, 1H), 7.12 - 7.07 (m, 2H), 6.94 (dd, *J* = 14.0, 2.4 Hz, 1H), 6.85 (dd, *J* = 9.2, 2.4 Hz, 1H), 3.54 (s, 2H), 3.43 - 3.36 (m, 4H), 2.54 (s, 3H), 2.51 - 2.45 (m, 4H), 2.41 (s, 3H).

### Example 20: Preparation of compound N-cyclopropyl-5-(4-((8-fluoro-2-methyl-3-oxo-3,4-dihydroquinoxalin-6-yl)methyl)piperazin-1-yl)picolinamide (20)

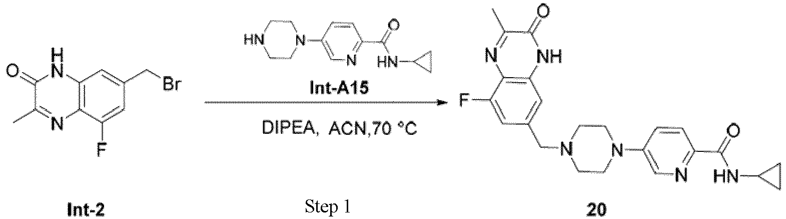

Except that compound **Int-A15** was used to replace compound Int-1 in Example 1, a crude product of compound **20** was synthesized by a method similar to that described in Example 1. The obtained crude product was purified by a preparative silica gel plate to obtain the title compound **20** (1.29 mg).

MS m/z (ES): 437.2 [M+H]⁺.

¹H NMR (400 MHz, DMSO-*d*₆) δ 12.42 (s, 1H), 8.35 (d, *J* = 5.2 Hz, 1H), 8.24 (d, *J= 2.8* Hz, 1H), 7.83 (d, *J= 8.8* Hz, 1H), 7.40 (dd, *J=* 8.8, 2.8 Hz, 1H), 7.14 - 7.06 (m, 2H), 3.60 (s, 2H), 3.38 - 3.31 (m, 4H), 2.89 - 2.80 (m, 1H), 2.58 - 2.53 (m, 4H), 2.41 (s, 3H), 0.70 - 0.64 (m, 2H), 0.64 - 0.58 (m, 2H).

### Example 21: Preparation of compound 5-(4-((8-fluoro-2-methyl-3-oxo-3,4-dihydroquinoxalin-6-yl)methyl)piperazin-1-yl)-N-(2-methoxyethyl)-6-methylpicolinamide (21)

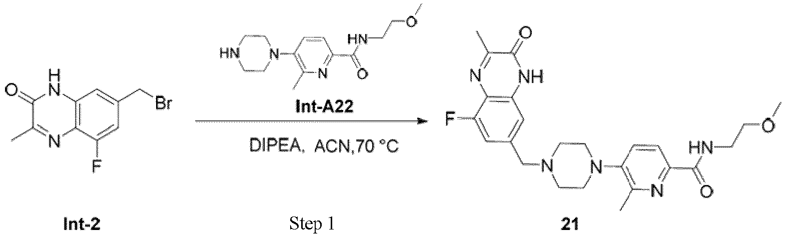

Except that compound **Int-A22** was used to replace compound **Int-1** in Example 1, a crude product of compound 21 was synthesized by a method similar to that described in Example 1. The obtained crude product was purified by a preparative silica gel plate to obtain the title compound 21 (4.0 mg).

MS m/z (ES): 469.2 [M+H]⁺.

¹H NMR (400 MHz, DMSO-*d*₆) δ 12.43 (s, 1H), 8.44 - 8.35 (m, 1H), 7.81 (d, *J* = 8.0 Hz, 1H), 7.50 (d, *J= 8.4* Hz, 1H), 7.14 - 7.06 (m, 2H), 3.62 (s, 2H), 3.49 - 3.42 (m, 4H), 3.27 (s, 3H), 3.04 - 2.89 (m, 4H), 2.64 - 2.55 (m, 4H), 2.49 (s, 3H), 2.41 (s, 3H).

### Example 22: Preparation of compound 5-(4-((2,8-dimethyl-3-oxo-3,4-dihydroquinoxalin-6-yl)methyl)piperazin-1-yl)-N-(2-methoxyethyl)-6-methylpicolinamide (22)

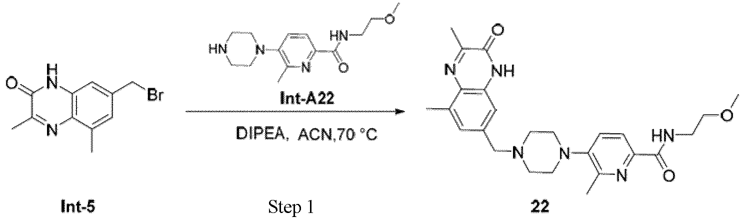

Except that compound **Int-5** was used to replace compound **Int-2** in Example 21, a crude product of compound **22** was synthesized by a method similar to that described in Example 21. The obtained crude product was purified by a preparative silica gel plate to obtain the title compound **22** (10.0 mg).

MS m/z (ES): 465.2 [M+H]⁺.

¹H NMR (400 MHz, DMSO-*d*₆) δ 12.21 (s, 1H), 8.43 - 8.36 (m, 1H), 7.81 (d, *J* = 8.4 Hz, 1H), 7.49 (d, *J= 8.4* Hz, 1H), 7.12 (s, 1H), 7.11 (s, 1H), 3.58 (s, 2H), 3.49 - 3.41 (m, 4H), 3.27 (s, 3H), 3.00 - 2.91 (m, 4H), 2.62 - 2.53 (m, 4H), 2.55 (s, 3H), 2.41 (s, 3H).

### Example 23: Preparation of compound 2-(4-((8-fluoro-2-methyl-3-oxo-3,4-dihydroquinoxalin-6-yl)methyl)piperazin-1-yl) thiazol-5-nitrile (23)

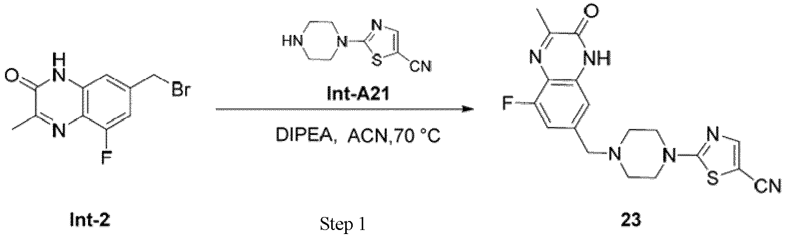

Except that compound **Int-A21** was used to replace compound **Int-1** in Example 1, a crude product of compound **23** was synthesized by a method similar to that described in Example 1. The obtained crude product was purified by a preparative silica gel plate to obtain the title compound **23** (4.0 mg).

MS m/z (ES): 385.1 [M+H]⁺.

¹H NMR (400 MHz, DMSO-*d*₆) δ 12.39 (s, 1H), 8.02 (s, 1H), 7.12 - 7.05 (m, 2H), 3.61 (s, 2H), 3.59 - 3.53 (m, 4H), 2.57 - 2.50 (m, 4H), 2.41 (s, 3H).

### Example 24: Preparation of compound (S)-5-(4-((8-fluoro-2-methyl-3-oxo-3,4-dihydroquinoxalin-6-yl)methyl)-2-methylpiperazin-1-yl)-N-methylpicolinamide (24)

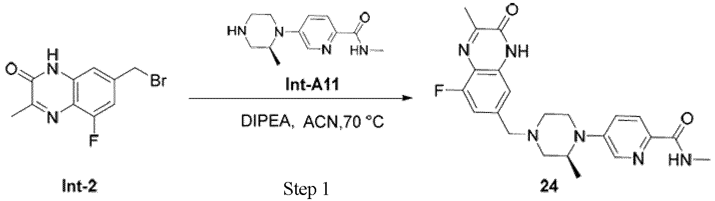

Except that compound **Int-A11** was used to replace compound **Int-1** in Example 1, a crude product of compound **24** was synthesized by a method similar to that described in Example 1. The obtained crude product was purified by a preparative silica gel plate to obtain the title compound **24** (7.00 mg).

MS m/z (ES): 425.2 [M+H]⁺.

¹H NMR (600 MHz, DMSO-*d*₆) δ 12.48 (s, 1H), 8.36 (q, *J= 4.8* Hz, 1H), 8.21 (d, *J=* 3.0 Hz, 1H), 7.82 (d, *J* = 9.0 Hz, 1H), 7.33 (dd, *J* = 9.0, 3.0 Hz, 1H), 7.13 (s, 1H), 7.11 (d, *J= 11.4* Hz, 1H), 4.25-4.20 (m, 1H), 3.65 (d, *J=* 13.8 Hz, 1H), 3.60 (d, *J=* 12.0 Hz, 1H), 3.52 (d, *J* = 14.4 Hz, 1H), 3.10 (td, *J* = 12.0, 3.6 Hz, 1H), 2.92 (d, *J* = 10.2 Hz, 1H), 2.78 (d, *J= 4.8* Hz, 3H), 2.72 (d, *J= 10.8* Hz, 1H), 2.41 (s, 3H), 2.33 (dd, *J* = 11.4, 3.6 Hz, 1H), 2.22 (td, *J=* 11.4, 3.6 Hz, 1H), 1.15 (d, *J=* 6.0 Hz, 3H).

### Example 25: Preparation of compound (R)-5-(4-((8-fluoro-2-methyl-3-oxo-3,4-dihydroquinoxalin-6-yl)methyl)-2-methylpiperazin-1-yl)-N-methylpicolinamide (25)

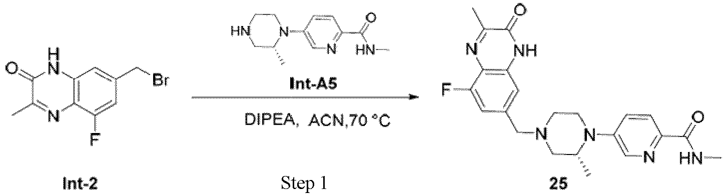

Except that compound **Int-A5** was used to replace compound **Int-1** in Example 1, a crude product of compound **25** was synthesized by a method similar to that described in Example 1. The obtained crude product was purified by a preparative silica gel plate to obtain the title compound **25** (3.66 mg).

MS m/z (ES): 425.2 [M+H]⁺.

¹H NMR (600 MHz, DMSO-*d*₆) δ 12.48 (s, 1H), 8.36 (q, *J= 4.8* Hz, 1H), 8.21 (d, *J* = 3.0 Hz, 1H), 7.82 (d, *J* = 9.0 Hz, 1H), 7.33 (dd, *J* = 9.0, 3.0 Hz, 1H), 7.13 (s, 1H), 7.11 (d, *J= 11.4* Hz, 1H), 4.25-4.20 (m, 1H), 3.65 (d, *J=* 13.8 Hz, 1H), 3.60 (d, *J=* 12.0 Hz, 1H), 3.52 (d, *J* = 14.4 Hz, 1H), 3.10 (td, *J* = 12.0*,* 3.6 Hz, 1H), 2.92 (d, *J* = 10.2 Hz, 1H), 2.78 (d, *J= 4.8* Hz, 3H), 2.72 (d, *J= 10.8* Hz, 1H), 2.41 (s, 3H), 2.33 (dd, *J* = 11.4, 3.6 Hz, 1H), 2.22 (td, *J=* 11.4, 3.6 Hz, 1H), 1.15 (d, *J=* 6.0 Hz, 3H).

### Example 26: Preparation of compound 7-((4-(6-(difluoromethyl)-2-methylpyridin-3-yl)piperazin-1-yl)methyl)-5-fluoro-3-methylquinoxalin-2(1H)-one (26)

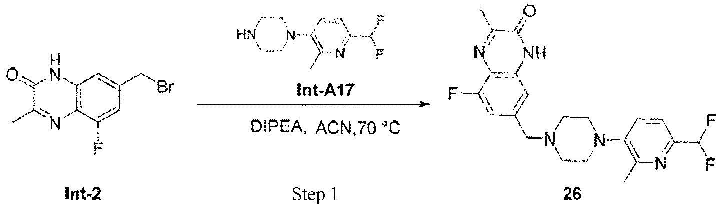

Except that compound **Int-A17** was used to replace compound **Int-1** in Example 1, a crude product of compound **26** was synthesized by a method similar to that described in Example 1. The obtained crude product was purified by a preparative silica gel plate to obtain the title compound **26** (12.62 mg).

MS m/z (ES): 418.2 [M+H]⁺.

¹H NMR (400 MHz, DMSO-*d*₆) δ 7.52 (d, *J= 8.4* Hz, 1H), 7.46 (d, *J= 8.0* Hz, 1H), 7.10 - 7.02 (m, 2H), 6.81 (t, *J* = 55.2 Hz, 1H), 3.61 (s, 2H), 3.00-2.89 (m, 4H), 2.65-2.53 (m, 4H), 2.46 (s, 3H), 2.40 (s, 3H).

### Example 27: Preparation of compound 7-((4-(6-(difluoromethyl)pyridin-3-yl)piperazin-1-yl)methyl)-5-fluoro-3-methylquinoxalin-2(1H)-one (27)

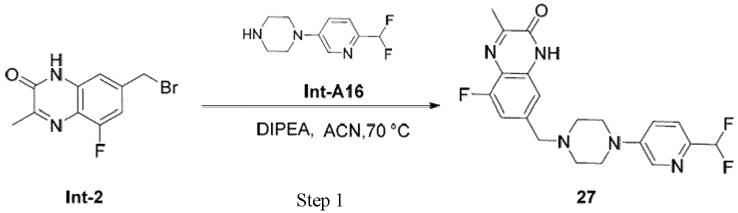

Except that compound **Int-A16** was used to replace compound Int-1 in Example 1, a crude product of compound **27** was synthesized by a method similar to that described in Example 1. The obtained crude product was purified by a preparative silica gel plate to obtain the title compound **27** (50.20 mg).

MS m/z (ES): 403.2 [M+H]⁺.

¹H NMR (400 MHz, DMSO-*d*₆) δ 12.42 (s, 1H), 8.35 (d, *J= 2.8* Hz, 1H), 7.49 (d, *J= 8.8* Hz, 1H), 7.42 (dd, *J= 8.8,* 2.8 Hz, 1H), 7.13 - 7.06 (m, 2H), 6.81 (t, *J* = 55.2 Hz, 1H), 3.60 (s, 2H), 3.36 - 3.29 (m, 4H), 2.59 - 2.52 (m, 4H), 2.41 (s, 3H).

### Example 28: Preparation of compound N-(2,2-difluoroethyl)-5-(4-((8-fluoro-2-methyl-3-oxo-3,4-dihydroquinoxalin-6-yl) methyl)piperazin-1-yl)-6-methylpicolinamide (28)

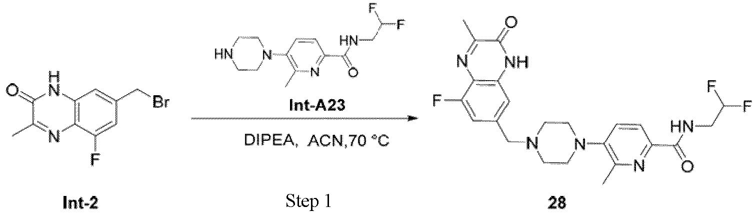

Except that compound **Int-A23** was used to replace compound **Int-1** in Example 1, a crude product of compound **28** was synthesized by a method similar to that described in Example 1. The obtained crude product was purified by a preparative silica gel plate to obtain the title compound 28 (3.30 mg).

MS m/z (ES): 475.2 [M+H]⁺.

¹H NMR (400 MHz, DMSO-*d*₆) δ 12.41 (s, 1H), 8.73 (t, *J* = 6.0 Hz, 1H), 7.84 (d, *J= 8.4* Hz, 1H), 7.51 (d, *J= 8.4* Hz, 1H), 7.14 - 7.06 (m, 2H), 6.13 (tt, *J* = 56.4, 4.0 Hz,, 1H), 3.76 - 3.64 (m, 2H), 3.63 (s, 2H), 3.02 - 2.93 (m, 4H), 2.64 - 2.56 (m, 4H), 2.51 (s, 3H), 2.41 (s, 3H).

### Example 29: Preparation of compound 5-(4-((8-fluoro-2-methyl-3-oxo-3,4-dihydroquinoxalin-6-yl)methyl)piperazin-1-yl)-N-(2-fluoroethyl)-6-methylpicolinamide (29)

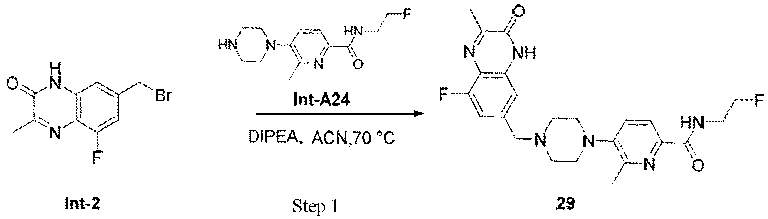

### Except that compound Int-A24 was used to replace compound Int-1 in Example 1, a crude product of compound 29 was synthesized by a method similar to that described in Example 1. The obtained crude product was purified by a preparative silica gel plate to obtain the title compound 29 (8.93 mg).

MS m/z (ES): 457.2 [M+H]⁺.

¹H NMR (400 MHz, DMSO-*d*₆) δ 12.44 (s, 1H), 8.66 - 8.54 (m, 1H), 7.83 (d, *J* = 8.0 Hz, 1H), 7.51 (d, *J* = 8.0 Hz, 1H), 7.17 - 7.03 (m, 2H), 4.54 (dt, *J* = 47.2*,* 5.2 Hz, 2H), 3.67 - 3.52 (m, 4H), 3.05 - 2.88 (m, 4H), 2.65 - 2.55 (m, 4H), 2.51 (s, 3H), 2.42 (s, 3H).

### Example 30: Preparation of compound 5-(4-((2-ethyl-8-fluoro-3-oxo-3,4-dihydroquinoxalin-6-yl)methyl)piperazin-1-yl)-N-(2-fluoroethyl)-6-methylpicolinamide (30)

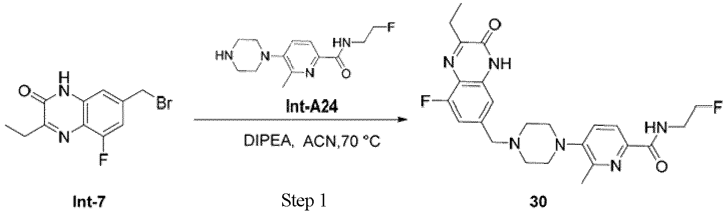

Except that compound **Int-7** was used to replace compound **Int-2** in Example 29, a crude product of compound **30** was synthesized by a method similar to that described in Example 29. The obtained crude product was purified by a preparative silica gel plate to obtain the title compound **30** (9.83 mg).

MS m/z (ES): 471.2 [M+H]⁺.

¹H NMR (400 MHz, DMSO-*d*₆) δ 12.36 (s, 1H), 8.59 (t, *J* = 6.0 Hz, 1H), 7.82 (d, *J= 8.4* Hz, 1H), 7.50 (d, *J= 8.4* Hz, 1H), 7.15 - 7.07 (m, 2H), 4.54 (dt, *J* = 47.6, 5.2 Hz, 2H), 3.68-3.50 (m, 4H), 3.02 - 2.91 (m, 4H), 2.81 (q, *J* = 7.4 Hz, 2H), 2.65-2.55 (m, 4H), 2.51 (s, 3H), 1.22 (t, *J* = 7.2 Hz, 3H).

### Example 31: Preparation of compound 5-(4-((2-ethyl-8-fluoro-3-oxo-3,4-dihydroquinoxalin-6-yl)methyl)piperazin-1-yl)-N-(2-hydroxyethyl)-6-methylpicolinamide (31)

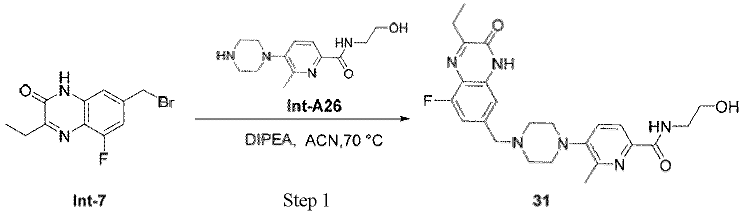

Except that compound **Int-A26** was used to replace compound **Int-A24** in Example 30, a crude product of compound **31** was synthesized by a method similar to that described in Example 30. The obtained crude product was purified by a preparative silica gel plate to obtain the title compound **31** (5.80 mg).

MS m/z (ES): 469.2 [M+H]⁺.

¹H NMR (400 MHz, DMSO-*d*₆) δ 12.39 (s, 1H), 8.40 (t, *J* = 5.6 Hz, 1H), 7.81 (d, *J* = 8.0 Hz, 1H), 7.50 (d, *J* = 8.4 Hz, 1H), 7.09 - 7.00 (m, 2H), 4.79 (s, 1H), 3.62 (s, 2H), 3.54 -3.47 (m, 2H), 3.39 - 3.33 (m, 2H), 3.01 - 2.91 (m, 4H), 2.79 (q, *J* = 7.2 Hz, 2H), 2.64 - 2.55 (m, 4H), 2.49 (s, 3H), 1.21 (t, *J* = 7.6 Hz, 3H).

### Example 32: Preparation of compound 5-(4-((2-ethyl-8-fluoro-3-oxo-3,4-dihydroquinoxalin-6-yl)methyl)piperazin-1-yl)-N, 6-dimethylpicolinamide (32)

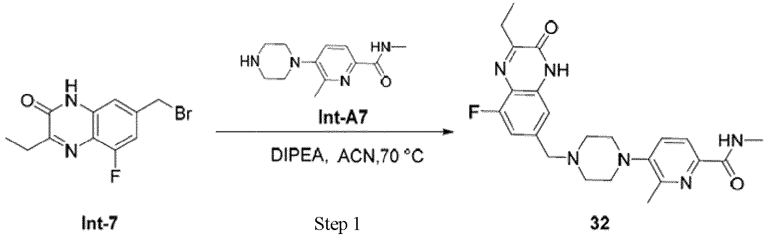

Except that compound **Int-A7** was used to replace compound **Int-A24** in Example 30, a crude product of compound **32** was synthesized by a method similar to that described in Example 30. The obtained crude product was purified by a preparative silica gel plate to obtain the title compound **32** (9.00 mg).

MS m/z (ES): 439.2 [M+H]⁺.

¹H NMR (400 MHz, DMSO-d₆) δ 8.41 (q, *J* = 4.8 Hz, 1H), 7.79 (d, *J* = 8.0 Hz, 1H), 7.49 (d, *J* = 8.0 Hz, 1H), 7.00 (s, 1H), 6.92 (d, *J* = 11.2 Hz, 1H), 3.59 (s, 2H), 3.00 - 2.90 (m, 4H), 2.80 (d, *J* = 4.8 Hz, 3H), 2.76 (q, *J* = 7.6 Hz, 2H), 2.63 - 2.54 (m, 4H), 2.48 (s, 3H), 1.19 (t, *J* = 7.6 Hz, 3H).

### Example 33: Preparation of compound 5-(4-((2-ethyl-8-fluoro-3-oxo-3,4-dihydroquinoxalin-6-yl)methyl)piperazin-1-yl)-6-methyl-N-(oxetan-3-ylmethyl)picolinamide (33)

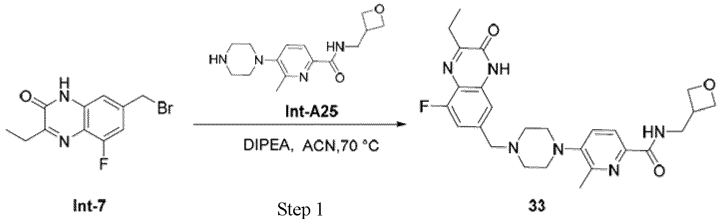

Except that compound **Int-A25** was used to replace compound **Int-A24** in Example 30, a crude product of compound **33** was synthesized by a method similar to that described in Example 30. The obtained crude product was purified by a preparative silica gel plate to obtain the title compound **33** (6.6 mg).

MS m/z (ES): 495.2 [M+H]⁺.

### Example 34: Preparation of compound 5-(4-((2-ethyl-8-fluoro-3-oxo-3,4-dihydroquinoxalin-6-yl)methyl)piperazin-1-yl)-N-(2-methoxyethyl)-6-methylpicolinamide (34)

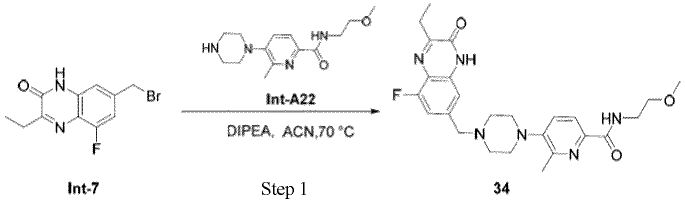

Except that compound **Int-A22** was used to replace compound **Int-A24** in Example 30, a crude product of compound **34** was synthesized by a method similar to that described in Example 30. The obtained crude product was purified by a preparative silica gel plate to obtain the title compound **34** (13.70 mg).

MS m/z (ES): 483.2 [M+H]⁺.

¹H NMR (400 MHz, DMSO-d₆) δ 8.43 - 8.36 (m, 1H), 7.82 (d, *J* = 8.4 Hz, 1H), 7.50 (d, *J* = 8.0 Hz, 1H), 7.13 - 7.04 (m, 2H), 3.63 (s, 2H), 3.48 - 3.43 (m, 4H), 3.27 (s, 3H), 3.00 - 2.91 (m, 4H), 2.81 (q, *J* = 7.6 Hz, 2H), 2.65 - 2.55 (m, 4H), 2.50 (s, 3H), 1.22 (t, *J* = 7.2 Hz, 3H).

### Example 35: Preparation of compound 4-(4-((2-ethyl-8-fluoro-3-oxo-3,4-dihydroquinoxalin-6-yl)methyl)piperazin-1-yl)-2-fluorobenzonitrile (35)

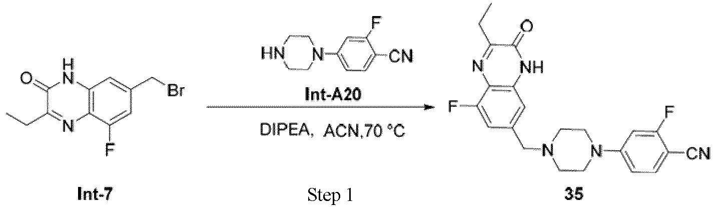

Except that compound **Int-A20** was used to replace compound **Int-A24** in Example 30, a crude product of compound **35** was synthesized by a method similar to that described in Example 30. The obtained crude product was purified by a preparative silica gel plate to obtain the title compound **35** (5.60 mg).

MS m/z (ES): 410.1 [M+H]⁺.

¹H NMR (400 MHz, DMSO-d₆) δ 12.43 (s, 1H), 7.59 (t, *J* = 8.4 Hz, 1H), 7.12 - 7.05 (m, 2H), 6.95 (dd, *J* = 14.4, 2.4 Hz, 1H), 6.85 (dd, *J* = 9.2, 2.4 Hz, 1H), 3.59 (s, 2H), 3.45 - 3.37 (m, 4H), 2.80 (q, *J* = 7.2 Hz, 2H), 2.53 - 2.47 (m, 4H), 1.21 (t, *J* = 7.2 Hz, 3H).

### Example 36: Preparation of compound 5-(4-((7,8-difluoro-2-methyl-3-oxo-3,4-dihydroquinoxalin-6-yl)methyl)piperazin-1-yl)-N-(2-methoxyethyl)-6-methylpicolinamide (36)

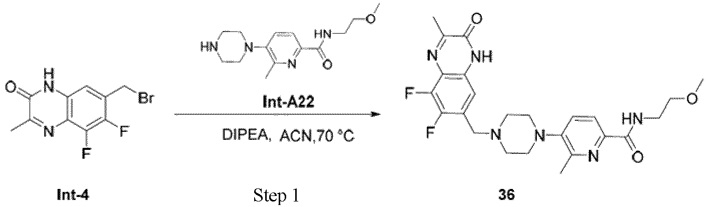

Except that compound **Int-A22** was used to replace compound Int-1 in Example 8, a crude product of compound **36** was synthesized by a method similar to that described in Example 8. The obtained crude product was purified by a preparative silica gel plate to obtain the title compound **36** (13.58 mg).

MS m/z (ES): 487.2 [M+H]⁺.

¹H NMR (400 MHz, DMSO-d₆) δ 8.43 - 8.36 (m, 1H), 7.81 (d, *J* = 8.4 Hz, 1H), 7.49 (d, *J* = 8.4 Hz, 1H), 7.17 (d, *J* = 4.8 Hz, 1H), 3.73 (s, 2H), 3.48 - 3.43 (m, 4H), 3.27 (s, 3H), 3.01 - 2.91 (m, 4H), 2.67 - 2.56 (m, 4H), 2.49 (s, 3H), 2.43 (s, 3H).

### Example 37: Preparation of compound 6-chloro-5-(4-((7-methoxy-2-methyl-3-oxo-3,4-dihydroquinoxalin-6-yl)methyl) piperazin-1-yl)-N-methylpicolinamide (37)

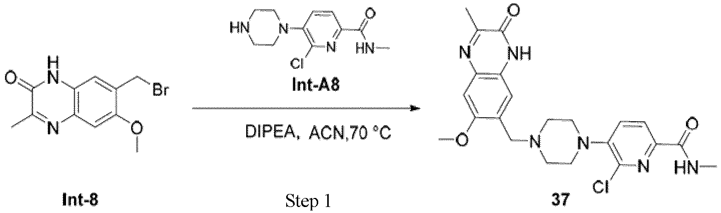

Except that compound **Int-8** was used to replace compound **Int-2** in Example 7, a crude product of compound **37** was synthesized by a method similar to that described in Example 7. The obtained crude product was purified by a preparative silica gel plate to obtain the title compound **37** (1.06 mg).

MS m/z (ES): 457.1 [M+H]⁺.

### Example 38: Preparation of compound 5-(4-((8-fluoro-2-methyl-3-oxo-3,4-dihydroquinoxalin-6-yl)methyl)piperazin-1-yl)-N,3-dimethylpicolinamide (38)

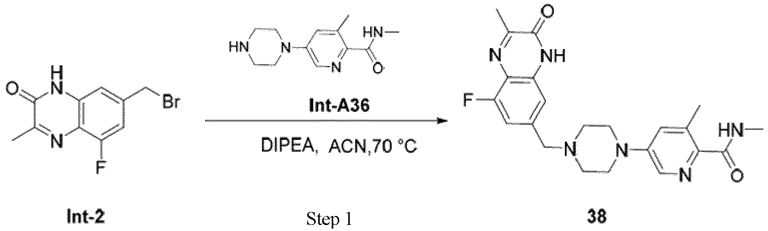

Except that compound **Int-A36** was used to replace compound **Int-1** in Example 1, a crude product of compound **38** was synthesized by a method similar to that described in Example 1. The obtained crude product was purified by a preparative silica gel plate to obtain the title compound **38** (9.72 mg).

MS m/z (ES): 425.2 [M+H]⁺.

¹H NMR (400 MHz, DMSO-d₆) δ 12.43 (s, 1H), 8.31 (q, *J* = 4.8 Hz, 1H), 8.11 (d, *J* = 2.4 Hz, 1H), 7.19-7.05 (m, 3H), 3.60 (s, 2H), 3.35-3.29 (m, 4H), 2.74 (d, *J* = 4.8 Hz, 3H), 2.60 - 2.51 (m, 7H), 2.41 (s, 3H).

### Example 39: Preparation of compound 3-fluoro-5-(4-((8-fluoro-2-methyl-3-oxo-3,4-dihydroquinoxalin-6-yl)methyl) piperazin-1-yl)-N-methylpicolinamide (39)

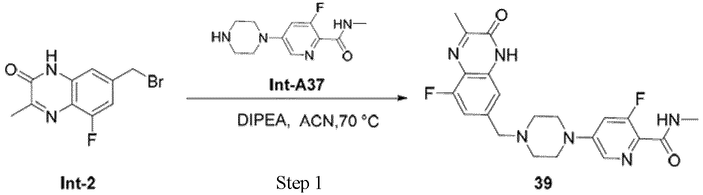

Except that compound **Int-A37** was used to replace compound **Int-1** in Example 1, a crude product of compound **39** was synthesized by a method similar to that described in Example 1. The obtained crude product was purified by a preparative silica gel plate to obtain the title compound **39** (2.81 mg).

MS m/z (ES): 429.1 [M+H]⁺.

### Example 40: Preparation of compound 5-(4-((2,7-dimethyl-3-oxo-3,4-dihydroquinoxalin-6-yl)methyl)piperazin-1-yl)-N,6-dimethylpicolinamide (40)

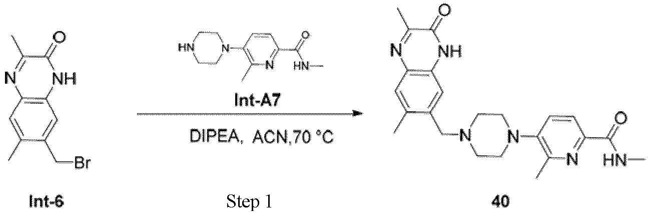

Except that compound **Int-6** was used to replace compound **Int-2** in Example 6, a crude product of compound **40** was synthesized by a method similar to that described in Example 6. The obtained crude product was purified by a preparative silica gel plate to obtain the title compound **40** (1.35 mg).

MS m/z (ES): 421.2 [M+H]⁺.

¹H NMR (400 MHz, DMSO-*d*₆) δ 12.18 (s, 1H), 8.42 (q, *J* = 4.4 Hz, 1H), 7.80 (d, *J* = 8.4 Hz, 1H), 7.51 (s, 1H), 7.48 (d, *J* = 8.4 Hz, 1H), 7.26 (s, 1H), 3.59 (s, 2H), 3.01 - 2.91 (m, 4H), 2.80 (d, *J* = 5.2 Hz, 3H), 2.65 - 2.55 (m, 4H), 2.50 (s, 3H), 2.40 (s, 3H), 2.39 (s, 3H).

### Example 41: Preparation of compound 5-(4-((7-methoxy-2-methyl-3-oxo-3,4-dihydroquinoxalin-6-yl)methyl)piperazin-1-yl )-N,6-dimethylpicolinamide (41)

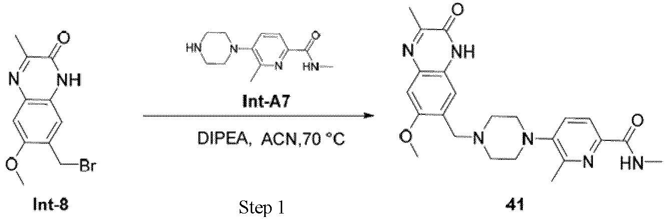

Except that compound **Int-8** was used to replace compound **Int-6** in Example 40, a crude product of compound **41** was synthesized by a method similar to that described in Example 40. The obtained crude product was purified by a preparative silica gel plate to obtain the title compound **41** (7.19 mg).

MS m/z (ES): 437.2 [M+H]⁺.

¹H NMR (400 MHz, DMSO-*d*₆) δ 12.16 (s, 1H), 8.42 (q, *J* = 4.8 Hz, 1H), 7.81 (d, *J* = 8.0 Hz, 1H), 7.48 (d, *J* = 8.4 Hz, 1H), 7.38 (s, 1H), 7.26 (s, 1H), 3.85 (s, 3H), 3.63 (s, 2H), 3.02 - 2.91 (m, 4H), 2.80 (d, *J* = 4.8 Hz, 3H), 2.67 - 2.57 (m, 4H), 2.50 (s, 3H), 2.39 (s, 3H).

### Example 42: Preparation of compound 5-(4-((2-ethyl-8-fluoro-3-oxo-3,4-dihydroquinoxalin-6-yl)methyl)piperazin-1-yl)-6-methyl-N-(oxetan-3-yl)picolinamide (42)

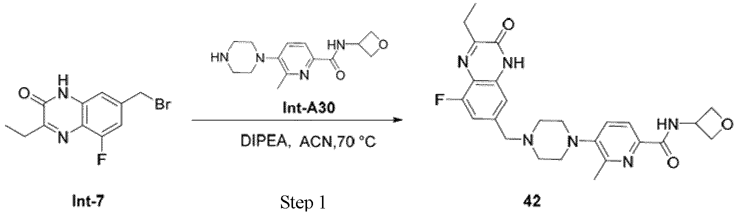

Except that compound **Int-A30** was used to replace compound **Int-A24** in Example 30, a crude product of compound **42** was synthesized by a method similar to that described in Example 30. The obtained crude product was purified by a preparative silica gel plate to obtain the title compound **42** (8.23 mg).

MS m/z (ES): 481.2 [M+H]⁺.

¹H NMR (400 MHz, DMSO-*d*₆) δ 12.43 (s, 1H), 9.08 (d, *J* = 7.2 Hz, 1H), 7.79 (d, *J* = 8.4 Hz, 1H), 7.49 (d, *J* = 8.4 Hz, 1H), 7.15 - 7.08 (m, 2H), 5.08 - 4.95 (m, 1H), 4.73 (t, J = 6.4 Hz, 2H), 4.66 (t, *J* = 6.8 Hz, 2H), 3.63 (s, 2H), 3.03 - 2.92 (m, 4H), 2.81 (q, *J* = 7.6 Hz, 2H), 2.66 - 2.56 (m, 4H), 2.53 (s, 3H), 1.22 (t, *J* = 7.6 Hz, 3H).

### Example 43: Preparation of compound N-cyclopropyl-5-(4-((2-ethyl-8-fluoro-3-oxo-3,4-dihydroquinoxalin-6-yl)methyl) piperazin-1-yl)-6-methylpicolinamide (43)

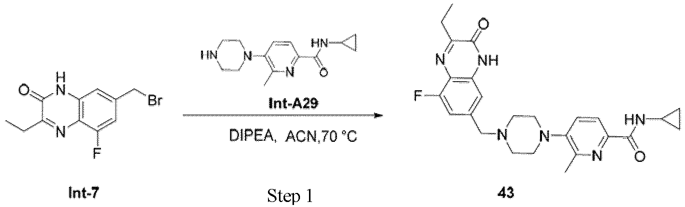

Except that compound **Int-A29** was used to replace compound **Int-A24** in Example 30, a crude product of compound **43** was synthesized by a method similar to that described in Example 30. The obtained crude product was purified by a preparative silica gel plate to obtain the title compound **43** (5.86 mg).

MS m/z (ES): 465.2 [M+H]⁺.

### Example 44: Preparation of compound 5-(4-((2-ethyl-8-fluoro-3-oxo-3,4-dihydroquinoxalin-6-yl)methyl)piperazin-1-yl)-N-((1s,3s)-3-hydroxycyclobutyl)-6-methylpicolinamide (44)

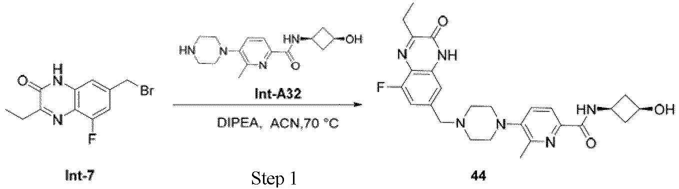

Except that compound **Int-A32** was used to replace compound **Int-A24** in Example 30, a crude product of compound **44** was synthesized by a method similar to that described in Example 30. The obtained crude product was purified by a preparative silica gel plate to obtain the title compound **44** (7.83 mg).

MS m/z (ES): 495.2 [M+H]⁺.

### Example 45: Preparation of compound 5-(4-((2-ethyl-8-fluoro-3-oxo-3,4-dihydroquinoxalin-6-yl)methyl)piperazin-1-yl)-N-((1r,3r)-3-hydroxycyclobutyl)-6-methylpicolinamide (45)

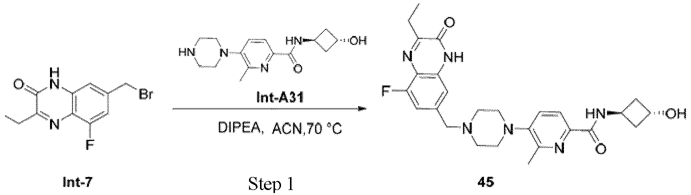

Except that compound **Int-A31** was used to replace compound **Int-A24** in Example 30, a crude product of compound **45** was synthesized by a method similar to that described in Example 30. The obtained crude product was purified by a preparative silica gel plate to obtain the title compound **45** (4.56 mg).

MS m/z (ES): 495.2 [M+H]⁺.

### Example 46: Preparation of compound (R)-5-(4-((8-fluoro-2-methyl-3-oxo-3,4-dihydroquinoxalin-6-yl)methyl)piperazin-1-yl)-6-methyl-N-(tetrahydrofuran-3-yl)picolinamide (46)

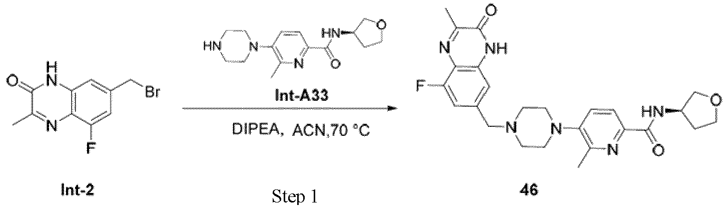

Except that compound **Int-A33** was used to replace compound Int-1 in Example 1, a crude product of compound **46** was synthesized by a method similar to that described in Example 1. The obtained crude product was purified by a preparative silica gel plate to obtain the title compound **46** (2.38 mg).

MS m/z (ES): 481.2 [M+H]⁺.

¹H NMR (400 MHz, DMSO-*d*₆) δ 12.44 (s, 1H), 8.36 (d, *J* = 7.2 Hz, 1H), 7.80 (d, *J* = 8.4 Hz, 1H), 7.49 (d, *J* = 8.4 Hz, 1H), 7.13 - 7.06 (m, 2H), 4.52 - 4.38 (m, 1H), 3.91 - 3.77 (m, 2H), 3.76 - 3.67 (m, 1H), 3.62 (s, 2H), 3.59 (dd, *J* = 8.8, 4.4 Hz, 1H), 3.03 - 2.91 (m, 4H), 2.64 - 2.55 (m, 4H), 2.50 (s, 3H), 2.41 (s, 3H), 2.23 - 2.11 (m, 1H), 1.99 - 1.88 (m, 1H).

### Example 47: Preparation of compound (R)-5-(4-((8-fluoro-2-methyl-3-oxo-3,4-dihydroquinoxalin-6-yl)methyl)piperazin-1-yl)-6-methyl-N-(tetrahydrofuran-3-yl)picolinamide (47)

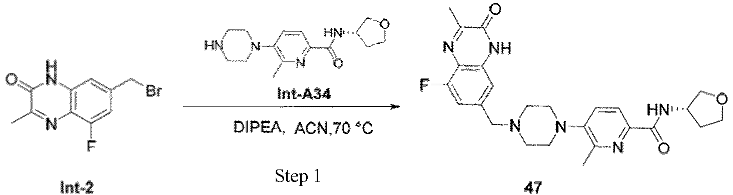

Except that compound **Int-A34** was used to replace compound **Int-1** in Example 1, a crude product of compound **47** was synthesized by a method similar to that described in Example 1. The obtained crude product was purified by a preparative silica gel plate to obtain the title compound **47** (6.98 mg).

MS m/z (ES): 481.2 [M+H]⁺.

¹H NMR (400 MHz, DMSO-*d*₆) δ 12.44 (s, 1H), 8.36 (d, *J* = 7.2 Hz, 1H), 7.80 (d, *J* = 8.4 Hz, 1H), 7.49 (d, *J* = 8.4 Hz, 1H), 7.13 - 7.06 (m, 2H), 4.52 - 4.38 (m, 1H), 3.91 - 3.77 (m, 2H), 3.76 - 3.67 (m, 1H), 3.62 (s, 2H), 3.59 (dd, *J* = 8.8, 4.4 Hz, 1H), 3.03 - 2.91 (m, 4H), 2.64 - 2.55 (m, 4H), 2.50 (s, 3H), 2.41 (s, 3H), 2.23 - 2.11 (m, 1H), 1.99 - 1.88 (m, 1H).

### Example 48: Preparation of compound (R)-5-(4-((2-ethyl-8-fluoro-3-oxo-3,4-dihydroquinoxalin-6-yl)methyl)piperazin-1-yl) -N-(2-hydroxypropyl)-6-methylpicolinamide (48)

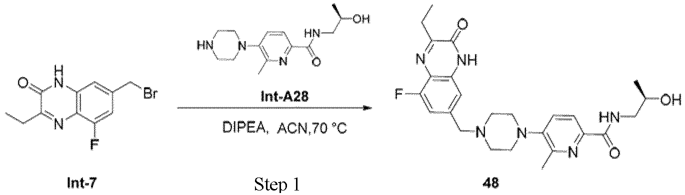

Except that compound **Int-A28** was used to replace compound **Int-A24** in Example 30, a crude product of compound **48** was synthesized by a method similar to that described in Example 30. The obtained crude product was purified by a preparative silica gel plate to obtain the title compound **48** (4.62 mg).

MS m/z (ES): 483.2 [M+H]⁺.

### Example 49: Preparation of compound (S)-5-(4-((2-ethyl-8-fluoro-3-oxo-3,4-dihydroquinoxalin-6-yl)methyl)piperazin-1-yl) -N-(2-hydroxypropyl)-6-methylpicolinamide (49)

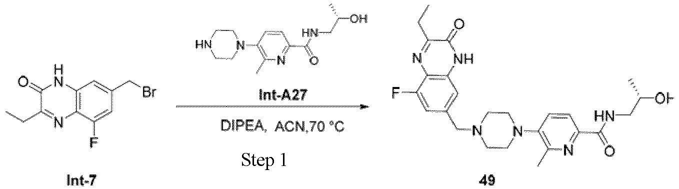

Except that compound **Int-A27** was used to replace compound **Int-A24** in Example 30, a crude product of compound **49** was synthesized by a method similar to that described in Example 30. The obtained crude product was purified by a preparative silica gel plate to obtain the title compound **49** (3.54 mg).

MS m/z (ES): 483.2 [M+H]⁺.

### Example 50: Preparation of compound N-(2-hydroxyethyl)-5-(4-((7-methoxy-2-methyl-3-oxo-3,4-dihydroquinoxalin-6-yl) methyl)piperazin-1-yl)-6-methylpicolinamide (50)

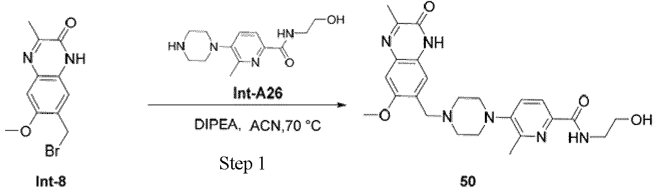

Except that compound **Int-8** was used to replace compound **Int-7** in Example 31, a crude product of compound **50** was synthesized by a method similar to that described in Example 31. The obtained crude product was purified by a preparative silica gel plate to obtain the title compound **50** (2.56 mg).

MS m/z (ES): 467.2 [M+H]⁺.

### Example 51: Preparation of compound 5-(4-((8-chloro-2-methyl-3-oxo-3,4-dihydroquinoxalin-6-yl)methyl)piperazin-1-yl)-N,6-dimethylpicolinamide (51)

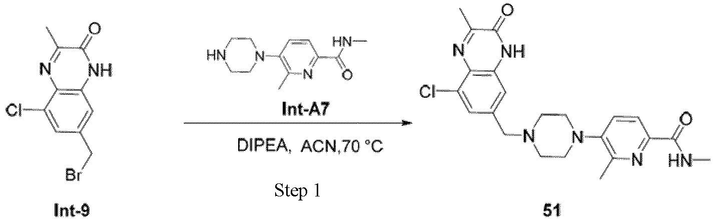

Except that compound **Int-9** was used to replace compound **Int-6** in Example 40, a crude product of compound **51** was synthesized by a method similar to that described in Example 40. The obtained crude product was purified by a preparative silica gel plate to obtain the title compound **51** (3.19 mg).

MS m/z (ES): 441.1 [M+H]⁺.

¹H NMR (400 MHz, DMSO-*d*₆) δ 8.42 (q, *J* = 5.2 Hz, 1H), 7.79 (d, *J* = 8.4 Hz, 1H), 7.48 (d, *J* = 8.4 Hz, 1H), 7.08 - 6.98 (m, 2H), 3.55 (s, 2H), 3.02 - 2.87 (m, 4H), 2.80 (d, *J* = 4.8 Hz, 3H), 2.64 - 2.54 (m, 4H), 2.48 (s, 3H), 2.32 (s, 3H).

### Example 52: Preparation of compound (R)-5-(4-((7,8-difluoro-2-methyl-3-oxo-3,4-dihydroquinoxalin-6-yl)methyl) piperazin -1-yl)-6-methyl-N-(tetrahydrofuran-3-yl)picolinamide (52)

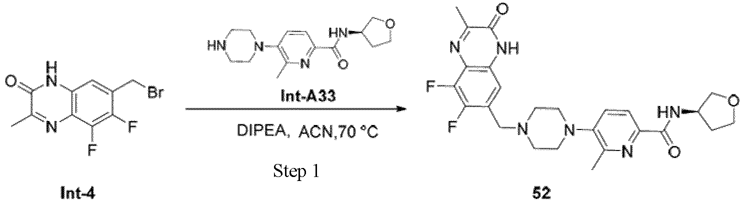

Except that compound Int-4 was used to replace compound Int-2 in Example 46, a crude product of compound **52** was synthesized by a method similar to that described in Example 46. The obtained crude product was purified by a preparative silica gel plate to obtain the title compound **52** (6.85 mg).

MS m/z (ES): 499.2 [M+H]⁺.

¹H NMR (400 MHz, DMSO-*d*₆) δ 8.37 (d, *J* = 7.6 Hz, 1H), 7.80 (d, *J* = 8.4 Hz, 1H), 7.48 (d, *J* = 8.4 Hz, 1H), 7.10 (d, *J* = 4.4 Hz, 1H), 4.51 - 4.40 (m, 1H), 3.91 - 3.79 (m, 2H), 3.75 - 3.67 (m, 3H), 3.59 (dd, *J* = 8.8, 4.4 Hz, 1H), 3.01 - 2.89 (m, 4H), 2.67 - 2.58 (m, 4H), 2.50 (s, 3H), 2.40 (s, 3H), 2.23 - 2.11 (m, 1H), 2.00 - 1.88 (m, 1H).

### Example 53: Preparation of compound 5-(4-((7,8-difluoro-2-methyl-3-oxo-3,4-dihydroquinoxalin-6-yl)methyl)piperazin-1-yl)-6-methyl-N-(oxetan-3-ylmethyl)picolinamide (53)

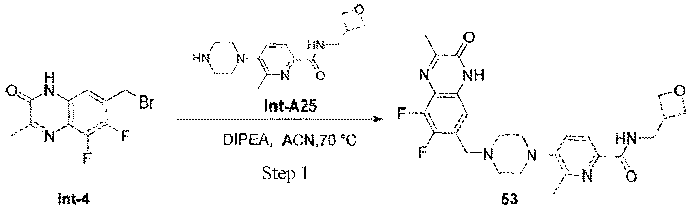

Except that compound **Int-A25** was used to replace compound **Int-A33** in Example 52, a crude product of compound **53** was synthesized by a method similar to that described in Example 52. The obtained crude product was purified by a preparative silica gel plate to obtain the title compound **53** (3.99 mg).

MS m/z (ES): 499.2 [M+H]⁺.

¹H NMR (400 MHz, DMSO-*d*₆) δ 8.68 (t, *J* = 6.0 Hz, 1H), 7.81 (d, *J* = 8.4 Hz, 1H), 7.48 (d, *J* = 8.4 Hz, 1H), 7.15 (d, *J* = 5.2 Hz, 1H), 4.60 (dd, *J* = 7.6, 6.0 Hz, 2H), 4.34 (t, *J* = 6.0 Hz, 2H), 3.72 (s, 2H), 3.56 (t, *J* = 6.8 Hz, 2H), 3.21 - 3.12 (m, 1H), 3.01 - 2.89 (m, 4H), 2.67 - 2.58 (m, 4H), 2.50 (s, 3H), 2.42 (s, 3H).

### Example 54: Preparation of compound 5-(4-((7,8-difluoro-2-methyl-3-oxo-3,4-dihydroquinoxalin-6-yl)methyl)piperazin-1-yl)-N-(2-fluoroethyl)-6-methylpicolinamide (54)

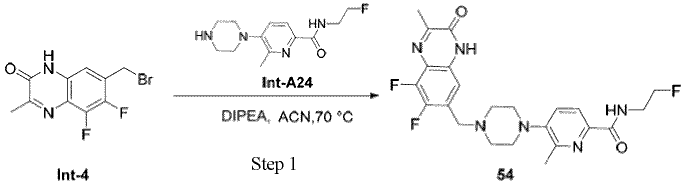

Except that compound **Int-A24** was used to replace compound **Int-A33** in Example 52, a crude product of compound **54** was synthesized by a method similar to that described in Example 52. The obtained crude product was purified by a preparative silica gel plate to obtain the title compound **54** (8.88 mg).

MS m/z (ES): 475.2 [M+H]⁺.

¹H NMR (400 MHz, DMSO-d₆) δ 12.50 (s, 1H), 8.61 (t, *J* = 5.6 Hz, 1H), 7.83 (d, *J* = 8.4 Hz, 1H), 7.51 (d, *J* = 8.0 Hz, 1H), 7.21 (s, 1H), 4.54 (dt, *J* = 47.6, 4.4 Hz, 2H), 3.67 - 3.52 (m, 4H), 3.08 - 2.88 (m, 4H), 2.77 - 2.57 (m, 4H), 2.51 (s, 3H), 2.44 (s, 3H).

### Example 55: Preparation of compound N-cyclopropyl-5-(4-((8-fluoro-2-methyl-3-oxo-3,4-dihydroquinoxalin-6-yl)methyl) piperazin-1-yl)-6-methylpicolinamide (55)

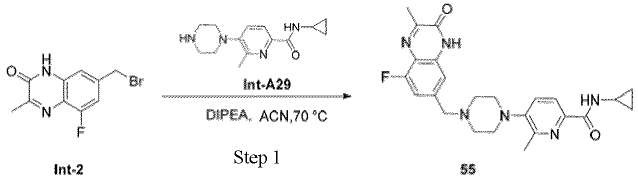

Except that compound **Int-A29** was used to replace compound **Int-1** in Example 1, a crude product of compound **55** was synthesized by a method similar to that described in Example 1. The obtained crude product was purified by a preparative silica gel plate to obtain the title compound **55** (6.01 mg).

MS m/z (ES): 451.2 [M+H]⁺.

¹H NMR (400 MHz, DMSO-*d*₆) δ 12.39 (s, 1H), 8.32 (d, *J* = 4.8 Hz, 1H), 7.79 (d, *J* = 8.4 Hz, 1H), 7.48 (d, *J* = 8.4 Hz, 1H), 7.14 - 7.06 (m, 2H), 3.62 (s, 2H), 3.03 - 2.91 (m, 4H), 2.89 - 2.80 (m, 1H), 2.63 - 2.55 (m, 4H), 2.48 (s, 3H), 2.41 (s, 3H), 0.72 - 0.66 (m, 2H), 0.66 - 0.60 (m, 2H).

### Example 56: Preparation of compound 5-(4-((2-cyclopropyl-8-fluoro-3-oxo-3,4-dihydroquinoxalin-6-yl)methyl)piperazin-1-yl)-N,6-dimethylpicolinamide (56)

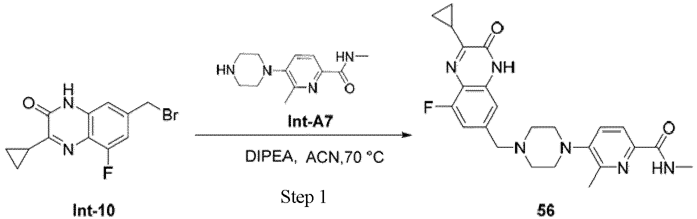

Except that compound **Int-10** was used to replace compound **Int-2** in Example 6, a crude product of compound **55** was synthesized by a method similar to that described in Example 6. The obtained crude product was purified by a preparative silica gel plate to obtain the title compound **55** (40.2 mg).

MS m/z (ES): 451.2 [M+H]⁺.

¹H NMR (400 MHz, DMSO-*d*₆) δ 12.45 (s, 1H), 8.46-8.37 (m, 1H), 7.80 (d, *J* = 8.0 Hz, 1H), 7.48 (d, *J* = 8.4 Hz, 1H), 7.12 - 7.02 (m, 2H), 3.61 (s, 2H), 3.01 - 2.89 (m, 4H), 2.80 (d, *J* = 4.8 Hz, 3H), 2.73 - 2.64 (m, 1H), 2.63 - 2.54 (m, 4H), 2.49 (s, 3H), 1.12 - 1.03 (m, 4H).

### Example 57: Preparation of compound 6-chloro-5-(4-((2-cyclopropyl-8-fluoro-3-oxo-3,4-dihydroquinoxalin-6-yl)methyl) piperazin-1-yl)-N-methylpicolinamide (57)

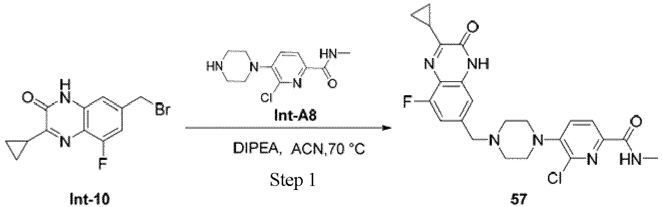

Except that compound **Int-A8** was used to replace compound **Int-A7** in Example 56, a crude product of compound **57** was synthesized by a method similar to that described in Example 56. The obtained crude product was purified by a preparative silica gel plate to obtain the title compound **57** (13.4 mg).

MS m/z (ES): 471.1 [M+H]⁺.

¹H NMR (400 MHz, DMSO-*d*₆) δ 12.44 (s, 1H), 8.46 - 8.38 (m, 1H), 7.94 (d, *J* = 8.0 Hz, 1H), 7.67 (d, *J* = 8.0 Hz, 1H), 7.11 - 7.04 (m, 2H), 3.61 (s, 2H), 3.20 - 3.04 (m, 4H), 2.79 (d, *J* = 4.8 Hz, 3H), 2.73 - 2.64 (m, 1H), 2.63 - 2.54 (m, 4H), 1.12 - 1.03 (m, 4H).

### Pharmacological test evaluation

### Test Example 1: Proliferation inhibitory test of BRCA1-mutated MDA-MB-436 cells

### 1. Test principle

CCK-8 was used to measure the content of mitochondrial dehydrogenase in cells at different drug concentrations, and the luminescence intensity was used to reflect the cell activity. The IC₅₀ values of different compounds on BRCA1-mutated MDA-MB-436 cells were calculated using the survival rate, and the proliferation inhibitory effect of the compounds of the present disclosure on BRCA1-mutated MDA-MB-436 cells was studied to evaluate the anti-tumor efficacy of the test compounds at the cellular level.

### 2. Test materials

### 2.1. Test reagents and instruments:

Culture medium special for MDA-MB-436 cells (DMEM), Procell;
Fetal bovine serum (FBS), Hyclone;
Phosphate buffered saline (PBS), Gibico;
Dimethyl sulfoxide: DMSO, Sigma;
CCK-8, Beyotime;
96-well cell culture plate, Coring;
Centrifuge, Eppendorf;
CO₂ incubator, Thermo Scientific;
Microscope, OLYMMPUS;
Full-automatic cell counter, Gibco;
Multifunctional microplate reader (Omega), BMG.

### 2.2. Test cells:

MDA-MB-436 cells, purchased from Procell.

### 2.3. Compounds

Test drugs: Compounds AZD5305 and AZD9574 were purchased from Chengdu Dingdangchem Medical Technology Co., Ltd;
Test drugs: Compounds 1-57 were sequentially prepared by the synthesis methods in Examples 1-57, respectively.

### 3. Test method

### 3.1. Test steps

On Day -1, the cells were inoculated into the 96-well cell culture plate at a fixed cell density and then placed in the 5% CO₂ cell incubator for culture overnight at 37°C.

On Day 0, the compound to be tested was prepared into a 10 mM stock solution and diluted into 9 concentration points at the concentration of 1,000 nM, 300 nM, 100 nM, 30 nM, 10 nM, 3 nM, 1 nM, 0.3 nM and 0.1 nM in two duplicated wells. A fixed volume of the diluted compound was added to the 96-well cell culture plate and then placed in the 5% CO₂ cell incubator for culture at 37°C for 5 days.

On Day 5, a fixed volume of CCK-8 was added to the 96-well cell culture plate and incubated in the 5% CO₂ cell incubator at 37°C for 1.5 h, and the plate was read by a chemiluminescence module of the multifunctional microplate reader.

### 3.2. Data analysis

$Survival percentage \left(%\right) = \frac{Test group - Blank group}{DMSO group - Blank group} \times 100$

With the logarithmic value of the concentration as an X axis and the survival percentage as a Y axis, a dose-effect curve was fitted by using a log(inhibitor) vs. response-Variable slop (Four parameters) formula of the analysis software Graphpad Prism 9, thereby obtaining the IC₅₀ value of the anti-proliferation inhibitory activity of each compound against BRCA1-mutated MDA-MB-436 cells.

### 4. Test results

The inhibitory activity of the compounds against the proliferation of BRCA1-mutated MDA-MB-436 cells was determined according to the above method. The results were shown in Table 1.

**Table 1. Inhibitory activity of compounds against BRCA1-mutated MDA-MB-436 cells**

| Compound number | IC₅₀ (nM) | Compound number | IC₅₀(nM) |
|---|---|---|---|
| AZD5305 | 0.67 | AZD9574 | 21.82 |
| 6 | 2.06 | 7 | 2.31 |
| 8 | 11.11 | 9 | 5.84 |
| 10 | 3.63 | 19 | 8.31 |
| 22 | 6.95 | 28 | 4.53 |
| 29 | 9.83 | 30 | 5.94 |
| 31 | 8.77 | 32 | 5.24 |
| 33 | 6.91 | 34 | 5.87 |
| 37 | 4.88 | 41 | 6.49 |
| 42 | 4.02 | 46 | 8.38 |
| 51 | 1.05 | 54 | 4.97 |
| 55 | 2.20 | | |

### 5. Test conclusion:

As can be seen from the test data of the inhibitory activity of the compounds against BRCA1-mutated MDA-MB-436 cells in Table 1, the compounds of the present application have a strong cell proliferation inhibitory activity against BRCA1-mutated MDA-MB-436 cells, and have obvious advantages compared with the positive control AZD9574.

### Test Example 2: Proliferation inhibitory test of BRCA1-mutated HCC1395 cells

### 1. Test principle

CCK-8 was used to measure the content of mitochondrial dehydrogenase in cells at different drug concentrations, and the luminescence intensity was used to reflect the cell activity. The IC₅₀ values of different compounds on BRCA1-mutated HCC1395 cells were calculated using the survival rate, and the proliferation inhibitory effect of the compounds of the present disclosure on BRCA1-mutated HCC1395 cells was studied to evaluate the anti-tumor efficacy of the test compounds at the cellular level.

### 2. Test materials

### 2.1. Test reagents and instruments:

RPMI-1640 culture medium, Hyclone;
Fetal bovine serum (FBS), Gibco;
Phosphate buffered saline (PBS), Wisent Biotechnology (Nanjing) Co., Ltd;
Dimethyl sulfoxide: DMSO, Sigma;
CCK-8, Beyotime;
96-well cell culture plate, Coring;
Centrifuge, Eppendorf;
CO₂ incubator, Thermo Scientific;
Microscope, OLYMMPUS;
Full-automatic cell counter, Gibco;
Multifunctional microplate reader (Omega), BMG.

### 2.2. Test cells:

HCC1395 cells, purchased from ATCC.

### 2.3. Compounds

Test compounds were obtained by the same method as recorded in **Test Example 1.**

### 3. Test method

### 3.1. Test steps

On Day -1, the cells were inoculated into the 96-well cell culture plate at a fixed cell density and then placed in the 5% CO₂ cell incubator for culture overnight at 37°C.

On Day 0, the compound to be tested was prepared into a 10 mM stock solution and diluted into 9 concentration points at the concentration of 1,000 nM, 300 nM, 100 nM, 30 nM, 10 nM, 3 nM, 1 nM, 0.3 nM and 0.1 nM in two duplicated wells. A fixed volume of the diluted compound was added to the 96-well cell culture plate and then placed in the 5% CO₂ cell incubator for culture at 37°C for 5 days.

On Day 5, a fixed volume of CCK-8 was added to the 96-well cell culture plate and incubated in the 5% CO₂ cell incubator at 37°C for 1.5 h, and the plate was read by a chemiluminescence module of the multifunctional microplate reader.

### 3.2. Data analysis

$Survival percentage \left(%\right) = \frac{Test group - Blank group}{DMSO group - Blank group} \times 100$

With the logarithmic value of the concentration as an X axis and the survival percentage as a Y axis, a dose-effect curve was fitted by using a log(inhibitor) vs. response-Variable slop (Four parameters) formula of the analysis software Graphpad Prism 9, thereby obtaining the IC₅₀ value of the anti-proliferation inhibitory activity of each compound against BRCA1-mutated HCC1395 cells.

### 4. Test results

The inhibitory activity of the compounds against the proliferation of BRCA1-mutated HCC1395 cells was determined according to the above method. The results were shown in Table 2.

**Table 2. Inhibitory activity of compounds against BRCA1-mutated HCC1395 cells**

| Compound number | IC₅₀(nM) | Compound number | IC₅₀(nM) |
|---|---|---|---|
| AZD9574 | 9.93 | AZD5305 | 1.76 |
| 6 | 2.45 | 9 | 2.29 |
| 28 | 2.03 | 32 | 1.82 |
| 37 | 0.95 | 51 | 1.31 |
| 55 | 2.56 | | |

### 5. Test conclusion:

As can be seen from the test data of the inhibitory activity of the compounds against the BRCA1-mutated HCC1395 cells in Table 2, the compounds of the present application have a strong cell proliferation inhibitory activity against BRCA1-mutated HCC1395 cells, and have certain advantages compared with the positive control AZD9574.

### Test Example 3: PARP1 and PARP2 enzyme activity test

### 1. Test purpose

This test is used to evaluate the effect of the test compounds on the PARP1 and PARP2 enzyme activities, and the IC₅₀ value of the test compounds on the PARP1 and PARP2 enzymes was calculated using the inhibitory rate.

### 2. Test materials

### 2.1. Compounds:

Test compounds were obtained by the same method as recorded in **Test Example 1.**

### 2.2. Test reagents and instruments

PARP1 Chemiluminescent assay kit, BPS, 80551;
PARP2 Chemiluminescent assay kit, BPS, 80552;
PBS, In house, 20210819;
Tween-20, Sigma, P9416;
96-well polypropylene plate, Nunc, 249944;
Centrifuge, XiangYi, TDZ5-WS;
Plate reader, BMG, PHERAstar FSX.

### 3. Test method

### 3.1. Preparation of compounds

Preparation of 10 mM compound storage solutions: Compound powders were dissolved in 100% DMSO to prepare 10 mM compound storage solutions, respectively.

### 3.2. Enzyme reaction process

(1) A 5x histone mixture was diluted with PBS at 1:5, added into each well at 50 µL, and incubated overnight at 4°C.
(2) Washing was performed with 200 µL of PBST buffer for three times, and incubation was performed in Blocking buffer for 90 min.
(3) Washing was performed with 200 µL of PBST buffer for three times.
(4) 5 µL of an inhibitor solution was added to each well.
(5) 20 µL of diluted PARP was added to each well.
(6) 25 µL of biotinylated substrate was added to each well and incubated at room temperature for 1 h.
(7) The wells were washed with 200 µL of PBST buffer for three times and sucked to dryness with clean paper towels.
(8) 50 µL of diluted Streptavidin-HRP was added to each well and incubated for 30 min.
(9) Washing was performed with 200 µL of PBST buffer for three times.
(10) 100 µL of a mixture of ELISA ECL substrate A and ELISA ECL substrate B was added to each well.
(11) A chemiluminescence signal value was read immediately.

### 3.3. Data analysis

$Y = Bottom + \left(Top-Bottom\right) / \left(1+{10}^{∧}\left(\left(LogIC50-X\right)∗Hillslope\right)\right)$

With the logarithmic value of the concentration of the compound as an X axis and the chemiluminescence signal as a Y axis, Top and Bottom were platforms with the same unit as Y, LogIC50 was a logarithmic unit same as X, Hillslop was Slop factor or Hill slope, and the IC₅₀ value was fitted by using a nonlinear regression equation.

### 4. Test results

The enzymatic inhibitory activity of the compounds of the present application against PARP1 and PARP2 was determined according to the above method. The results were shown in Table 3.

**Table 3. Enzymatic inhibitory activity of compounds against PARP1 and PARP2**

| Compound number | PARP1 IC₅₀(nM) | PARP2 IC₅₀(nM) |
|---|---|---|
| AZD5305 | 0.5262 | 21.03 |
| AZD9574 | 0.6021 | 3479 |
| 1 | 0.9645 | 998.4 |
| 6 | 0.6383 | 2666 |
| 9 | 1.163 | 2663 |
| 32 | 0.7174 | 280.7 |
| 55 | 0.7041 | 3716 |
| 56 | 0.5531 | 226.9 |

### 5. Test conclusion:

As can be seen from the test data of the enzymatic inhibitory activity of the compounds against PARP1 and PARP2 in Table 3, the compounds of the present application have a strong inhibitory activity against PARP1 at enzymatic level, and a weak inhibitory effect on PARP2, and the enzymatic selectivity of some compounds for PARP1 and PARP2 is comparable to that of the positive control AZD9574. The compounds of the present application have obvious advantages compared with the compound AZD5305, and can reduce hematotoxicity induced by PARP2.

### Test Example 4: Study on pharmacokinetics of rats

### 1. Test principle

With SD rats as test animals, the plasma concentration in the plasma of rats at different time points after oral administration of the compounds of the present application was determined by an LC-MS/MS method. Pharmacokinetic parameters of the compounds of the present application in rats were obtained to study pharmacokinetic characteristics.

### 2. Test materials

### 2.1. Compounds:

Test compounds were obtained by the same method as recorded in **Test Example 1.**

### 2.2. Test instruments:

Shimadzu LC-30A AB API4500 tandom mass spectrometer, vacuum blood collection tube, blood collection needle, filter paper, syringe, etc.

### 2.3. Test animals

SD rats, female, body weight 180-220 g, 3 rats per group. After being purchased, the animals were fed in an animal house for an adaptation period of at least 3 days and then used for testing after passing quarantine.

### 3. Test method

3.1. Grouping: The SD rats were randomly grouped according to Table 4, which had no statistical difference in the body weight between groups after grouping.

**Table 4 Test grouping and administration reaimen**

| Test compound | Administration route | Administration dose (mg/kg) | Administration concentration (mg/mL) | Administration volume (mL/kg) |
|---|---|---|---|---|
| 6 | Gavage | 10 | 2.5 | 4.0 |
| 9 | Gavage | 10 | 2.5 | 4.0 |
| 32 | Gavage | 3 | 0.75 | 4.0 |
| 34 | Gavage | 3 | 0.75 | 4.0 |
| 55 | Gavage | 3 | 0.75 | 4.0 |
| AZD9574 | Gavage | 3 | 0.75 | 4.0 |

### 3.2. Vehicle: 10% DMSO and 90% (20% SBE-β-CD in saline).

### 3.3. Collection and determination of blood samples:

According to Table 4, each compound was prepared into a clear solution with the vehicle, respectively, and each group was administered by gavage with the corresponding test drug. Before administration, and at 15 min, 30 min, 1 h, 2 h, 4 h, 6 h, 8 h and 24 h after the administration, a fixed volume of blood was collected through sinuses jugularis, placed in an EDTA-K2 anticoagulant tube, and centrifuged at 4,000 rpm for 10 min to separate the plasma in a centrifuge tube. The plasma was frozen in a refrigerator at -80°C.

### 3.4. Analysis method

The plasma stored at -80°C at each time point was taken out, and a fixed volume of acetonitrile was added thereto. After being vortexed at 1,500 rpm for 2 min, it was centrifuged for 15 min (3,500 r/min). A fixed volume of solution supernatant was taken for LC-MS/MS analysis.

### 4. Calculation of pharmacokinetic parameters:

The pharmacokinetic behaviors of the test compounds were fitted with a non-compartmental model, and the main pharmacokinetic parameters were calculated using DAS3.31 software. The results were shown in Table 5.

**Table 5. Pharmacokinetic parameters of the test compounds of the present application**

| Test compound | Administration method | Cₘₐₓ (ng/mL) | T_{1/2} (h) | AUCₗₐₛₜ (h·ng·mL⁻¹ ) | CI_F_obs (L/h/kg) |
|---|---|---|---|---|---|
| 6 | po.(10mg/kg) | 11.31×10³ | 6.07 | 72.60×10³ | 0.13 |
| 9 | po.(10mg/kg) | 31.90×10³ | 7.74 | 339.71×1 0³ | 0.03 |
| 32 | po.(3mg/kg) | 7180 | 7.39 | 50613 | 0.05 |
| 34 | po.(3mg/kg) | 3233 | 5.20 | 16953 | 0.18 |
| 55 | po.(3mg/kg) | 2207 | 6.26 | 21346 | 0.14 |
| AZD9574 | po.(3mg/kg) | 702 | 6.06 | 6455 | 0.37 |

### 5. Test conclusion:

As can be seen from the test results in Table 5, compared with the positive control AZD9574, the multiple compounds of the present application have higher blood exposure, higher maximum plasma concentration and lower clearance in animals, and thus have better pharmacokinetic properties.

## Claims

1. A compound having a structure of Formula (I) or a pharmaceutically acceptable salt, a stereoisomer, a tautomer or a N-oxide thereof: 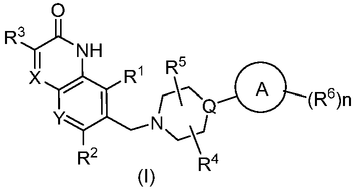 wherein:
X is selected from N, CH or CR^{a}, wherein R^{a} is selected from halogen, C₁₋₄ alkyl, C₃₋₆ cycloalkyl, -O-(C₁₋₄ alkyl) or halogenated C₁₋₄ alkyl;
Y is selected from N, CH or CR^{b}, wherein R^{b} is selected from halogen, cyano, C₁₋₄ alkyl, C₂₋₆ alkenyl, C₃₋₆ cycloalkyl, 4-6 membered heterocyclyl or -OR^{z}, wherein the C₁₋₄ alkyl, C₃₋₆ cycloalkyl or 4-6 membered heterocyclyl is unsubstituted or each independently substituted with one or more substituents selected from fluoro, cyano, hydroxy, C₁₋₄ alkyl or -O-(C₁₋₄ alkyl);
provided that: when X is selected from N or CH, Y is selected from CR^{b};
when X is selected from CR^{a}, Y is selected from N, CH or CR^{b};
R¹ is selected from hydrogen, halogen, cyano, C₁₋₄ alkyl, -O-(C₁₋₄ alkyl), -O-(halogenated C₁₋₄ alkyl) or halogenated C₁₋₄ alkyl;
R² is selected from hydrogen, F, Cl, Br, cyano, C₁₋₄ alkyl, C₃₋₆ cycloalkyl, -O-(C₁₋₄ alkyl) or halogenated C₁₋₄ alkyl;
R³ is selected from cyano, C₁₋₄ alkyl, C₃₋₆ cycloalkyl or 4-6 membered heterocyclyl, wherein the C₁₋₄ alkyl, C₃₋₆ cycloalkyl or 4-6 membered heterocyclyl is unsubstituted or each independently substituted with one or more substituents selected from F, cyano, C₁₋₄ alkyl, C₃₋₆ cycloalkyl, 4-6 membered heterocyclyl, -O-(C₁₋₄ alkyl) or halogenated C₁₋₄ alkyl;
Q is selected from N or CR^{c}, wherein R^{c} is selected from F, hydroxy, cyano, C₁₋₄ alkyl, -O-(C₁₋₄ alkyl) or halogenated C₁₋₄ alkyl;
R⁴ and R⁵ at each occurrence are each independently selected from hydrogen or C₁₋₄ alkyl, or R⁴ and R⁵ are connected to each other to form a ring; 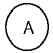
is selected from phenyl, or five-membered or six-membered heteroaryl containing 1 to 2 atoms selected from N, O or S;
R⁶ at each occurrence is each independently selected from hydrogen, halogen, cyano, -OR^{z}, -C(=O)-R^{z}, -C(=O)-NH-R^{z}, C₁₋₄ alkyl, C₃₋₆ cycloalkyl or 4-6 membered heterocyclyl, wherein the C₁₋₄ alkyl, C₃₋₆ cycloalkyl or 4-6 membered heterocyclyl is unsubstituted or each independently substituted with one or more substituents selected from F, cyano, hydroxy, C₁₋₄ alkyl, -O-(C₁₋₄ alkyl) or halogenated C₁₋₄ alkyl;
n is 1, 2 or 3; and
R^{z} at each occurrence is each independently selected from hydrogen, C₁₋₄ alkyl, C₃₋₆ cycloalkyl or 4-6 membered heterocyclyl, wherein the C₁₋₄ alkyl, C₃₋₆ cycloalkyl or 4-6 membered heterocyclyl is unsubstituted or each independently substituted with one or more substituents selected from F, cyano, hydroxy, C₁₋₄ alkyl, -O-(C₁₋₄ alkyl) or fluorinated C₁₋₄ alkyl.

2. The compound of Formula (I) or the pharmaceutically acceptable salt, the stereoisomer, the tautomer or the N-oxide thereof according to claim 1, **characterized in that**:
X is selected from N, CH or CR^{a}, wherein R^{a} is selected from F, Cl, Br, C₁₋₄ alkyl, C₃₋₆ cycloalkyl, -OMe or fluorinated C₁₋₄ alkyl; preferably, R^{a} is selected from F, Cl, Br, methyl, ethyl, isopropyl, cyclopropyl, -OMe, -CF₃, -CHF₂ or -CH₂F; more preferably, R^{a} is selected from F, Cl, Br, methyl, cyclopropyl, -OMe, -CHF₂ or -CH₂F;
Y is selected from N, CH or CR^{b}, wherein R^{b} is selected from F, Cl, Br, cyano, methyl, ethyl, isopropyl, cyclopropyl, cyclobutyl, oxetanyl, oxacyclohexyl or -OR^{z}, wherein the methyl, ethyl, isopropyl, cyclopropyl, cyclobutyl, oxetanyl or oxacyclohexyl is unsubstituted or each independently substituted with one or more substituents selected from fluoro, cyano, hydroxy, methyl or -OMe; preferably, R^{b} is selected from F, Cl, Br, cyano, methyl, ethyl, isopropyl, cyclopropyl, cyclobutyl, oxetanyl or -OR^{z}, wherein the methyl, ethyl, isopropyl, cyclopropyl or cyclobutyl is unsubstituted or each independently substituted with one or two substituents selected from fluoro, cyano, hydroxy or methyl;
provided that: when X is selected from N or CH, Y is selected from CR^{b};
when X is selected from CR^{a}, Y is selected from N, CH or CR^{b}.

3. The compound of Formula (I) or the pharmaceutically acceptable salt, the stereoisomer, the tautomer or the N-oxide thereof according to claim 1 or 2, **characterized in that**:
R¹ is selected from hydrogen, F, Cl, Br, cyano, methyl, ethyl, isopropyl, -OMe, -O-(fluorinated C₁₋₄ alkyl) or fluorinated C₁₋₄ alkyl; and preferably, R¹ is selected from hydrogen, F, Cl, methyl, -OMe, -CHF₂ or -CH₂F.

4. The compound of Formula (I) or the pharmaceutically acceptable salt, the stereoisomer, the tautomer or the N-oxide thereof according to any one of claims 1-3, **characterized in that**:
R² is selected from hydrogen, F, Cl, Br, cyano, methyl, ethyl, cyclopropyl, -OMe or fluorinated C₁₋₄ alkyl; and preferably, R² is selected from hydrogen, F, Cl, Br, methyl, cyclopropyl, -OMe, -CHF₂ or -CH₂F.

5. The compound of Formula (I) or the pharmaceutically acceptable salt, the stereoisomer, the tautomer or the N-oxide thereof according to any one of claims 1-4, **characterized in that**:
R³ is selected from cyano, methyl, ethyl, isopropyl, cyclopropyl, cyclobutyl or oxetanyl, wherein the methyl, ethyl, isopropyl, cyclopropyl, cyclobutyl or oxetanyl is unsubstituted or each independently substituted with one or more substituents selected from F, cyano, methyl, cyclopropyl, -OMe or fluorinated C₁₋₄ alkyl; and
preferably, R³ is selected from cyano, methyl, ethyl, isopropyl, cyclopropyl, cyclobutyl or oxetanyl, wherein the methyl, ethyl, isopropyl, cyclopropyl, cyclobutyl or oxetanyl is unsubstituted or each independently substituted with one or two substituents selected from F, cyano, -OMe, -CHF₂ or -CH₂F.

6. The compound of Formula (I) or the pharmaceutically acceptable salt, the stereoisomer, the tautomer or the N-oxide thereof according to any one of claims 1-5, **characterized in that**:
Q is selected from N or CR^{c}, wherein R^{c} is selected from F, hydroxy, cyano, methyl, ethyl, -OMe or fluorinated C₁₋₄ alkyl;
preferably, Q is selected from N or CR^{c}, wherein R^{c} is selected from F, hydroxy, cyano, methyl, -OMe, -CHF₂ or -CH₂F;
more preferably, Q is selected from N or CR^{c}, wherein R^{c} is selected from F, hydroxy, -OMe or methyl;
R⁴ and R⁵ at each occurrence are each independently selected from hydrogen, methyl or ethyl, or R⁴ and R⁵ are connected to each other to form a ring;
preferably, R⁴ and R⁵ at each occurrence are each independently selected from hydrogen or methyl, or R⁴ and R⁵ are connected to each other to form a ring, forming 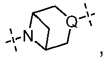 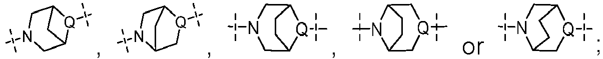 and
more preferably, R⁴ and R⁵ at each occurrence are each independently selected from hydrogen or methyl, or R⁴ and R⁵ are connected to each other to form a ring, forming

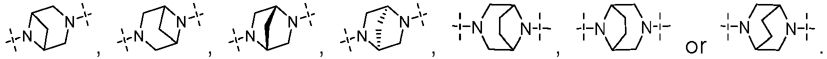

7. The compound of Formula (I) or the pharmaceutically acceptable salt, the stereoisomer, the tautomer or the N-oxide thereof according to any one of claims 1-6, **characterized in that**: 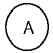
is selected from phenyl, pyridyl, pyrimidyl, pyrazinyl, pyridazinyl, pyrazolyl, oxazolyl, isooxazolyl, thienyl or thiazolyl;
preferably, 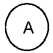 is selected from phenyl, pyridyl, pyrazolyl, oxazolyl, thienyl or thiazolyl;
more preferably, 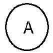 is selected from 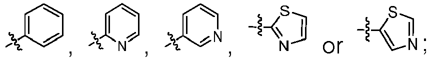 R⁶ at each occurrence is each independently selected from hydrogen, F, Cl, Br, cyano, -OR^{z}, -C(=O)-R^{z}, -C(=O)-NH-R^{z}, methyl, ethyl, isopropyl, cyclopropyl, cyclobutyl or oxetanyl, wherein the methyl, ethyl, isopropyl, cyclopropyl, cyclobutyl or oxetanyl is unsubstituted or each independently substituted with one or more substituents selected from F, cyano, hydroxy, methyl, -OMe, -CF₃ or -CHF₂;
preferably, R⁶ at each occurrence is each independently selected from hydrogen, F, Cl, Br, cyano, -OR^{z}, -C(=O)-R^{z}, -C(=O)-NH-R^{z}, methyl, ethyl, isopropyl, cyclopropyl, cyclobutyl or oxetanyl, wherein the methyl, ethyl, isopropyl, cyclopropyl, cyclobutyl or oxetanyl is unsubstituted or each independently substituted with one or two substituents selected from F, hydroxy or -OMe;
R^{z} at each occurrence is each independently selected from hydrogen, methyl, ethyl, isopropyl, cyclopropyl, cyclobutyl, oxetanyl or oxacyclopentyl, wherein the methyl, ethyl, isopropyl, cyclopropyl, cyclobutyl or oxacyclopentyl is unsubstituted or each independently substituted with one or more substituents selected from fluoro, cyano, -OH, -OMe, oxetanyl or methyl; and
n is 1 or 2.

8. A compound having a structure of Formula (II) or a pharmaceutically acceptable salt, a stereoisomer, a tautomer or a N-oxide thereof: 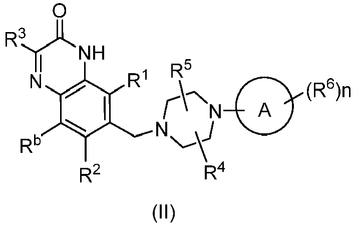 wherein:
R¹ is selected from hydrogen, F, Cl, Br, cyano, methyl, ethyl, isopropyl or -OMe;
R² is selected from hydrogen, halogen, cyano, C₁₋₄ alkyl, C₃₋₆ cycloalkyl, -O-(C₁₋₄ alkyl) or fluorinated C₁₋₄ alkyl;
R³ is selected from cyano, methyl, ethyl, isopropyl, cyclopropyl, cyclobutyl or oxetanyl, wherein the methyl, ethyl, isopropyl, cyclopropyl, cyclobutyl or oxetanyl is unsubstituted or each independently substituted with one or more substituents selected from F, cyano, methyl, cyclopropyl or -OMe;
R^{b} is selected from F, Cl, Br, cyano, C₁₋₄ alkyl, C₃₋₆ cycloalkyl, 4-6 membered heterocyclyl or -OR^{z}, wherein the C₁₋₄ alkyl, C₃₋₆ cycloalkyl or 4-6 membered heterocyclyl is unsubstituted or each independently substituted with one or more substituents selected from fluoro, cyano, hydroxy, C₁₋₄ alkyl or -O-(C₁₋₄ alkyl);
R⁴ and R⁵ at each occurrence are each independently selected from hydrogen or C₁₋₄ 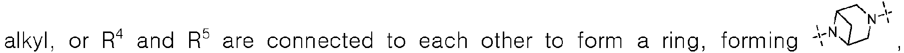 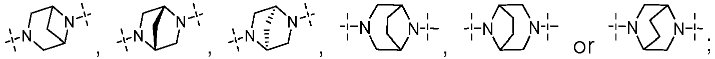 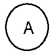
is selected from phenyl, pyridyl, pyrimidyl, pyrazinyl, pyridazinyl, pyrazolyl, oxazolyl, isooxazolyl, thienyl or thiazolyl;
R⁶ at each occurrence is each independently selected from hydrogen, halogen, cyano, -OR^{z}, -C(=O)-R^{z}, -C(=O)-NH-R^{z}, C₁₋₄ alkyl, C₃₋₆ cycloalkyl or 4-6 membered heterocyclyl, wherein the C₁₋₄ alkyl, C₃₋₆ cycloalkyl or 4-6 membered heterocyclyl is unsubstituted or each independently substituted with one or more substituents selected from F, cyano, hydroxy, C₁₋₄ alkyl, -O-(C₁₋₄ alkyl) or halogenated C₁₋₄ alkyl;
n is 1, 2 or 3; and
R^{z} at each occurrence is each independently selected from hydrogen, C₁₋₄ alkyl, C₃₋₆ cycloalkyl or 4-6 membered heterocyclyl, wherein the C₁₋₄ alkyl, C₃₋₆ cycloalkyl or 4-6 membered heterocyclyl is unsubstituted or each independently substituted with one or more substituents selected from F, cyano, hydroxy, C₁₋₄ alkyl, -O-(C₁₋₄ alkyl), 4-6 membered heterocyclyl or fluorinated C₁₋₄ alkyl.

9. A compound having a structure of Formula (III) or a pharmaceutically acceptable salt, a stereoisomer, a tautomer or a N-oxide thereof: 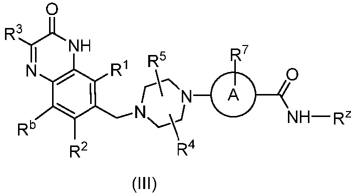 wherein:
R¹ is selected from hydrogen, F, Cl, methyl, -OMe, -CHF₂ or -CH₂F;
R² is selected from hydrogen, halogen, cyano, C₁₋₄ alkyl, C₃₋₆ cycloalkyl, -O-(C₁₋₄ alkyl) or halogenated C₁₋₄ alkyl;
R³ is selected from cyano, methyl, ethyl, isopropyl, cyclopropyl, cyclobutyl or oxetanyl, wherein the methyl, ethyl, isopropyl, cyclopropyl, cyclobutyl or oxetanyl is unsubstituted or each independently substituted with one or two substituents selected from F, cyano, methyl, cyclopropyl or -OMe;
R^{b} is selected from F, Cl, Br, cyano, methyl, ethyl, isopropyl, cyclopropyl, cyclobutyl, oxetanyl or -OR^{z}, wherein the methyl, ethyl, isopropyl, cyclopropyl or cyclobutyl is unsubstituted or each independently substituted with one or two substituents selected from fluoro, cyano, hydroxy or methyl;
R⁴ and R⁵ at each occurrence are each independently selected from hydrogen or C₁₋₄ alkyl, or R⁴ and R⁵ are connected to each other to form a ring, forming 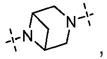 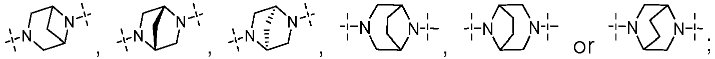 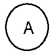
is selected from 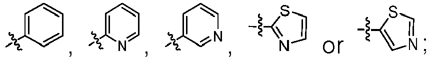
R⁷ is selected from hydrogen, F, Cl, Br, cyano, C₁₋₄ alkyl, C₃₋₆ cycloalkyl, 4-6 membered heterocyclyl or -OR^{z}, wherein the C₁₋₄ alkyl, C₃₋₆ cycloalkyl or 4-6 membered heterocyclyl is unsubstituted or each independently substituted with one or more substituents selected from F, cyano, hydroxy, C₁₋₄ alkyl, -O-(C₁₋₄ alkyl) or halogenated C₁₋₄ alkyl;
n is 1 or 2; and
R^{z} at each occurrence is each independently selected from hydrogen, C₁₋₄ alkyl, C₃₋₆ cycloalkyl or 4-6 membered heterocyclyl, wherein the C₁₋₄ alkyl, C₃₋₆ cycloalkyl or 4-6 membered heterocyclyl is unsubstituted or each independently substituted with one or more substituents selected from F, cyano, hydroxy, C₁₋₄ alkyl, -O-(C₁₋₄ alkyl), 4-6 membered heterocyclyl or fluorinated C₁₋₄ alkyl.

10. A compound having a structure of Formula (IV) or a pharmaceutically acceptable salt, a stereoisomer, a tautomer or a N-oxide thereof: 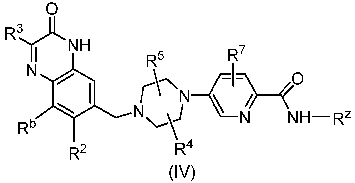 wherein,
R² is selected from hydrogen, F, Cl, Br, cyano, C₁₋₄ alkyl, C₃₋₆ cycloalkyl, -O-(C₁₋₄ alkyl) or fluorinated C₁₋₄ alkyl;
R³ is selected from cyano, methyl, ethyl, n-propyl, isopropyl, cyclopropyl, cyclobutyl or 4-6 membered heterocyclyl, wherein the methyl, ethyl, n-propyl, isopropyl, cyclopropyl, cyclobutyl or 4-6 membered heterocyclyl is unsubstituted or each independently substituted with one or more substituents selected from F, cyano, methyl or -OMe;
R^{b} is selected from F, Cl, Br, cyano, methyl, ethyl, n-propyl, isopropyl, cyclopropyl, cyclobutyl, oxetanyl or -OR^{z}, wherein the methyl, ethyl, n-propyl, isopropyl, cyclopropyl or cyclobutyl is unsubstituted or each independently substituted with one or two substituents selected from fluoro, cyano, hydroxy or methyl;
R⁴ and R⁵ at each occurrence are each independently selected from hydrogen, methyl or ethyl, or R⁴ and R⁵ are connected to each other to form a ring, forming 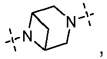 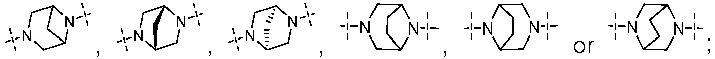
R⁷ is selected from hydrogen, halogen, cyano, -OR^{z}, C₁₋₄ alkyl, C₃₋₆ cycloalkyl or 4-6 membered heterocyclyl, wherein the C₁₋₄ alkyl, C₃₋₆ cycloalkyl or 4-6 membered heterocyclyl is unsubstituted or each independently substituted with one or more substituents selected from F, hydroxy, methyl, ethyl, methoxy or ethoxy;
n is 1 or 2; and
R^{z} at each occurrence is each independently selected from hydrogen, methyl, ethyl, n-propyl, isopropyl, cyclopropyl, cyclobutyl, oxetanyl or oxacyclopentyl, wherein the methyl, ethyl, n-propyl, isopropyl, cyclopropyl or cyclobutyl is unsubstituted or each independently substituted with one or more substituents selected from F, cyano, hydroxy, methyl, ethyl, methoxy, ethoxy, oxetanyl or fluoromethyl.

11. The compound or the pharmaceutically acceptable salt, the stereoisomer, the tautomer or the N-oxide thereof according to any one of claims 1, 8, 9 or 10, wherein the compound is selected from:

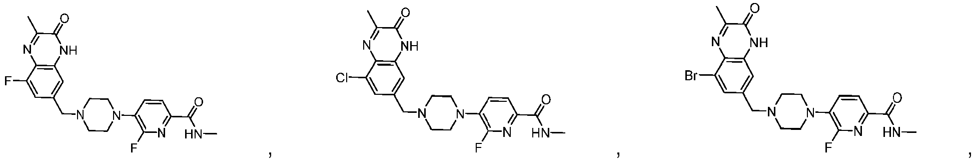

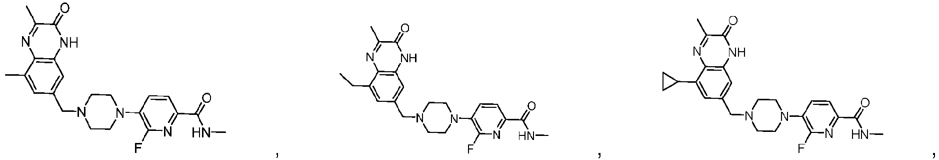

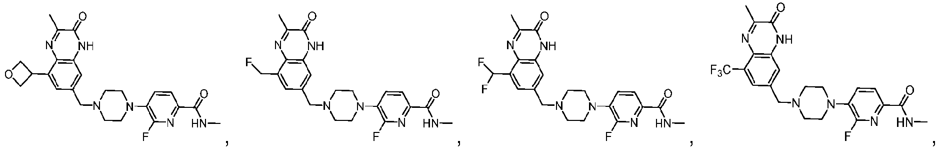

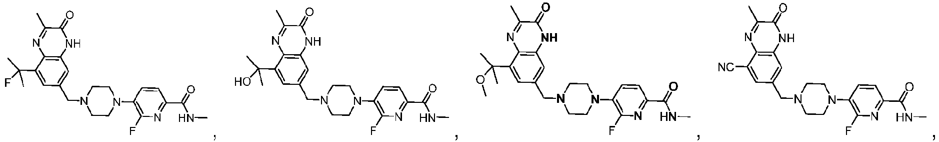

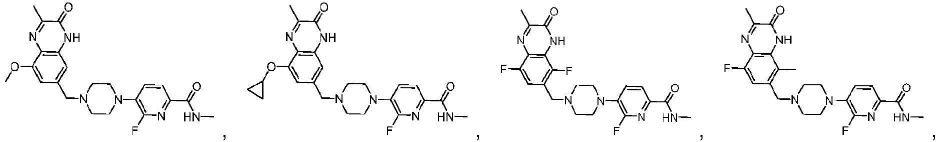

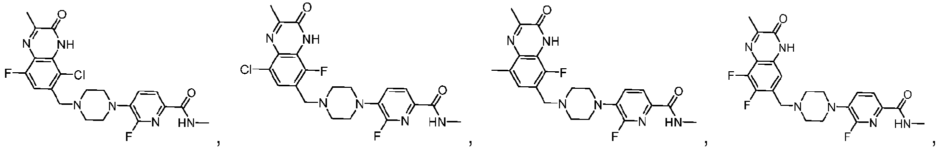

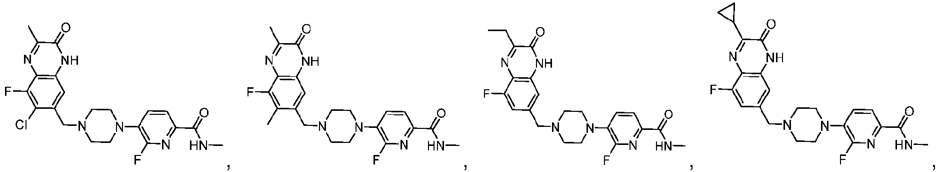

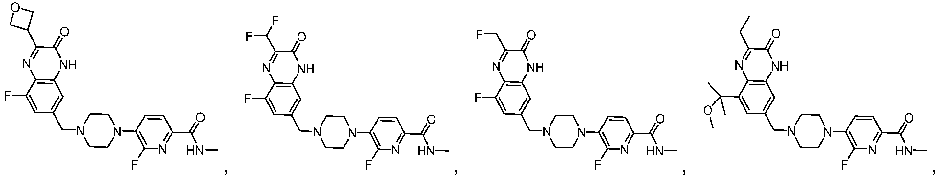

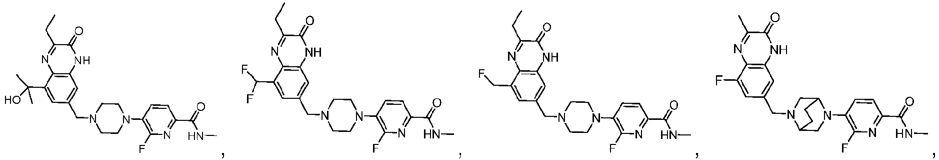

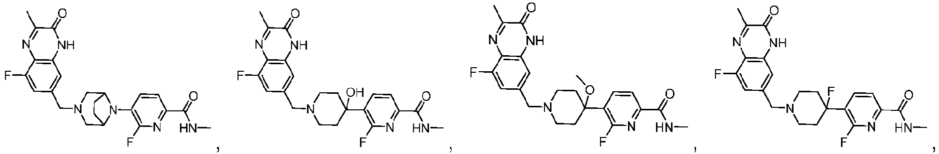

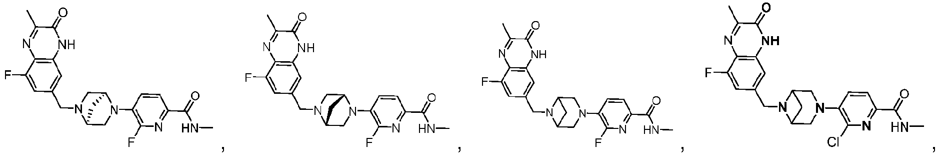

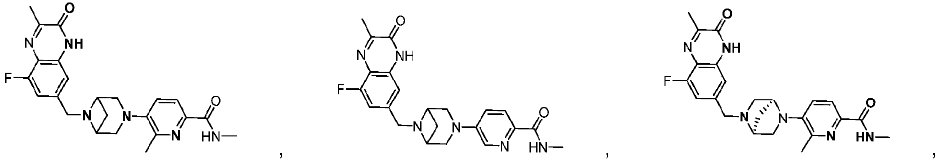

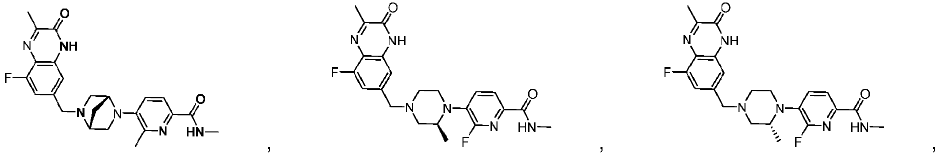

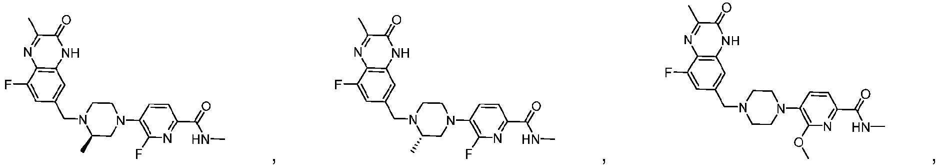

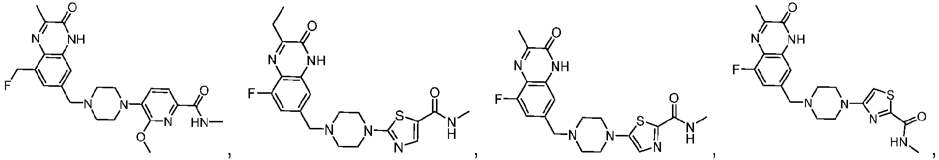

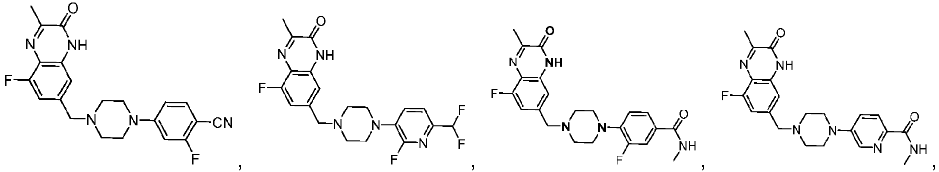

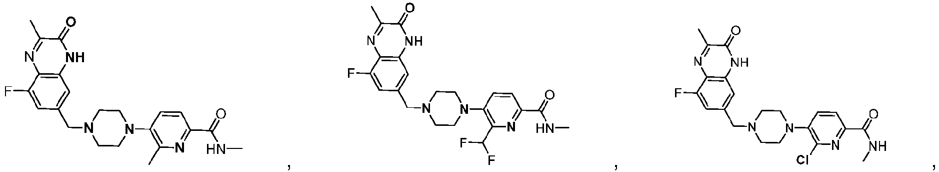

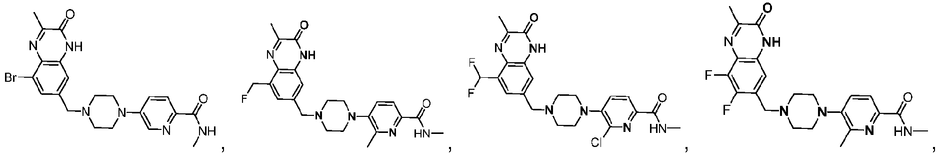

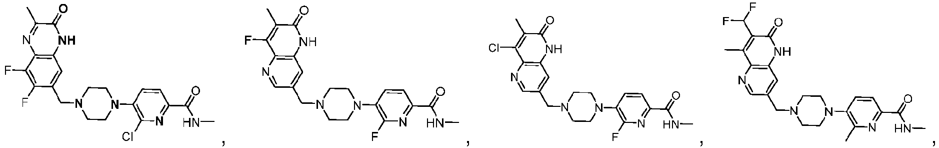

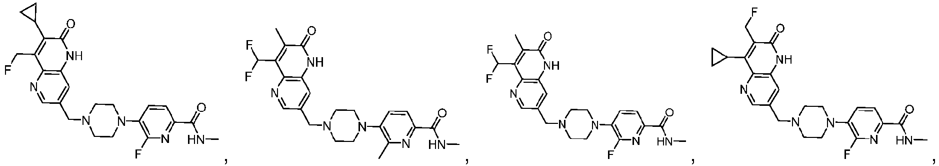

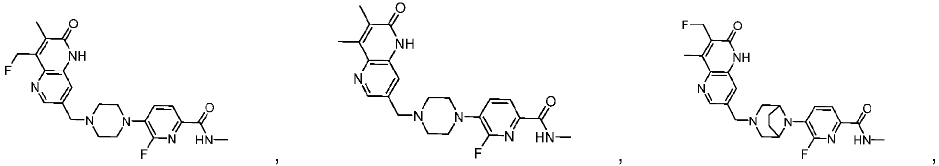

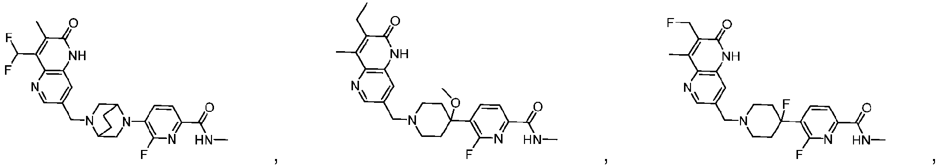

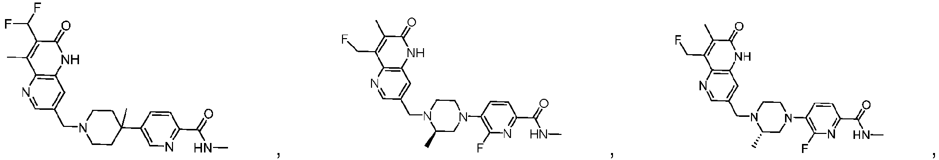

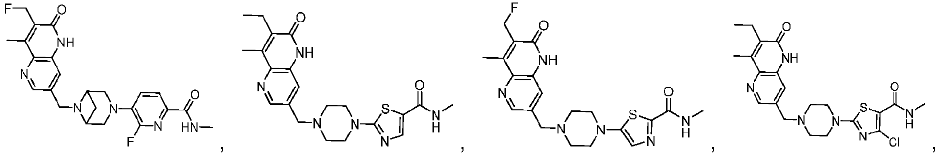

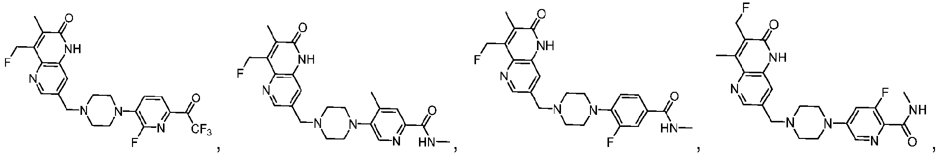

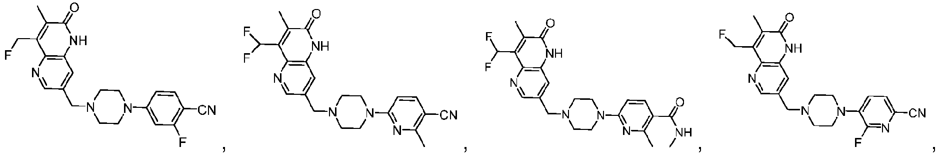

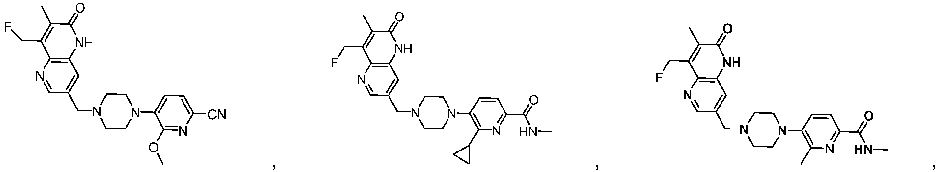

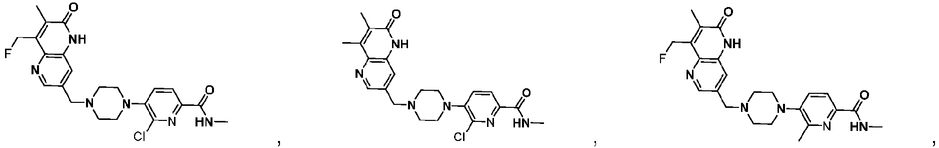

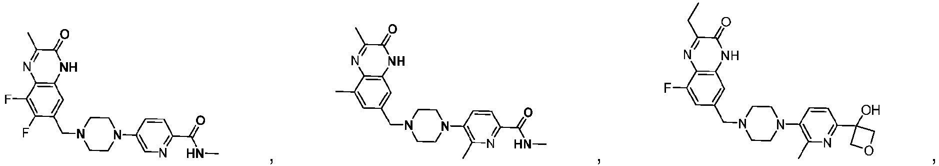

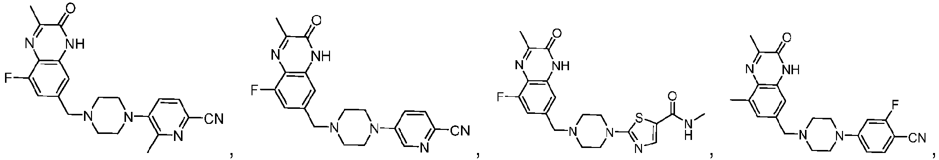

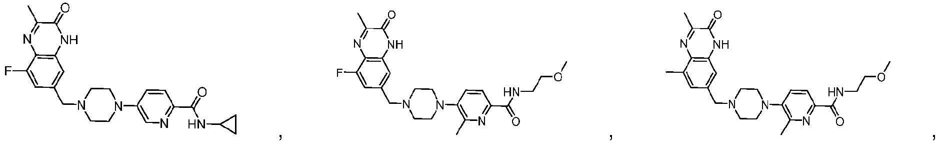

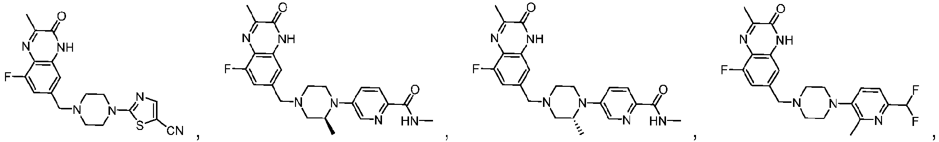

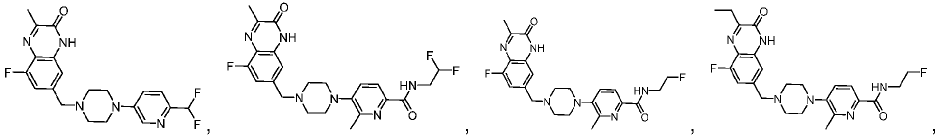

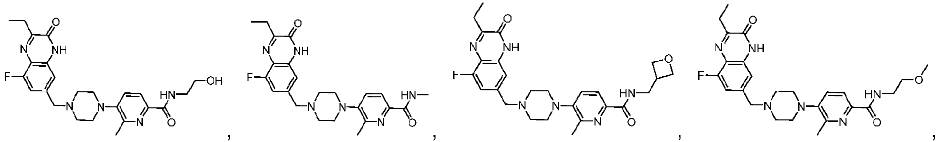

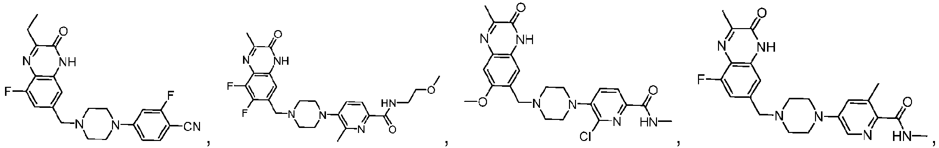

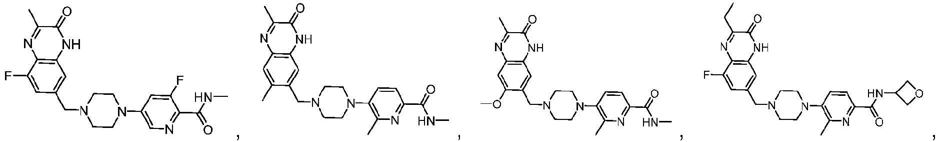

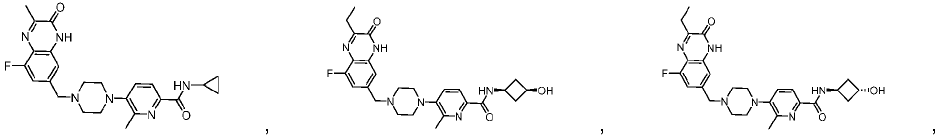

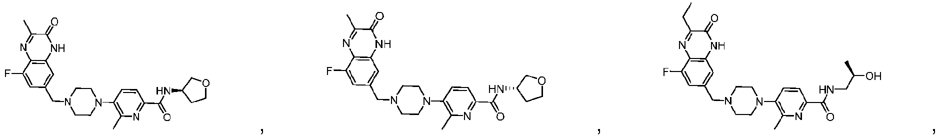

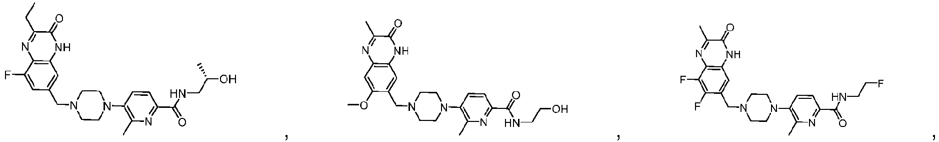

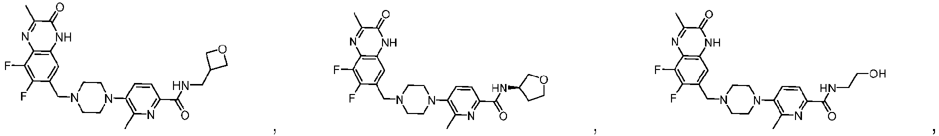

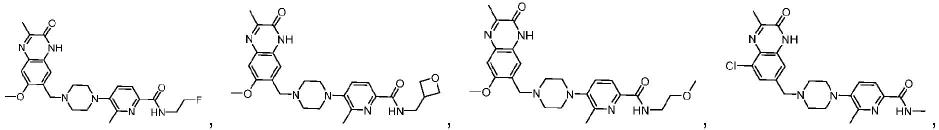

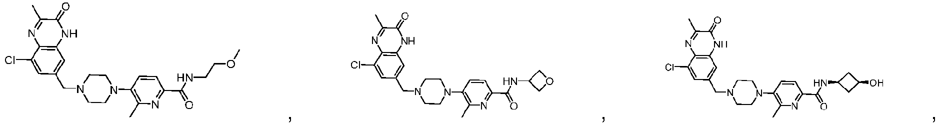

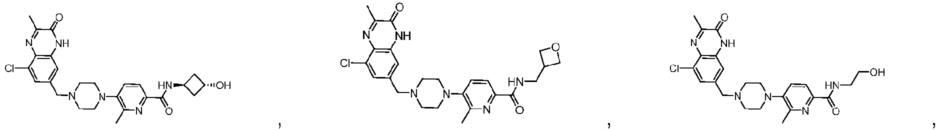

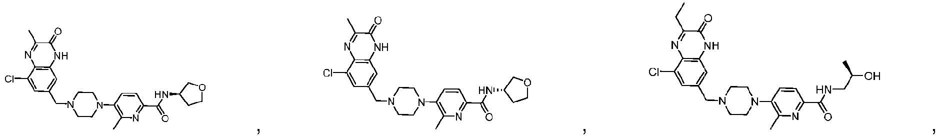

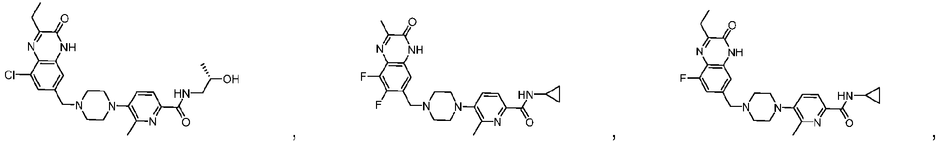

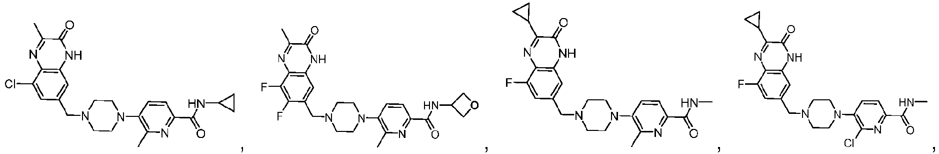

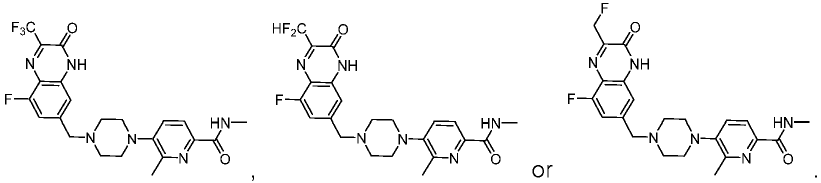

12. A pharmaceutical composition, comprising: an effective dose of the compound of Formula (I) or the pharmaceutically acceptable salt, the stereoisomer, the tautomer or the N-oxide thereof according to any one of claims 1-11, and a pharmaceutically acceptable carrier and/or excipient; or further comprising one or more other therapeutic agents.

13. Use of the compound or the pharmaceutically acceptable salt, the stereoisomer, the tautomer or the N-oxide thereof according to any one of claims 1-11, or the pharmaceutical composition according to claim 12 in the preparation of a PARP1 inhibitor, wherein the PARP1 inhibitor is used in the preparation of a drug for treating cancer; preferably, the cancer is a PARP1-mediated BRCA gene defective tumor; and more preferably, the tumor is selected from breast cancer, ovarian cancer, pancreatic cancer, prostate cancer, colorectal cancer, bladder cancer, gastrointestinal cancer, lung cancer or blood cancer.
